# EUROPEAN PATENT APPLICATION

(11) **EP 1 344 761 A1**
(43) Date of publication of application: **17.09.2003**
(21) Application number: 00978032.1
(22) Date of filing: 30.11.2000
(51) Int. Cl.: C07B 61/00, G06F 17/50

(54) **METHOD OF DESIGNING THE MOLECULAR STRUCTURE OF INHIBITOR TO ENZYME**

(71) Applicant: Toyo Suisan Kaisha, Ltd., Tokyo 108-8501 (JP)
(72) Inventor: MIZUSHINA, Yoshiyuki, Noda-shi, Chiba 278-0022 (JP); SAKAGUCHI, Kengo, Tsukuba-shi, Ibaraki 300-2667 (JP); SUGAWARA, Fumio, Niiza-shi, Saitama 352-0012 (JP)
(74) Representative: Ford, Timothy James
(86) International application number: JP0008467
(87) International publication number: WO02044112

(57) **Abstract**

A method of designing a molecular structure of an inhibitor of a target enzyme by analyzing, in the tertiary structure of a first enzyme, a geometric configuration formed by amino acids to which an inhibitor on the first enzyme is to bind. The method comprises (1) a step of determining the amino acid group of the first enzyme to which the inhibitor is to bind, (2) a step of determining the geometric configuration of the amino acid group of the step (1), (3) a step of searching for an amino acid group having a geometric configuration similar to that of the step (2), in the target enzyme, and (4) a step of searching weather or not (i) the amino acids constituting the amino acid group are present at the surface of the target enzyme, and (ii) the amino acids have been conserved in different species by comparison with an enzyme derived from an organism of different types from the organism from which the target enzyme is derived. When the requirements are satisfied, it is assumed that the geometric configuration formed by amino acids to which the inhibitor of the first enzyme is to bind, is also present in the target enzyme, then the molecular structure of an inhibitor of the target enzyme is designed.

SEQUENCE LISTING

<110> TOYO SUISAN KAISHA, LTD.

<120> A METHOD FOR DESIGNING A MOLECULAR STRUCTURE OF AN
      INHIBITOR FOR AN ENZYME

<130> 00S1359P

<140>
<141>

<160> 46

<170> PatentIn Ver. 2.0

<210> 1
<211> 335
<212> PRT
<213> rat DNA polymerase beta

<400> 1
Met Ser Lys Arg Lys Ala Pro Gln Glu Thr Leu Asn Gly Gly Ile Thr
  1               5                  10                  15

Asp Met Leu Val Glu Leu Ala Asn Phe Glu Lys Asn Val Ser Gln Ala
             20                  25                  30

Ile His Lys Tyr Asn Ala Tyr Arg Lys Ala Ala Ser Val Ile Ala Lys
         35                  40                  45

Tyr Pro His Lys Ile Lys Ser Gly Ala Glu Ala Lys Lys Leu Pro Gly
     50                  55                  60

Val Gly Thr Lys Ile Ala Glu Lys Ile Asp Glu Phe Leu Ala Thr Gly
 65                  70                  75                  80

Lys Leu Arg Lys Leu Glu Lys Ile Arg Gln Asp Asp Thr Ser Ser Ser
                 85                  90                  95

Ile Asn Phe Leu Thr Arg Val Thr Gly Ile Gly Pro Ser Ala Ala Arg
            100                 105                 110

Lys Leu Val Asp Glu Gly Ile Lys Thr Leu Glu Asp Leu Arg Lys Asn
        115                 120                 125

Glu Asp Lys Leu Asn His His Gln Arg Ile Gly Leu Lys Tyr Phe Glu
    130                 135                 140

Asp Phe Glu Lys Arg Ile Pro Arg Glu Glu Met Leu Gln Met Gln Asp
145                 150                 155                 160

Ile Val Leu Asn Glu Val Lys Lys Leu Asp Pro Glu Tyr Ile Ala Thr
                165                 170                 175

Val Cys Gly Ser Phe Arg Arg Gly Ala Glu Ser Ser Gly Asp Met Asp
            180                 185                 190

Val Leu Leu Thr His Pro Asn Phe Thr Ser Glu Ser Ser Lys Gln Pro
        195                 200                 205

Lys Leu Leu His Arg Val Val Glu Gln Leu Gln Lys Val Arg Phe Ile
    210                 215                 220

Thr Asp Thr Arg Ser Lys Gly Glu Thr Lys Phe Met Gly Val Cys Gln
225                 230                 235                 240

Leu Pro Ser Glu Asn Asp Glu Asn Glu Tyr Pro His Arg Arg Ile Asp
                245                 250                 255

Ile Arg Leu Ile Pro Lys Asp Gln Tyr Tyr Cys Gly Val Leu Tyr Phe
            260                 265                 270

Thr Gly Ser Asp Ile Phe Asn Lys Asn Met Arg Ala His Ala Leu Glu
        275                 280                 285

Lys Gly Phe Thr Ile Asn Glu Tyr Thr Ile Arg Pro Leu Gly Val Thr
    290                 295                 300

Gly Val Ala Gly Glu Pro Leu Pro Val Asp Ser Glu Gln Asp Ile Phe
305                 310                 315                 320

Asp Tyr Ile Gln Trp Arg Tyr Arg Glu Pro Lys Asp Arg Ser Glu
                325                 330                 335

<210> 2
<211> 1429
<212> PRT
<213> yeast DNA topoisomerase

<400> 2
Met Ser Thr Glu Pro Val Ser Ala Ser Asp Lys Tyr Gln Lys Ile Ser
1               5                   10                  15

Gln Leu Glu His Ile Leu Lys Arg Pro Asp Thr Tyr Ile Gly Ser Val
            20                  25                  30

Glu Thr Gln Glu Gln Leu Gln Trp Ile Tyr Asp Glu Glu Thr Asp Cys
        35                  40                  45

Met Ile Glu Lys Asn Val Thr Ile Val Pro Gly Leu Phe Lys Ile Phe
    50                  55                  60

Asp Glu Ile Leu Val Asn Ala Ala Asp Asn Asn Lys Val Arg Asp Pro
65                  70                  75                  80

Ser Met Lys Arg Ile Asp Val Asn Ile His Ala Glu Glu His Thr Ile
                85                  90                  95

Glu Val Lys Asn Asp Gly Lys Gly Ile Pro Ile Glu Ile His Asn Lys
            100                 105                 110

Glu Asn Ile Tyr Ile Pro Glu Met Ile Phe Gly His Leu Leu Thr Ser
        115                 120                 125

Ser Asn Tyr Asp Asp Asp Glu Lys Lys Val Thr Gly Gly Arg Asn Gly
    130                 135                 140

Tyr Gly Ala Lys Leu Cys Asn Ile Phe Ser Thr Glu Phe Ile Leu Glu
145                 150                 155                 160

Thr Ala Asp Leu Asn Val Gly Gln Lys Tyr Val Gln Lys Trp Glu Asn
                165                 170                 175

Asn Met Ser Ile Cys His Pro Pro Lys Ile Thr Ser Tyr Lys Lys Gly
            180                 185                 190

Pro Ser Tyr Thr Lys Val Thr Phe Lys Pro Asp Leu Thr Arg Phe Gly

        195                 200                 205

Met Lys Glu Leu Asp Asn Asp Ile Leu Gly Val Met Arg Arg Arg Val
    210                 215                 220

Tyr Asp Ile Asn Gly Ser Val Arg Asp Ile Asn Val Tyr Leu Asn Gly
225                 230                 235                 240

Lys Ser Leu Lys Ile Arg Asn Phe Lys Asn Tyr Val Glu Leu Tyr Leu
                245                 250                 255

Lys Ser Leu Glu Lys Lys Arg Gln Leu Asp Asn Gly Glu Asp Gly Ala
            260                 265                 270

Ala Lys Ser Asp Ile Pro Thr Ile Leu Tyr Glu Arg Ile Asn Asn Arg
        275                 280                 285

Trp Glu Val Ala Phe Ala Val Ser Asp Ile Ser Phe Gln Gln Ile Ser
    290                 295                 300

Phe Val Asn Ser Ile Ala Thr Thr Met Gly Gly Thr His Val Asn Tyr
305                 310                 315                 320

Ile Thr Asp Gln Ile Val Lys Lys Ile Ser Glu Ile Leu Lys Lys Lys
                325                 330                 335

Lys Lys Lys Ser Val Lys Ser Phe Gln Ile Lys Asn Asn Met Phe Ile
            340                 345                 350

Phe Ile Asn Cys Leu Ile Glu Asn Pro Ala Phe Thr Ser Gln Thr Lys
        355                 360                 365

Glu Gln Leu Thr Thr Arg Val Lys Asp Phe Gly Ser Arg Cys Glu Ile
    370                 375                 380

Pro Leu Glu Tyr Ile Asn Lys Ile Met Lys Thr Asp Leu Ala Thr Arg
385                 390                 395                 400

Met Phe Glu Ile Ala Asp Ala Asn Glu Glu Asn Ala Leu Lys Lys Ser
                405                 410                 415

Asp Gly Thr Arg Lys Ser Arg Ile Thr Asn Tyr Pro Lys Leu Glu Asp
            420                 425                 430

Ala Asn Lys Ala Gly Thr Lys Glu Gly Tyr Lys Cys Thr Leu Val Leu
        435                 440                 445

Thr Glu Gly Asp Ser Ala Leu Ser Leu Ala Val Ala Gly Leu Ala Val
    450                 455                 460

Val Gly Arg Asp Tyr Tyr Gly Cys Tyr Pro Leu Arg Gly Lys Met Leu
465                 470                 475                 480

Asn Val Arg Glu Ala Ser Ala Asp Gln Ile Leu Lys Asn Ala Glu Ile
                485                 490                 495

Gln Ala Ile Lys Lys Ile Met Gly Leu Gln His Arg Lys Lys Tyr Glu
            500                 505                 510

Asp Thr Lys Ser Leu Arg Tyr Gly His Leu Met Ile Met Thr Asp Gln
        515                 520                 525

Asp His Asp Gly Ser His Ile Lys Gly Leu Ile Ile Asn Phe Leu Glu
    530                 535                 540

Ser Ser Phe Leu Gly Leu Leu Asp Ile Gln Gly Phe Leu Leu Glu Phe
545                 550                 555                 560

Ile Thr Pro Ile Ile Lys Val Ser Ile Thr Lys Pro Thr Lys Asn Thr
                565                 570                 575

Ile Ala Phe Tyr Asn Met Pro Asp Tyr Glu Lys Trp Arg Glu Glu Glu
            580                 585                 590

Ser His Lys Phe Thr Trp Lys Gln Lys Tyr Tyr Lys Gly Leu Gly Thr
        595                 600                 605

Ser Leu Ala Gln Glu Val Arg Glu Tyr Phe Ser Asn Leu Asp Arg His
    610                 615                 620

Leu Lys Ile Phe His Ser Leu Gln Gly Asn Asp Lys Asp Tyr Ile Asp
625                 630                 635                 640

Leu Ala Phe Ser Lys Lys Lys Ala Asp Asp Arg Lys Glu Trp Leu Arg
                645                 650                 655

Gln Tyr Glu Pro Gly Thr Val Leu Asp Pro Thr Leu Lys Glu Ile Pro
            660                 665                 670

Ile Ser Asp Phe Ile Asn Lys Glu Leu Ile Leu Phe Ser Leu Ala Asp
        675                 680                 685

Asn Ile Arg Ser Ile Pro Asn Val Leu Asp Gly Phe Lys Pro Gly Gln
    690                 695                 700

Arg Lys Val Leu Tyr Gly Cys Phe Lys Lys Asn Leu Lys Ser Glu Leu
705                 710                 715                 720

Lys Val Ala Gln Leu Ala Pro Tyr Val Ser Glu Cys Thr Ala Tyr His
                725                 730                 735

His Gly Glu Gln Ser Leu Ala Gln Thr Ile Ile Gly Leu Ala Gln Asn
            740                 745                 750

Phe Val Gly Ser Asn Asn Ile Tyr Leu Leu Leu Pro Asn Gly Ala Phe
        755                 760                 765

Gly Thr Arg Ala Thr Gly Gly Lys Asp Ala Ala Ala Ala Arg Tyr Ile
    770                 775                 780

Tyr Thr Glu Leu Asn Lys Leu Thr Arg Lys Ile Phe His Pro Ala Asp
785                 790                 795                 800

Asp Pro Leu Tyr Lys Tyr Ile Gln Glu Asp Glu Lys Thr Val Glu Pro
                805                 810                 815

Glu Trp Tyr Leu Pro Ile Leu Pro Met Ile Leu Val Asn Gly Ala Glu
            820                 825                 830

Gly Ile Gly Thr Gly Arg Ser Thr Tyr Ile Pro Pro Phe Asn Pro Leu
        835                 840                 845

Glu Ile Ile Lys Asn Ile Arg His Leu Met Asn Asp Glu Glu Leu Glu
    850                 855                 860

Gln Met His Pro Trp Phe Arg Gly Trp Thr Gly Thr Ile Glu Glu Ile
865                 870                 875                 880

Glu Pro Leu Arg Tyr Arg Met Tyr Gly Arg Ile Glu Gln Ile Gly Asp

                885                 890                 895

Asn Val Leu Glu Ile Thr Glu Leu Pro Ala Arg Thr Trp Thr Ser Thr
            900                 905                 910

Ile Lys Glu Tyr Leu Leu Leu Gly Leu Ser Gly Asn Asp Lys Ile Lys
        915                 920                 925

Pro Trp Ile Lys Asp Met Glu Glu Gln His Asp Asp Asn Ile Lys Phe
    930                 935                 940

Ile Ile Thr Leu Ser Pro Glu Glu Met Ala Lys Thr Arg Lys Ile Gly
945                 950                 955                 960

Phe Tyr Glu Arg Phe Lys Leu Ile Ser Pro Ile Ser Leu Met Asn Met
                965                 970                 975

Val Ala Phe Asp Pro His Gly Lys Ile Lys Lys Tyr Asn Ser Val Asn
            980                 985                 990

Glu Ile Leu Ser Glu Phe Tyr Tyr Val Arg Leu Glu Tyr Tyr Gln Lys
        995                 1000                1005

Arg Lys Asp His Met Ser Glu Arg Leu Gln Trp Glu Val Glu Lys Tyr
    1010                1015                1020

Ser Phe Gln Val Lys Phe Ile Lys Met Ile Ile Glu Lys Glu Leu Thr
1025                1030                1035                1040

Val Thr Asn Lys Pro Arg Asn Ala Ile Ile Gln Glu Leu Glu Asn Leu
                1045                1050                1055

Gly Phe Pro Arg Phe Asn Lys Glu Gly Lys Pro Tyr Tyr Gly Ser Pro
            1060                1065                1070

Asn Asp Glu Ile Ala Glu Gln Ile Asn Asp Val Lys Gly Ala Thr Ser
        1075                1080                1085

Asp Glu Glu Asp Glu Glu Ser Ser His Glu Asp Thr Glu Asn Val Ile
    1090                1095                1100

Asn Gly Pro Glu Glu Leu Tyr Gly Thr Tyr Glu Tyr Leu Leu Gly Met
1105                1110                1115                1120

Arg Ile Trp Ser Leu Thr Lys Glu Arg Tyr Gln Lys Leu Leu Lys Gln
                1125                1130                1135

Lys Gln Glu Lys Glu Thr Glu Leu Glu Asn Leu Leu Lys Leu Ser Ala
            1140                1145                1150

Lys Asp Ile Trp Asn Thr Asp Leu Lys Ala Phe Glu Val Gly Tyr Gln
        1155                1160                1165

Glu Phe Leu Gln Arg Asp Ala Glu Ala Arg Gly Gly Asn Val Pro Asn
    1170                1175                1180

Lys Gly Ser Lys Thr Lys Gly Lys Gly Lys Arg Lys Leu Val Asp Asp
1185                1190                1195                1200

Glu Asp Tyr Asp Pro Ser Lys Lys Asn Lys Lys Ser Thr Ala Arg Lys
                1205                1210                1215

Gly Lys Lys Ile Lys Leu Glu Asp Lys Asn Phe Glu Arg Ile Leu Leu
            1220                1225                1230

Glu Gln Lys Leu Val Thr Lys Ser Lys Ala Pro Thr Lys Ile Lys Lys
        1235                1240                1245

Glu Lys Thr Pro Ser Val Ser Glu Thr Lys Thr Glu Glu Glu Glu Asn
    1250                1255                1260

Ala Pro Ser Ser Thr Ser Ser Ser Ser Ile Phe Asp Ile Lys Lys Glu
1265                1270                1275                1280

Asp Lys Asp Glu Gly Glu Leu Ser Lys Ile Ser Asn Lys Phe Lys Lys
                1285                1290                1295

Ile Ser Thr Ile Phe Asp Lys Met Gly Ser Thr Ser Ala Thr Ser Lys
            1300                1305                1310

Glu Asn Thr Pro Glu Gln Asp Asp Val Ala Thr Lys Lys Asn Gln Thr
        1315                1320                1325

Thr Ala Lys Lys Thr Ala Val Lys Pro Lys Leu Ala Lys Lys Pro Val
    1330                1335                1340

Arg Lys Gln Gln Lys Val Val Glu Leu Ser Gly Glu Ser Asp Leu Glu
1345                1350                1355                1360

Ile Leu Asp Ser Tyr Thr Asp Arg Glu Asp Ser Asn Lys Asp Glu Asp
                1365                1370                1375

Asp Ala Ile Pro Gln Arg Ser Arg Arg Gln Arg Ser Ser Arg Ala Ala
            1380                1385                1390

Ser Val Pro Lys Lys Ser Tyr Val Glu Thr Leu Glu Leu Ser Asp Asp
        1395                1400                1405

Ser Phe Ile Glu Asp Asp Glu Glu Glu Asn Gln Gly Ser Asp Val Ser
    1410                1415                1420

Phe Asn Glu Glu Asp
1425

<210> 3
<211> 100
<212> PRT
<213> Human immunodeficiency virus type 1

<400> 3
Pro Ile Ser Pro Ile Glu Thr Val Pro Val Lys Leu Lys Pro Gly Met
1               5                   10                  15

Asp Gly Pro Lys Val Lys Gln Trp Pro Leu Thr Glu Glu Lys Ile Lys
            20                  25                  30

Ala Leu Val Glu Ile Cys Thr Glu Met Glu Lys Glu Gly Lys Ile Ser
        35                  40                  45

Lys Ile Gly Pro Glu Asn Pro Tyr Asn Thr Pro Val Phe Ala Ile Lys
    50                  55                  60

Lys Lys Asp Ser Thr Lys Trp Arg Lys Leu Val Asp Phe Arg Glu Leu
65                  70                  75                  80

Asn Lys Arg Thr Gln Asp Phe Trp Glu Val Gln Leu Gly Ile Pro His
                85                  90                  95

Pro Ala Gly Leu
            100

<210> 4
<211> 100
<212> PRT
<213> Simian immunodeficiency virus

<400> 4
Leu Ser Asp Lys Ile Pro Ile Thr Lys Val Lys Leu Lys Pro Gly Val
 1               5                   10                  15

Asp Gly Pro Arg Ile Lys Gln Trp Pro Leu Ser Lys Glu Lys Ile Val
            20                  25                  30

Gly Leu Gln Lys Ile Cys Asp Arg Leu Glu Glu Glu Gly Lys Ile Ser
        35                  40                  45

Arg Val Asp Pro Gly Asn Asn Tyr Asn Thr Pro Ile Phe Ala Ile Lys
    50                  55                  60

Lys Lys Asp Lys Asn Glu Trp Arg Lys Leu Ile Asp Phe Arg Glu Leu
65                  70                  75                  80

Asn Lys Leu Thr Gln Asp Phe His Glu Leu Gln Leu Gly Ile Pro His
                85                  90                  95

Pro Ala Gly Ile
            100

<210> 5
<211> 100
<212> PRT
<213> Human immunodeficiency virus type 2

<400> 5
Pro Val Ala Lys Ile Glu Pro Ile Lys Ile Met Leu Lys Pro Gly Lys
 1               5                   10                  15

Asp Gly Pro Arg Leu Arg Gln Trp Pro Leu Thr Lys Glu Lys Ile Glu
            20                  25                  30

Ala Leu Lys Glu Ile Cys Glu Lys Met Glu Lys Glu Gly Gln Leu Glu
        35                  40                  45

Glu Ala Pro Pro Thr Asn Pro Tyr Asn Thr Pro Thr Phe Ala Ile Arg
    50                  55                  60

Lys Lys Asp Lys Asn Lys Trp Arg Met Leu Ile Asp Phe Arg Glu Leu
65                  70                  75                  80

Asn Lys Val Thr Gln Asp Phe Thr Glu Ile Gln Leu Gly Ile Pro His
                85                  90                  95

Pro Ala Gly Leu
            100

<210> 6
<211> 100
<212> PRT
<213> Sooty mangabey

<400> 6
Pro Ile Ala Lys Val Glu Pro Ile Lys Val Thr Leu Lys Pro Gly Lys
1               5                   10                  15

Glu Gly Pro Lys Leu Arg Gln Trp Pro Leu Ser Lys Glu Lys Ile Ile
            20                  25                  30

Ala Leu Arg Glu Ile Cys Glu Lys Met Glu Lys Asp Gly Gln Leu Glu
        35                  40                  45

Glu Ala Pro Pro Thr Asn Pro Tyr Asn Thr Pro Thr Phe Ala Ile Lys
    50                  55                  60

Lys Lys Asp Lys Asn Lys Trp Arg Met Leu Ile Asp Phe Arg Glu Leu
65                  70                  75                  80

Asn Lys Val Thr Gln Asp Phe Thr Glu Val Gln Leu Gly Ile Pro His
                85                  90                  95

Pro Ala Gly Leu
            100

<210> 7
<211> 100
<212> PRT
<213> Equine infectious anemia virus

<400> 7
Gln Leu Ser Lys Glu Ile Lys Phe Arg Lys Ile Glu Leu Lys Glu Gly
1               5                   10                  15

Thr Met Gly Pro Lys Ile Pro Gln Trp Pro Leu Thr Lys Glu Lys Leu
            20                  25                  30

Glu Gly Ala Lys Glu Ile Val Gln Arg Leu Leu Ser Glu Gly Lys Ile
        35                  40                  45

Ser Glu Ala Ser Asp Asn Asn Pro Tyr Asn Ser Pro Ile Phe Val Ile
    50                  55                  60

Lys Lys Arg Ser Gly Lys Trp Arg Leu Leu Gln Asp Leu Arg Glu Leu
65                  70                  75                  80

Asn Lys Thr Val Gln Val Gly Thr Glu Ile Ser Arg Gly Leu Pro His
                85                  90                  95

Pro Gly Gly Leu
            100


<210> 8
<211> 99
<212> PRT
<213> Feline immunodeficiency virus

<400> 8
Ile Ser Asp Lys Ile Pro Ile Val Lys Val Lys Met Lys Asp Pro Asn
1               5                   10                  15

Lys Gly Pro Gln Ile Lys Gln Trp Pro Leu Ser Asn Glu Lys Ile Glu
            20                  25                  30

Ala Leu Thr Glu Ile Val Glu Arg Leu Glu Arg Glu Gly Lys Val Lys

35                  40                  45

Arg Ala Asp Pro Asn Asn Pro Trp Asn Thr Pro Val Phe Ala Ile Lys
    50                  55                  60

Lys Lys Ser Gly Lys Trp Arg Met Leu Ile Asp Phe Arg Glu Leu Asn
65                  70                  75                  80

Lys Leu Thr Glu Lys Gly Ala Glu Val Gln Leu Gly Leu Pro His Pro
                85                  90                  95

Ala Gly Leu

<210> 9
<211> 100
<212> PRT
<213> Visna lentivirus

<400> 9
Leu Glu Glu Lys Lys Ile Pro Ile Thr Glu Val Arg Leu Lys Glu Gly
1               5                   10                  15

Cys Lys Gly Pro His Ile Ala Gln Trp Pro Leu Thr Gln Glu Lys Leu
            20                  25                  30

Glu Gly Leu Lys Glu Ile Val Asp Arg Leu Glu Lys Glu Gly Lys Val
        35                  40                  45

Gly Arg Ala Pro Pro His Trp Thr Cys Asn Thr Pro Ile Phe Cys Ile
    50                  55                  60

Lys Lys Lys Ser Gly Lys Trp Arg Met Leu Ile Asp Phe Arg Glu Leu
65                  70                  75                  80

Asn Lys Gln Thr Glu Asp Leu Ala Glu Ala Gln Leu Gly Leu Pro His
                85                  90                  95

Pro Gly Gly Leu
            100

<210> 10
<211> 100
<212> PRT
<213> Ovine lentivirus

<400> 10
Leu Glu Glu Lys Lys Ile Pro Ile Thr Gln Val Lys Leu Lys Glu Gly
1               5                   10                  15

Cys Lys Gly Pro His Ile Ala Gln Trp Pro Leu Thr Gln Glu Lys Leu
            20                  25                  30

Glu Gly Leu Lys Glu Ile Val Asp Lys Leu Glu Lys Glu Gly Lys Val
        35                  40                  45

Gly Arg Ala Pro Pro His Trp Thr Cys Asn Thr Pro Ile Phe Cys Ile
    50                  55                  60

Lys Lys Lys Ser Gly Lys Trp Arg Met Leu Ile Asp Phe Arg Glu Leu
65                  70                  75                  80

Asn Lys Gln Thr Glu Asp Leu Ala Glu Ala Gln Leu Gly Leu Pro His
                85                  90                  95

Pro Gly Gly Leu
            100


<210> 11
<211> 100
<212> PRT
<213> Caprine arthritis encephalitis virus

<400> 11
Leu Glu Glu Lys Arg Ile Pro Ile Thr Lys Val Lys Leu Lys Glu Gly
1               5                   10                  15

Cys Thr Gly Pro His Val Pro Gln Trp Pro Leu Thr Glu Glu Lys Leu
            20                  25                  30

Lys Gly Leu Thr Glu Ile Ile Asp Lys Leu Val Glu Glu Gly Lys Leu
        35                  40                  45

Gly Lys Ala Pro Pro His Trp Thr Cys Asn Thr Pro Ile Phe Cys Ile
    50                  55                  60

Lys Lys Lys Ser Gly Lys Trp Arg Met Leu Ile Asp Phe Arg Glu Leu
65                  70                  75                  80

Asn Lys Gln Thr Glu Asp Leu Thr Glu Ala Gln Leu Gly Leu Pro His
                85                  90                  95

Pro Gly Gly Leu
            100

<210> 12
<211> 99
<212> PRT
<213> Bovine immunodeficiency virus

<400> 12
Val His Thr Glu Lys Ile Glu Pro Leu Pro Val Lys Val Arg Gly Pro
1               5                   10                  15

Gly Pro Lys Val Pro Gln Trp Pro Leu Thr Lys Glu Lys Tyr Gln Ala
            20                  25                  30

Leu Lys Glu Ile Val Lys Asp Leu Leu Ala Glu Gly Lys Ile Ser Glu
        35                  40                  45

Ala Ala Trp Asp Asn Pro Tyr Asn Thr Pro Val Phe Val Ile Lys Lys
    50                  55                  60

Lys Gly Thr Gly Arg Trp Arg Met Leu Met Asp Phe Arg Glu Leu Asn
65                  70                  75                  80

Lys Ile Thr Val Lys Gly Gln Glu Phe Ser Thr Gly Leu Pro Tyr Pro
                85                  90                  95

Pro Gly Ile

<210> 13
<211> 97

<212> PRT
<213> Mason-Pfizer monkey virus

<400> 13
Ile Leu Ala Pro Gln Gln Cys Ala Glu Pro Ile Thr Trp Lys Ser Asp
1               5                   10                  15

Glu Pro Val Trp Val Asp Gln Trp Pro Leu Thr Asn Asp Lys Leu Ala
            20                  25                  30

Ala Ala Gln Gln Leu Val Gln Glu Gln Leu Glu Ala Gly His Ile Thr
        35                  40                  45

Glu Ser Ser Ser Pro Trp Asn Thr Pro Ile Phe Val Ile Lys Lys Lys
    50                  55                  60

Ser Gly Lys Trp Arg Leu Leu Gln Asp Leu Arg Ala Val Asn Ala Thr
65                  70                  75                  80

Met Val Leu Met Gly Ala Leu Gln Pro Gly Leu Pro Ser Pro Val Ala
                85                  90                  95

Ile

<210> 14
<211> 97
<212> PRT
<213> Simian retrovirus SRV-1

<400> 14
Met Leu Ala Pro Gln Gln Cys Ala Glu Pro Ile Thr Trp Lys Ser Asp
1               5                   10                  15

Glu Pro Val Trp Val Asp Gln Trp Pro Leu Thr Ser Glu Lys Leu Ala
            20                  25                  30

Ala Ala Gln Gln Leu Val Gln Glu Gln Leu Glu Ala Gly His Ile Thr
        35                  40                  45

Glu Ser Asn Ser Pro Trp Asn Thr Pro Ile Phe Val Ile Lys Lys Lys
    50                  55                  60

Ser Gly Lys Trp Arg Leu Leu Gln Asp Leu Arg Ala Val Asn Ala Thr
65                  70                  75                  80

Met Val Leu Met Gly Ala Leu Gln Pro Gly Leu Pro Ser Pro Val Ala
                85                  90                  95

Ile

<210> 15
<211> 97
<212> PRT
<213> Simian retrovirus SRV-2

<400> 15
Ile Leu Ala Pro Gln Arg Tyr Ala Asp Pro Ile Thr Trp Lys Ser Asp
1               5                   10                  15

Glu Pro Val Trp Val Asp Gln Trp Pro Leu Thr Gln Glu Lys Leu Ala
            20                  25                  30

Ala Ala Gln Gln Leu Val Gln Glu Gln Leu Gln Ala Gly His Ile Ile
        35                  40                  45

Glu Ser Asn Ser Pro Trp Asn Thr Pro Ile Phe Val Ile Lys Lys Lys
    50                  55                  60

Ser Gly Lys Trp Arg Leu Leu Gln Asp Leu Arg Ala Val Asn Ala Thr
65                  70                  75                  80

Met Val Leu Met Gly Ala Leu Gln Pro Gly Leu Pro Ser Pro Val Ala
                85                  90                  95

Ile

<210> 16
<211> 95
<212> PRT
<213> Squirrel monkey retrovirus

<400> 16
Ile Pro Val Pro His Ala Asp Lys Ile Ser Trp Lys Ile Thr Asp Pro
1               5                   10                  15

Val Trp Val Asp Gln Trp Pro Leu Thr Tyr Glu Lys Thr Leu Ala Ala
            20                  25                  30

Ile Ala Leu Val Gln Glu Gln Leu Ala Ala Gly His Ile Glu Pro Thr
        35                  40                  45

Asn Ser Pro Trp Asn Thr Pro Ile Phe Ile Ile Lys Lys Lys Ser Gly
    50                  55                  60

Ser Trp Arg Leu Leu Gln Asp Leu Arg Ala Val Asn Lys Val Met Val
65                  70                  75                  80

Pro Met Gly Ala Leu Gln Pro Gly Leu Pro Ser Pro Val Ala Ile
                85                  90                  95


<210> 17
<211> 97
<212> PRT
<213> Ovine pulmonary adenocarcinoma virus

<400> 17
Ser Asp Ser Pro Val Thr His Ala Asp Pro Ile Asp Trp Lys Ser Glu
1               5                   10                  15

Glu Pro Val Trp Val Asp Gln Trp Pro Leu Thr Gln Glu Lys Leu Ser
            20                  25                  30

Ala Ala Gln Gln Leu Val Gln Glu Gln Leu Arg Leu Gly His Ile Glu
        35                  40                  45

Pro Ser Thr Ser Ala Trp Asn Ser Pro Ile Phe Val Ile Lys Lys Lys
    50                  55                  60

Ser Gly Lys Trp Arg Leu Leu Gln Asp Leu Arg Lys Val Asn Glu Thr
65                  70                  75                  80

Met Met His Met Gly Ala Leu Gln Pro Gly Leu Pro Thr Pro Ser Ala

85                  90                  95

Ile

<210> 18
<211> 97
<212> PRT
<213> Mouse mammary tumor virus

<400> 18
Ala Ile Glu Ser Asn Leu Phe Ala Asp Gln Ile Ser Trp Lys Ser Asp
1               5                   10                  15

Gln Pro Val Trp Leu Asn Gln Trp Pro Leu Lys Gln Glu Lys Leu Gln
            20                  25                  30

Ala Leu Gln Gln Leu Val Thr Glu Gln Leu Gln Leu Gly His Leu Glu
        35                  40                  45

Glu Ser Asn Ser Pro Trp Asn Thr Pro Val Phe Val Ile Lys Lys Lys
    50                  55                  60

Ser Gly Lys Trp Arg Leu Leu Gln Asp Leu Arg Ala Val Asn Ala Thr
65                  70                  75                  80

Met His Asp Met Gly Ala Leu Gln Pro Gly Leu Pro Ser Pro Val Ala
                85                  90                  95

Val

<210> 19
<211> 97
<212> PRT
<213> Human endogenous retrovirus

<400> 19
Val Thr Val Glu Pro Pro Lys Pro Ile Pro Leu Thr Trp Lys Thr Glu
1               5                   10                  15

Lys Pro Val Trp Val Asn Gln Trp Pro Leu Pro Lys Gln Lys Leu Glu
            20                  25                  30

Ala Leu His Leu Leu Ala Asn Glu Gln Leu Glu Lys Gly His Ile Glu
        35                  40                  45

Pro Ser Phe Ser Pro Trp Asn Ser Pro Val Phe Val Ile Gln Lys Lys
    50                  55                  60

Ser Gly Lys Trp His Thr Leu Thr Asp Leu Arg Ala Val Asn Ala Val
65                  70                  75                  80

Ile Gln Pro Met Gly Pro Leu Gln Pro Gly Leu Pro Ser Pro Ala Met
                85                  90                  95

Ile

<210> 20
<211> 97
<212> PRT
<213> Mouse intracisternal A-particle

<400> 20
Ser Leu Ala Ala Ile Gly Ala Ala Arg Pro Ile Pro Trp Lys Thr Gly
1               5                   10                  15

Asp Pro Val Trp Val Pro Gln Trp His Leu Ser Ser Glu Lys Leu Glu
            20                  25                  30

Ala Val Ile Gln Leu Val Glu Glu Gln Leu Lys Leu Gly His Ile Asp
        35                  40                  45

Pro Ser Thr Ser Pro Trp Asn Thr Pro Ile Phe Val Ile Lys Lys Lys
    50                  55                  60

Ser Gly Lys Trp Arg Leu Leu His Asp Leu Arg Pro Ile Asn Glu Gln
65                  70                  75                  80

Met Asn Leu Phe Gly Pro Val Gln Arg Gly Leu Pro Val Leu Ser Ala
                85                  90                  95

Leu

<210> 21
<211> 96
<212> PRT
<213> Hamster intracisternal A-particles

<400> 21
Leu Gly Ala Val Glu Ala Ser Arg Pro Ile Pro Trp Lys Thr Glu Glu
1               5                   10                  15

Pro Leu Trp Val Ser Gln Trp Pro Leu Ser Ser Glu Lys Leu Glu Ala
            20                  25                  30

Val Thr Arg Leu Val Gln Glu Gln Glu Arg Leu Gly His Leu Glu Pro
        35                  40                  45

Ser Thr Ser Pro Trp Asn Thr Pro Ile Phe Val Ile Lys Lys Lys Ser
    50                  55                  60

Gly Lys Trp Arg Leu Leu His Asp Leu Arg Ala Ile Asn Asn Gln Met
65                  70                  75                  80

His Leu Phe Gly Pro Val Gln Arg Gly Leu Pro Leu Leu Ser Ala Leu
                85                  90                  95

<210> 22
<211> 97
<212> PRT
<213> Rous sarcoma virus

<400> 22
Thr Val Ala Leu His Leu Ala Ile Pro Leu Lys Trp Lys Pro Asp His
1               5                   10                  15

Thr Pro Val Trp Ile Asp Gln Trp Pro Leu Pro Glu Gly Lys Leu Val
            20                  25                  30

Ala Leu Thr Gln Leu Val Glu Lys Glu Leu Gln Leu Gly His Ile Glu
        35                  40                  45

Pro Ser Leu Ser Cys Trp Asn Thr Pro Val Phe Val Ile Arg Lys Ala
    50                  55                  60

Ser Gly Ser Tyr Arg Leu Leu His Asp Leu Arg Ala Val Asn Ala Lys
65                  70                  75                  80

Leu Val Pro Phe Gly Ala Val Gln Gln Gly Ala Pro Val Leu Ser Ala
                85                  90                  95

Leu

<210> 23
<211> 97
<212> PRT
<213> Human T-cell lymphotropic virus type 1

<400> 23
Asn Leu Ala Asn Thr Gly Ala Ser Arg Pro Trp Ala Arg Thr Pro Pro
1               5                   10                  15

Lys Ala Pro Arg Asn Gln Pro Val Pro Phe Lys Pro Glu Arg Leu Gln
            20                  25                  30

Ala Leu Gln His Leu Val Arg Lys Ala Leu Glu Ala Gly His Ile Glu
        35                  40                  45

Pro Tyr Thr Gly Pro Gly Asn Asn Pro Val Phe Pro Val Lys Lys Ala
    50                  55                  60

Asn Gly Thr Trp Arg Phe Ile His Asp Leu Arg Ala Thr Asn Ser Leu
65                  70                  75                  80

Thr Ile Asp Leu Ser Ser Ser Ser Pro Gly Pro Pro Asp Leu Ser Ser
                85                  90                  95

Leu

<210> 24
<211> 172
<212> PRT
<213> Human T-cell lymphotropic virus type 2

<400> 24
Gly Lys Ala Pro Arg His Pro Asp Pro Arg Arg Gln Trp Ala Asn Gln
1               5                   10                  15

His Pro Val Gln Thr Pro Pro Asn Pro Pro Thr His Ile Leu Ala Leu
            20                  25                  30

Pro Lys Val Pro Arg Tyr Pro Phe Leu Leu Pro Leu Arg His Pro Gln
        35                  40                  45

Gln Met Asp His His Trp Lys Gly Arg Pro Thr Thr Met Pro Gly Ala
    50                  55                  60

Ser Ile Pro Pro Arg Arg Pro Gln Pro Pro Pro Ile Ala Ala Asn Ser
65                  70                  75                  80

His Ser Lys His His Arg Pro Arg Thr Pro Ser Pro Thr Ser Pro Ser
                85                  90                  95

Gly Pro Ile Ser Phe Lys Pro Glu Arg Leu Gln Ala Leu Asn Asp Leu
            100                 105                 110

Val Ser Lys Ala Leu Glu Ala Gly His Ile Glu Pro Tyr Ser Gly Pro
        115                 120                 125

Gly Asn Asn Pro Val Phe Pro Val Lys Lys Pro Asn Gly Lys Trp Arg
    130                 135                 140

Phe Ile His Asp Leu Arg Ala Thr Asn Ala Ile Thr Thr Thr Leu Thr
145                 150                 155                 160

Ser Pro Ser Pro Gly Pro Pro Asp Leu Thr Ser Leu
                165                 170

<210> 25

<211> 100
<212> PRT
<213> Feline endogenous virus

<400> 25
Gly Arg Ala Lys Cys Gln Val Pro Ile Ile Ile Asp Leu Lys Pro Thr
1               5                   10                  15

Ala Met Pro Val Ser Ile Arg Gln Tyr Pro Met Ser Lys Glu Ala His
            20                  25                  30

Met Gly Ile Gln Pro His Ile Thr Arg Phe Leu Glu Leu Gly Val Leu
        35                  40                  45

Arg Pro Cys Arg Ser Pro Trp Asn Thr Pro Leu Leu Pro Val Lys Lys
    50                  55                  60

Pro Gly Thr Arg Asp Tyr Arg Pro Val Gln Asp Leu Arg Glu Val Asn
65                  70                  75                  80

Lys Arg Thr Met Asp Ile His Pro Thr Val Pro Asn Pro Tyr Asn Leu
                85                  90                  95

Leu Ser Thr Leu
            100


<210> 26
<211> 100
<212> PRT
<213> Baboon endogenous virus

<400> 26
Gly Arg Ala Lys Cys Gln Ala Pro Ile Ile Ile Asp Leu Lys Pro Thr
1               5                   10                  15

Ala Val Pro Val Ser Ile Lys Gln Tyr Pro Met Ser Leu Glu Ala His
            20                  25                  30

Met Gly Ile Arg Gln His Ile Ile Lys Phe Leu Glu Leu Gly Val Leu
        35                  40                  45

Arg Pro Cys Arg Ser Pro Trp Asn Thr Pro Leu Leu Pro Val Lys Lys

50                  55                  60

Pro Gly Thr Gln Asp Tyr Arg Pro Val Gln Asp Leu Arg Glu Ile Asn
65                  70                  75                  80

Lys Arg Thr Val Asp Ile His Pro Thr Val Pro Asn Pro Tyr Asn Leu
                85                  90                  95

Leu Ser Thr Leu
            100

<210> 27
<211> 100
<212> PRT
<213> Friend murine leukemia virus

<400> 27
Gly Leu Ala Val Arg Gln Ala Pro Leu Ile Ile Pro Leu Lys Ala Thr
1               5                   10                  15

Ser Thr Pro Val Ser Ile Lys Gln Tyr Pro Met Ser Gln Glu Ala Arg
            20                  25                  30

Leu Gly Ile Lys Pro His Ile Gln Arg Leu Leu Asp Gln Gly Ile Leu
        35                  40                  45

Val Pro Cys Gln Ser Pro Trp Asn Thr Pro Leu Leu Pro Val Lys Lys
    50                  55                  60

Pro Gly Thr Asn Asp Tyr Arg Pro Val Gln Asp Leu Arg Glu Val Asn
65                  70                  75                  80

Lys Arg Val Glu Asp Ile His Pro Thr Val Pro Asn Pro Tyr Asn Leu
                85                  90                  95

Leu Ser Gly Leu
            100

<210> 28
<211> 100
<212> PRT

<213> Moloney murine leukemia virus

<400> 28
Gly Leu Ala Val Arg Gln Ala Pro Leu Ile Ile Pro Leu Lys Ala Thr
1               5                   10                  15

Ser Thr Pro Val Ser Ile Lys Gln Tyr Pro Met Ser Gln Glu Ala Arg
            20                  25                  30

Leu Gly Ile Lys Pro His Ile Gln Arg Leu Leu Asp Gln Gly Ile Leu
        35                  40                  45

Val Pro Cys Gln Ser Pro Trp Asn Thr Pro Leu Leu Pro Val Lys Lys
    50                  55                  60

Pro Gly Thr Asn Asp Tyr Arg Pro Val Gln Asp Leu Arg Glu Val Asn
65                  70                  75                  80

Lys Arg Val Glu Asp Ile His Pro Thr Val Pro Asn Pro Tyr Asn Leu
                85                  90                  95

Leu Ser Gly Leu
            100


<210> 29
<211> 100
<212> PRT
<213> AKV murine leukemia virus

<400> 29
Gly Leu Ala Val Arg Gln Ala Pro Leu Ile Ile Pro Leu Lys Ala Thr
1               5                   10                  15

Ser Thr Pro Val Ser Ile Lys Gln Tyr Pro Met Ser Gln Glu Ala Lys
            20                  25                  30

Leu Gly Ile Lys Pro His Ile Gln Arg Leu Leu Asp Gln Gly Ile Leu
        35                  40                  45

Val Pro Cys Gln Ser Pro Trp Asn Thr Pro Leu Leu Pro Val Lys Lys
    50                  55                  60

Pro Gly Thr Asn Asp Tyr Arg Pro Val Gln Asp Leu Arg Glu Val Asn
65                  70                  75                  80

Lys Arg Val Glu Asp Ile His Pro Thr Val Pro Asn Pro Tyr Asn Leu
                85                  90                  95

Leu Ser Gly Leu
            100


<210> 30
<211> 100
<212> PRT
<213> Radiation murine leukemia virus

<400> 30
Gly Leu Ala Val Arg Gln Ala Pro Leu Ile Ile Pro Leu Lys Ala Thr
1               5                   10                  15

Ser Thr Pro Val Ser Ile Lys Gln Tyr Pro Met Ser Gln Glu Ala Lys
            20                  25                  30

Leu Gly Ile Lys Pro His Ile Gln Arg Leu Leu Asp Gln Gly Ile Leu
        35                  40                  45

Val Pro Cys Gln Ser Pro Trp Asn Thr Pro Leu Leu Pro Val Lys Lys
    50                  55                  60

Pro Gly Thr Asn Asp Tyr Arg Pro Val Gln Gly Leu Arg Glu Val Asn
65                  70                  75                  80

Lys Arg Val Glu Asp Ile His Pro Thr Val Pro Asn Pro Tyr Asn Leu
                85                  90                  95

Leu Ser Gly Leu
            100


<210> 31
<211> 100
<212> PRT
<213> Gibbon endogenous virus

<400> 31
Gly Leu Ala Asn Gln Val Pro Pro Val Val Val Glu Leu Arg Ser Gly
1               5                   10                  15

Ala Ser Pro Val Ala Val Arg Gln Tyr Pro Met Ser Lys Glu Ala Arg
            20                  25                  30

Glu Gly Ile Arg Pro His Ile Gln Lys Phe Leu Asp Leu Gly Val Leu
        35                  40                  45

Val Pro Cys Arg Ser Pro Trp Asn Thr Pro Leu Leu Pro Val Lys Lys
    50                  55                  60

Pro Gly Thr Asn Asp Tyr Arg Pro Val Gln Asp Leu Arg Glu Ile Asn
65                  70                  75                  80

Lys Arg Val Gln Asp Ile His Pro Thr Val Pro Asn Pro Tyr Asn Leu
                85                  90                  95

Leu Ser Ser Leu
            100

<210> 32
<211> 98
<212> PRT
<213> Simian foamy virus type 1

<400> 32
Val Gly His Arg Arg Ile Lys Pro His Asn Ile Ala Thr Gly Thr Leu
1               5                   10                  15

Ala Pro Arg Pro Gln Lys Gln Tyr Pro Ile Asn Pro Lys Ala Lys Pro
            20                  25                  30

Ser Ile Gln Ile Val Ile Asp Asp Leu Leu Lys Gln Gly Val Leu Ile
        35                  40                  45

Gln Gln Asn Ser Thr Met Asn Thr Pro Val Tyr Pro Val Pro Lys Pro
    50                  55                  60

Asp Gly Lys Trp Arg Met Val Leu Asp Tyr Arg Glu Val Asn Lys Thr
65                  70                  75                  80

Ile Pro Leu Ile Ala Ala Gln Asn Gln His Ser Ala Gly Ile Leu Ser
                85                  90                  95

Ser Ile

<210> 33
<211> 98
<212> PRT
<213> SImian foamy virus type 3

<400> 33
Val Gly His Arg Arg Ile Lys Pro His His Ile Ala Thr Gly Thr Val
1               5                   10                  15

Asn Pro Arg Pro Gln Lys Gln Tyr Pro Ile Asn Pro Lys Ala Lys Ala
            20                  25                  30

Ser Ile Gln Thr Val Ile Asn Asp Leu Leu Lys Gln Gly Val Leu Ile
        35                  40                  45

Gln Gln Asn Ser Ile Met Asn Thr Pro Val Tyr Pro Val Pro Lys Pro
    50                  55                  60

Asp Gly Lys Trp Arg Met Val Leu Asp Tyr Arg Glu Val Asn Lys Thr
65                  70                  75                  80

Ile Pro Leu Ile Ala Ala Gln Asn Gln His Ser Ala Gly Ile Leu Ser
                85                  90                  95

Ser Ile

<210> 34
<211> 81
<212> PRT
<213> BJT

<400> 34
Lys Pro Gly Gln Arg Lys Val Leu Tyr Gly Cys Phe Lys Lys Asn Leu

1                   5                   10                  15

Lys Ser Glu Leu Lys Val Ala Gln Leu Ala Pro Tyr Val Ser Glu Cys
                20                  25                  30

Thr Ala Tyr His His Gly Glu Gln Ser Leu Ala Gln Thr Ile Ile Gly
            35                  40                  45

Leu Ala Gln Asn Phe Val Gly Ser Asn Asn Ile Tyr Leu Leu Leu Pro
    50                  55                  60

Asn Gly Ala Phe Gly Thr Arg Ala Thr Gly Gly Lys Asp Ala Ala Ala
65                  70                  75                  80

Ala

<210> 35
<211> 81
<212> PRT
<213> JN0598

<400> 35
Lys Pro Gly Gln Arg Lys Val Leu Phe Thr Cys Phe Lys Arg Asn Asp
1                   5                   10                  15

Lys Arg Glu Val Lys Val Ala Gln Leu Ala Gly Ser Val Ala Glu Met
                20                  25                  30

Ser Ser Tyr His His Gly Glu Met Ser Leu Met Met Thr Ile Ile Asn
            35                  40                  45

Leu Ala Gln Asn Phe Val Gly Ser Asn Asn Leu Asn Leu Leu Gln Pro
    50                  55                  60

Ile Gly Gln Phe Gly Thr Arg Leu His Gly Gly Lys Asp Ser Ala Ser
65                  70                  75                  80

Pro

<210> 36
<211> 81
<212> PRT
<213> JS0703

<400> 36
Lys Pro Gly Gln Arg Lys Val Leu Phe Thr Cys Phe Lys Arg Asn Asp
1               5                   10                  15

Lys Arg Glu Val Lys Val Ala Gln Leu Ala Gly Ser Val Ala Glu Met
            20                  25                  30

Ser Ser Tyr His His Gly Glu Met Ser Leu Met Met Thr Ile Ile Asn
        35                  40                  45

Leu Ala Gln Asn Phe Val Gly Ser Asn Asn Leu Asn Leu Leu Gln Pro
    50                  55                  60

Ile Gly Gln Phe Gly Thr Arg Leu His Gly Gly Lys Asp Ser Ala Ser
65                  70                  75                  80

Pro

<210> 37
<211> 81
<212> PRT
<213> A44406

<400> 37
Lys Pro Gly Gln Arg Lys Val Leu Phe Thr Cys Phe Lys Arg Asn Asp
1               5                   10                  15

Lys Arg Glu Val Lys Val Ala Gln Leu Ala Gly Ser Val Ala Glu Met
            20                  25                  30

Ser Ser Tyr His His Gly Glu Met Ser Leu Met Met Thr Ile Ile Asn
        35                  40                  45

Leu Ala Gln Asn Phe Val Gly Ser Asn Asn Leu Asn Leu Leu Gln Pro
    50                  55                  60

Ile Gly Gln Phe Gly Thr Arg Leu His Gly Gly Lys Asp Ser Ala Ser
65                  70                  75                  80

Pro

<210> 38
<211> 81
<212> PRT
<213> A40493

<400> 38
Lys Pro Gly Gln Arg Lys Val Leu Phe Thr Cys Phe Lys Arg Asn Asp
1               5                   10                  15

Lys Arg Glu Val Lys Val Ala Gln Leu Ala Gly Ser Val Ala Glu Met
            20                  25                  30

Ser Ser Tyr His His Gly Glu Met Ser Leu Met Met Thr Ile Ile Asn
        35                  40                  45

Leu Ala Gln Asn Phe Val Gly Ser Asn Asn Leu Asn Leu Leu Gln Pro
    50                  55                  60

Ile Gly Gln Phe Gly Thr Arg Leu His Gly Gly Lys Asp Ser Ala Ser
65                  70                  75                  80

Pro

<210> 39
<211> 81
<212> PRT
<213> A39242

<400> 39
Lys Pro Gly Gln Arg Lys Val Leu Phe Thr Cys Phe Lys Arg Asn Asp
1               5                   10                  15

Lys Arg Glu Val Lys Val Ala Gln Leu Ala Gly Ser Val Ala Glu Met
            20                  25                  30

Ser Ala Tyr His His Gly Glu Gln Ala Leu Met Met Thr Ile Val Asn
        35                  40                  45

Leu Ala Gln Asn Phe Val Gly Ser Asn Asn Ile Asn Leu Leu Gln Pro
    50                  55                  60

Ile Gly Gln Phe Gly Thr Arg Leu His Gly Gly Lys Asp Ala Ala Ser
65                  70                  75                  80

Pro

<210> 40
<211> 81
<212> PRT
<213> S59969

<400> 40
Lys Pro Gly Gln Arg Lys Val Leu Phe Thr Cys Phe Lys Arg Asn Asp
1               5                   10                  15

Lys Arg Glu Val Lys Val Ala Gln Leu Ala Gly Ser Val Ala Glu Met
            20                  25                  30

Ser Ala Tyr His His Gly Glu Gln Ala Leu Met Met Thr Ile Val Asn
        35                  40                  45

Leu Ala Gln Asn Phe Val Gly Ser Asn Asn Ile Asn Leu Leu Gln Pro
    50                  55                  60

Ile Gly Gln Phe Gly Thr Arg Leu His Gly Gly Lys Asp Ala Ala Ser
65                  70                  75                  80

Pro

<210> 41
<211> 81
<212> PRT
<213> S02160

<400> 41
Lys Pro Gly Gln Arg Lys Val Met Phe Thr Cys Phe Lys Arg Asn Asp
1               5                   10                  15

Lys Arg Glu Val Lys Val Ala Gln Leu Ser Gly Ser Val Ala Glu Met
            20                  25                  30

Ser Ala Tyr His His Gly Glu Gln Ser Leu Gln Met Thr Ile Val Asn
        35                  40                  45

Leu Ala Gln Asn Phe Val Gly Ala Asn Asn Ile Asn Leu Leu Glu Pro
    50                  55                  60

Arg Gly Gln Phe Gly Thr Arg Leu Ser Gly Gly Lys Asp Cys Ala Ser
65                  70                  75                  80

Ala

<210> 42
<211> 81
<212> PRT
<213> ISZPT2

<400> 42
Lys Pro Gly Gln Arg Lys Val Met Phe Thr Cys Phe Lys Arg Asn Asp
1               5                   10                  15

Lys Arg Glu Val Lys Val Ala Gln Leu Ser Gly Ser Val Ala Glu Met
            20                  25                  30

Ser Ala Tyr His His Gly Glu Gln Ser Leu Gln Met Thr Ile Val Asn
        35                  40                  45

Leu Ala Gln Asn Phe Val Gly Ala Asn Asn Ile Asn Leu Leu Glu Pro
    50                  55                  60

Arg Gly Gln Phe Gly Thr Arg Leu Ser Gly Gly Lys Asp Cys Ala Ser
65                  70                  75                  80

Ala

<210> 43
<211> 81
<212> PRT
<213> S53599

<400> 43
Lys Pro Gly Gln Arg Lys Ile Leu Phe Val Ala Phe Lys Lys Ile Ala
1               5                   10                  15

Arg Lys Glu Met Lys Val Ala Gln Leu Val Gly Tyr Val Ser Leu Leu
            20                  25                  30

Ser Ala Tyr His His Gly Glu Gln Ser Leu Ala Ser Ala Ile Ile Gly
        35                  40                  45

Met Ala Gln Asp Tyr Val Gly Ser Asn Asn Ile Asn Leu Leu Leu Pro
    50                  55                  60

Asn Gly Gln Phe Gly Thr Arg Thr Ser Gly Gly Lys Asp Ser Ala Ser
65                  70                  75                  80

Ala

<210> 44
<211> 75
<212> PRT
<213> T06819

<400> 44
Asp Leu Phe Gly Ser Phe Lys Lys Lys Leu Tyr Lys Glu Ile Lys Val
1               5                   10                  15

Ala Gln Phe Ile Gly Tyr Val Ser Glu His Ser Ala Tyr His His Gly
            20                  25                  30

Glu Gln Ser Leu Ala Ser Thr Ile Ile Gly Met Ala Gln Asp Phe Val
        35                  40                  45

Gly Ser Asn Asn Ile Asn Leu Leu Lys Pro Asn Gly Gln Phe Gly Thr
    50                  55                  60

Cys Asn Leu Gly Gly Lys Asp His Ala Ser Ala
65                  70                  75

<210> 45
<211> 84
<212> PRT
<213> ISXFAS

<400> 45
Thr Arg Ala Arg Arg Lys Ile Leu Ala Gly Gly Val Lys Cys Phe Ala
1               5                   10                  15

Ser Asn Asn Arg Glu Arg Lys Val Phe Gln Phe Gly Gly Tyr Val Ala
            20                  25                  30

Asp His Met Phe Tyr His His Gly Asp Met Ser Leu Asn Thr Ser Ile
        35                  40                  45

Ile Lys Ala Ala Gln Tyr Tyr Pro Gly Ser Ser His Leu Tyr Pro Val
    50                  55                  60

Phe Ile Gly Ile Gly Ser Phe Gly Ser Arg His Leu Gly Gly Lys Asp
65                  70                  75                  80

Ala Gly Ser Pro

<210> 46
<211> 84
<212> PRT
<213> S27329

<400> 46
Thr Arg Ala Arg Arg Lys Ile Leu Ala Gly Gly Leu Lys Cys Phe Ala
1               5                   10                  15

Ser Asn Asn Arg Glu Arg Lys Val Phe Gln Phe Gly Gly Tyr Val Ala
            20                  25                  30

Asp His Met Phe Tyr His His Gly Asp Met Ser Leu Asn Thr Ser Ile
        35                  40                  45

Ile Lys Ala Ala Gln Tyr Tyr Pro Gly Ser Ser His Leu Tyr Pro Val
    50                  55                  60

Phe Ile Gly Ile Gly Ser Phe Gly Ser Arg His Leu Gly Gly Lys Asp
65                  70                  75                  80

Ala Gly Ser Pro

## Description

### Technical Field

The present invention relates to a method of designing an enzyme inhibitor.

More specifically, the present invention relates to a method of designing a molecular structure of an inhibitor on a target enzyme, the secondary and tertiary structures of which being known, comprising: analyzing, in the tertiary structure of a first enzyme whose primary, secondary and tertiary structures are known, a geometric configuration formed by amino acids to which a first inhibitor, having biochemical, inhibitory activity on the first enzyme, is to bind; and searching, in the target enzyme, the similar geometric configuration to the geometric configuration formed in the first enzyme, thereby designing a molecular structure of an inhibitor on the target enzyme.

Further, the present invention relates to another method of designing a molecular structure of an inhibitor on a target enzyme whose tertiary structure is unknown, comprising: analyzing, in the tertiary structure of a first enzyme whose primary, secondary and tertiary structures are known, a geometric configuration formed by amino acids to which a first inhibitor having biochemical, inhibitory activity on the first enzyme, is to bind; and searching, in a second enzyme whose primary, secondary and tertiary structures are known, the similar geometric configuration to the geometric configuration formed in the first enzyme, thereby designing the molecular structure of the inhibitor on the target enzyme.

### Background Art

DNA is a substance which carry genetic information. It is well known that "DNA replication" as a reaction in which DNA is doubled, "DNA repair" as a reaction in which errors and damages in the structure thereof are repaired, and "DNA recombination" as a reaction in which genetic information is exchanged, are the basis of all phenomena of living organism. A series of DNA synthesis reactions as described above are carried out mainly by the enzymes called DNA polymerase.

As DNA polymerase (hereinafter also referred to as "pol.") of eucaryote (mainly mammals), there have been discovered 8 types of DNA polymerase, which are identified as pol. α, β, γ, δ, ε, ζ, η and θ, according to the molecular weight, features in terms of protein-chemistry and differences in a function of DNA synthesis (1-4) (Note that the number in the parenthesis represents the number of the references listed in the reference section described below). It is assumed that these enzymes play a variety of roles, respectively. Specifically, pol. α, δ and ε are presumably involved in DNA replication of nucleus (1), pol. γ is presumably involved in DNA replication of mitochondria (1), and pol. β, ξ, η and θ are presumably involved in repair of nucleus DNA (1-4) Further, it has been suggested that pol. β is also involved in DNA recombination (1). However, biochemical roles of DNA polymerase in DNA synthesis reactions, are still unclear in many aspects.

Well-known examples of a molecular biological technique, which is often employed in the study of the roles played by enzymes, generally include: a method of producing a mutant having deficiency of the enzyme, and thereby investigating the phenotype; or a method of producing an individual whose gene encoding the enzyme has been knocked-out, thereby investigating the phenotype. However, in the case of DNA polymerase, application of such methods as described above most likely results in the death of the organism. Thus, it is difficult to analyze the roles of the enzyme by using such methods.

On the other hand, there has been another approach in which the functions of the DNA polymerase are studied on the basis of the analysis of an inhibitor on the enzyme. Conventionally, an inhibitor on an enzyme is searched by using natural substances, chemical files and the like. However, such conventional methods are basically the methods in which an inhibitor is discovered simply "by chance" after an enormous number of tests are carried out, which is by no means efficient.

If the structure of the amino acid residues constituting an enzyme can be identified up to the tertiary level thereof, the molecular structure of an inhibitor on the enzyme will more easily be deduced. However, an enzyme generally has a large molecular weight and a relatively complicated three-dimensional structure, and it is thus quite often difficult to analyze the tertiary structure thereof.

In consideration of the problems described above, an object of the present invention is to provide a method of efficiently discovering an inhibitor on an enzyme without carrying out a number of trial and error. Particularly, the object of the present invention is to provide a method of efficiently discovering an inhibitor on an enzyme whose molecular weight is approximately 30,000 or more, and thus, the tertiary structure of which cannot be identified with NMR, without carrying out a number of trial and error.

### Disclosure of Invention

The inventors of the present invention have discovered as a result of intense study, a method of efficiently designing molecular structure of an inhibitor on an enzyme to complete the present invention. The method comprises identifying the amino acid residues of a specific enzyme, to which residues an inhibitor is to bind in an interaction between the enzyme and the inhibitor, identifying the geometric configuration formed by the Cα atoms of each of the amino acid residues, and analyzing by search (1) whether or not these amino acids are those present at the surface of the enzyme protein and (2) whether or not these amino acids are those which have been conserved in terms of the evolution theory. By the method, the number of tests required for designing a molecular structure of an inhibitor on an enzyme can be significantly reduced, as compared with the conventional method where the inhibitor is searched at random.

### Brief Description of Drawings

FIG. 1 is a block diagram showing the scheme of each process of a first method of the present invention;
FIG. 2A is a view showing the amino acid sequence of rat pol. β. FIG. 2B is a view showing the amino acid sequence of enzyme DNA topoisomerase II (hereinafter also referred to as "topo II");
FIG. 3 is a view of a three-dimensional model showing the amino acid chain of rat pol. β;
FIG. 4 is a block diagram showing the scheme of each process of a second method of the present invention;
FIG. 5 is a graph showing the inhibitory activity of nervonic acid on rat pol. β;
FIG. 6 is ¹H-¹⁵N HMQC spectroscopy of rat pol. β;
FIG. 7 is a bar graph showing shift values of ¹H HMQC spectroscopy of rat pol. β;
FIG. 8 is a bar graph showing shift values of ¹⁵N HMQC spectroscopy of rat pol. β;
FIG. 9A is a view showing the geometric configuration of the amino acid residues of rat pol. β, which residues are to be bound to nervonic acid. FIG. 9B is a view showing the geometric configuration of the amino acid residues of yeast topo II, which residues are to be bound to nervonic acid;
FIG. 10A is a view showing a three-dimensional model of rat pol. β, in which the position of the amino acid residues, which residues are to be bound to nervonic acid, is clearly indicated. FIG. 10B is a view showing a three-dimensional model of yeast topo II, in which the position of the amino acid residues, which residues are to be bound to nervonic acid, is clearly indicated;
FIGS. 11A-11F are views showing the primary sequence of amino acids of 31 types of enzymes used in the search of example 1;
FIG. 12A is a view showing the geometric configuration of the amino acid residues of rat pol. β, which residues are to be bound to nervonic acid.
FIG. 12B is a view showing the geometric configuration of the amino acid residues of human immunodeficiency virus (HIV) reverse transcriptase, which residues are to be bound to nervonic acid;
FIG. 13A is a view showing a three-dimensional model of rat pol. β, in which the position of the amino acid residues, which residues are to be bound to nervonic acid, is clearly indicated. FIG. 13B is a view showing a three-dimensional model of HIV reverse transcriptase, in which the position of the amino acid residues, which residues are to be bound to nervonic acid, is clearly indicated;
FIG. 14A is a view showing a model in a state in which nervonic acid has bound to the 8 kDa domain of rat pol. β. FIG. 14B is a view in which the model of FIG. 14A has been turned by 90° around the vertical axis;
FIG. 15 is a graph showing the inhibitory activity of nervonic acid on rat DNA polymerase, HIV reverse transcriptase and the like;
FIG. 16 is a view showing the result of electrophoresis, which represents the inhibitory activity effected by nervonic acid on yeast topo II;
FIG. 17 is a view showing the primary sequence of amino acids of yeast topo II or the like, in which the amino acid residues which have been conserved, in terms of the evolution theory, are indicated;
FIG. 18 shows ¹H-¹⁵N HMQC spectroscopy of rat pol. β;
FIG. 19 is a bar graph showing shift values of ¹H HMQC spectroscopy of rat pol. β;
FIG. 20 is a bar graph showing shift values of ¹⁵N HMQC spectroscopy of rat pol. β;
FIG. 21A is a view showing the geometric configuration of the amino acid residues of rat pol. β, which residues are to be bound to lithocholic acid. FIG. 21B is a view showing the geometric configuration of the amino acid residues of yeast topo II, which residues are to be bound to lithocholic acid;
FIG. 22A is a view showing a three-dimensional model of rat pol. β, in which the position of the amino acid residues, which residues are to be bound to lithocholic acid, is clearly indicated. FIG. 22B is a view showing a three-dimensional model of yeast topo II, in which the position of the amino acid residues, which residues are to be bound to lithocholic acid, is clearly indicated; and
FIG. 23 is a view showing the primary sequence of amino acids of yeast topo II or the like, in which the amino acid residues which have been conserved, in terms of the evolution theory, are indicated.

### Best Mode for Carrying Out the Invention

According to one aspect of the present invention, there is provided a method of designing a molecular structure of an inhibitor on an enzyme whose secondary and tertiary structures are known. Hereinafter, this method will be referred to as "the first method of the present invention".

Specifically, the first method of the present invention is a method of designing a molecular structure of an inhibitor on a target enzyme whose secondary and tertiary structures are known, comprising:
analyzing, in the tertiary structure of a first enzyme whose primary, secondary and tertiary structures are known, a geometric configuration formed by amino acids to which a first inhibitor having biological, inhibitory activity on the first enzyme, is to bind; and
searching, in the target enzyme, amino acids constituting the similar geometric configuration to the geometric configuration that was analyzed in the first enzyme;
thereby designing the molecular structure of the inhibitor on the target enzyme.

In more detail, the first method of the present invention is a method of designing a molecular structure of an inhibitor on a target enzyme whose secondary and tertiary structures are known, comprising:
(1) a step of determining, in the tertiary structure f a first enzyme, a first pocket amino acid group consisting of plural amino acids to which a first inhibitor is to bind, wherein the plural amino acids are determined by comparing the ¹H-¹⁵N HMQC spectroscopy of the first enzyme in the absence of the first inhibitor with the ¹H-¹⁵N HMQC spectroscopy of the first enzyme in the presence of the first inhibitor, and selecting amino acids that satisfy the condition that the absolute value of ¹H chemical shift is equal to or more than a specific value and/or the absolute value of ¹⁵N chemical shift is equal to or more than another specific value;
(2) a step of determining the geometric configuration of the first pocket amino acid group, by measuring, in the tertiary structure of the first enzyme, the distances between the Cα atoms of each of the plural amino acids constituting the first pocket amino acid group;
(3) a step of detecting, in the tertiary structure of the target enzyme, one or more candidates for a target pocket amino acid group having a geometric configuration similar to the geometric configuration formed by the first pocket amino acid group determined in the step (2), wherein it is presumed that the candidate for the target pocket amino acid group is present in the target enzyme if the following conditions are satisfied:
   respective amino acids constituting the candidate for the target pocket amino acid group are of the same kinds as the plural amino acids constituting the first pocket amino acid group determined in the step (2), and
   the absolute values of differences between (a) respective distances, in the tertiary structure of the amino acids of the target enzyme, between the Cα atoms of each of the amino acids constituting the target pocket amino acid group, and (b) the respective distances between the Cα atoms in the first pocket amino acid group obtained in the step (2) are within a specific value; and
(4) a step of screening the amino acids constituting the candidate for the target pocket amino acid group by the following two requirements:
   (requirement 1) the amino acids are those present at the surface of the target enzyme in the tertiary structure thereof; and
   (requirement 2) the amino acids are those that have been conserved among species, when a comparison is made between the primary sequence of the amino acids of the target enzyme and the primary sequence of the amino acids of another enzyme having the same biochemical activity as the target enzyme but derived from an organism of a different species from that of the target enzyme, in terms of biological classification; and
   determining the amino acids satisfying the above two requirements to be those constituting the usable target pocket amino acid group for designing the molecular structure of the inhibitor on the target enzyme.

In the first method of the present invention, the molecular structure of the inhibitor on the target enzyme is designed on the basis of the kinds of amino acid residues of the first enzyme, to which the first inhibitor is to bind, and the geometric configuration of amino acid residues.

An example of carrying out the first method of the present invention mentioned above is as follows. However, the first method of the present invention is not limited to this.

A method of designing a molecular structure of an inhibitor on human immunodeficiency virus (HIV) reverse transcriptase by analyzing, in the tertiary structure of rat DNA polymerase β, a geometric configuration formed by amino acids to which nervonic acid is to bind; searching, in HIV reverse transcriptase, the similar geometric configuration to the geometric configuration analyzed in rat DNA polymerase β; thereby designing the molecular structure of the inhibitor on HIV reverse transcriptase on the basis of the kinds of amino acid residues of rat DNA polymerase β, to which nervonic acid is to bind, and the geometric configuration of the amino acid residues.

In more detail, the example of carrying out the first method of the present invention is a method of designing a molecular structure of an inhibitor on HIV reverse transcriptase comprising the following steps (1) to (4):
(1) a step of determining, in the tertiary structure of rat DNA polymerase β, its pocket amino acid group consisting of plural amino acids to which nervonic acid is to bind. In the step (1), the plural amino acids are determined by comparing the ¹H-¹⁵N HMQC spectroscopy of rat DNA polymerase β in the absence of nervonic acid with the ¹H-¹⁵N HMQC spectroscopy of rat DNA polymerase β in the presence of nervonic acid, and selected from those having the absolute value of ¹H chemical shift of 0.06 ppm or more, and/or having the absolute value of ¹⁵N chemical shift of 0.4 ppm or more. In this example, the plural amino acids constituting the rat DNA polymerase β pocket amino acid group are determined to be Leu-11, Lys-35, His-51 and Thr-79.
(2) a step of determining the geometric configuration of the rat DNA polymerase β pocket amino acid group. In the step (2), the distances, in the tertiary structure of rat DNA polymerase β, between the Cα atoms of each of Leu-11, Lys-35, His-51 and Thr-79 constituting the rat DNA polymerase β pocket amino acid group are measured, thereby the geometric configuration of a triangular pyramid formed by Leu-11, Lys-35, His-51 and Thr-79 is determined.
(3) a step of detecting, in the tertiary structure of the amino acids of HIV reverse transcriptase, one or more candidates for a target pocket amino acid group having a geometric configuration similar to the geometric configuration of the rat DNA polymerase β pocket amino acid group determined in the step (2). In the step (3), amino acids constituting the candidate for the target pocket amino acid group are the same type as those constituting the rat DNA polymerase β pocket amino acid group, i.e., Leu, Lys, His and Thr. Further, the differences between (a) and (b) are within ±2 × 10⁻¹ nm, wherein (a) is respective distances, in the tertiary structure of HIV reverse transcriptase, between the Cα atoms of each of the amino acids constituting the target pocket amino acid group, and (b) is the respective distances between the Cα atoms obtained in the step (2).
(4) a step of determining by screening whether or not the candidate for a target pocket amino acid group is usable for designing the molecular structure of the inhibitor on HIV reverse transcriptase. In the step (4), the amino acids, Lys, Leu, His and Thr, which are selected as the amino acids constituting the candidate for the target pocket amino acid group are screened by the following two requirements:
   (requirement 1) the amino acids are those present at the surface of HIV reverse transcriptase in the tertiary structure thereof; and
   (requirement 2) the amino acids are those that have been conserved among species, when a comparison is made between the primary sequence of the amino acids of HIV reverse transcriptase and the primary sequence of the amino acids of immunodeficiency virus reverse transcriptase that is derived from an organism of a different species, in terms of biological classification, from human.

In this example, Lys-65, Leu-100, His-235 and Thr-386 of HIV reverse transcriptase satisfying the above two requirements were determined to be amino acids constituting the target pocket amino acid group usable for designing a molecular structure of the inhibitor on HIV reverse transcriptase.

In the example of carrying out the first method of the present invention, the molecular structure of the inhibitor on HIV reverse transcriptase is designed on the basis of the kinds of amino acid residues of rat DNA polymerase β, to which nervonic acid is to bind, and the geometric configuration of the amino acid residues.

In another aspect of the present invention, there is provided a method of designing a molecular structure of an inhibitor on an enzyme whose tertiary structures is unknown. Hereinafter, this method will be referred to as "the second method of the present invention".

Specifically, the second method of the present invention is a method of designing a molecular structure of an inhibitor on a target enzyme whose tertiary structure is unknown, comprising:
analyzing, in the tertiary structure of a first enzyme whose primary, secondary and tertiary structures are known, a geometric configuration formed by amino acids to which a first inhibitor having biological, inhibitory activity on the first enzyme is to bind; and
searching, in a second enzyme whose primary, secondary and tertiary structures are known, amino acids constructing the similar geometric configuration to the geometric configuration that was analyzed in the first enzyme;
thereby designing the molecular structure of the inhibitor on the target enzyme whose tertiary structure is unknown.

In more detail, the second method of the present invention is a method of designing a molecular structure of an inhibitor on a target enzyme whose tertiary structure is unknown, comprising:
(1) a step of determining, in the tertiary structure of a first enzyme, a first pocket amino acid group consisting of plural amino acids to which a first inhibitor is to bind, wherein the plural amino acids are determined by comparing the ¹H-¹⁵N HMQC spectroscopy of the first enzyme in the absence of the first inhibitor with the ¹H-¹⁵N HMQC spectroscopy of the first enzyme in the presence of the first inhibitor, and selecting amino acids that satisfy the condition that the absolute value of ¹H chemical shift is equal to or more than a specific value and/or the absolute value of ¹⁵N chemical shift is equal to or more than another specific value;
(2) a step of determining the geometric configuration of the first pocket amino acid group, by measuring, in the tertiary structure of the first enzyme, the distances between the Cα atoms of each of the plural amino acids constituting the first pocket amino acid group;
(3) a step of detecting, in the tertiary structure of a second enzyme, a second pocket amino acid group having a geometric configuration similar to the geometric configuration formed by the first pocket amino acid group determined in the step (2), wherein it is presumed that the second pocket amino acid group has a geometric configuration similar to the geometric configuration formed by the first pocket amino acid group if the following requirements are satisfied:
   (requirement 1) the second enzyme has the same biochemical activity as that of the target enzyme;
   (requirement 2) the organism from which the second enzyme is derived, belongs to a different species, in terms of biological classification, from the organism from which the target enzyme is derived;
   (requirement 3) the primary, secondary and tertiary structures of the second enzyme are known; and
   (requirement 4) the second enzyme is biochemically inhibited by the first inhibitor, and
   respective amino acids constituting the second pocket amino acid group are of the same kinds as the plural amino acids constituting the first pocket amino acid group determined in the step (2), and
   the absolute values of differences between (a) respective distances, in the tertiary structure of the second enzyme, between the Cα atoms of each of the amino acids constituting the second pocket amino acid group, and (b) the respective distances between the Cα atoms in the first pocket amino acid group obtained in the step (2) are within a specific value; and
(4) a step of screening the amino acids constituting the second pocket amino acid group by the following two requirements:
   (requirement 1) the amino acids are those present at the surface of the second enzyme in the tertiary structure thereof; and
   (requirement 2) the amino acids are those that have been conserved among species, when a comparison is made among the primary sequence of the amino acids of the second enzyme, the primary sequence of the amino acid of the target enzyme and the primary sequence of the amino acids of another enzyme having the same biochemical activity as that of the second enzyme but derived from an organism of a different species from that of both the second enzyme and the target enzyme, in terms of biological classification; and
   determining the amino acids satisfying the above two requirements to be those constituting the usable target pocket amino acid group for designing the molecular structure of the inhibitor on the target enzyme.

In the second method of the present invention, the molecular structure of the inhibitor on the target enzyme is designed on the basis of the kinds of amino acid residues of the first enzyme, to which the first inhibitor is to bind, and the geometric configuration of the amino acid residues.

An example of carrying out the second method of the present invention mentioned above is as follows. However, the second method of the present invention is not limited to this.

A method of designing a molecular structure of an inhibitor on human DNA topoisomerase II whose tertiary structure is unknown by analyzing, in the tertiary structure of rat DNA polymerase β, a geometric configuration formed by amino acids to which nervonic acid is to bind; searching, in yeast DNA topoisomerase II, the similar geometric configuration to the geometric configuration that was analyzed in rat DNA polymerase β; thereby designing the molecular structure of the inhibitor on human topoisomerase II on the basis of the kinds of amino acid residues of yeast DNA topoisomerase II, to which nervonic acid is to bind, and the geometric configuration of the amino acid residues.

In more detail, the example of carrying out the second method of the present invention is a method of designing a molecular structure of an inhibitor on human topoisomerase II comprising the following steps (1) to (4):
(1) a step of determining, in the tertiary structure of rat DNA polymerase β, a rat DNA polymerase β pocket amino acid group consisting of plural amino acids to which nervonic acid is to bind. In the step (1), the plural amino acids are determined by comparing the ¹H-¹⁵N HMQC spectroscopy of rat DNA polymerase β in the absence of nervonic acid with the ¹H-¹⁵N HMQC spectroscopy of rat DNA polymerase β in the presence of nervonic acid, and selected from those having the absolute value of ¹H chemical shift of 0.06 ppm or more, and/or having the absolute value of ¹⁵N chemical shift of 0.4 ppm or more. In this example, the plural amino acids constituting the rat DNA polymerase β pocket amino acid group are determined to be Leu-11, Lys-35, His-51 and Thr-79.
(2) a step of determining the geometric configuration of the rat DNA polymerase β pocket amino acid group. In the step (2), the distances, in the tertiary structure of rat DNA polymerase β, between the Cα atoms of each of Leu-11, Lys-35, His-51 and Thr-79 constituting the rat DNA polymerase β pocket amino acid group are measured, thereby the geometric configuration of a triangular pyramid formed by Leu-11, Lys-35, His-51 and Thr-79 is determined.
(3) a step of selecting, in the tertiary structure of yeast DNA topoisomerase II, a yeast DNA topoisomerase II pocket amino acid group consisting of Leu, Lys, His and Thr, and having a geometric configuration similar to the geometric configuration of the rat DNA polymerase β pocket amino acid group determined in the step (2). In the step (3), when the differences between (a) and (b) are within ±2 × 10⁻¹ nm, wherein (a) is respective distances, in the tertiary structure of the selected amino acids, between the Cα atoms of Leu, Lys, His and Thr constituting the yeast DNA topoisomerase II pocket amino acid group, and (b) is the respective distances between the Cα atoms obtained in the step (2), such Leu, Lys, His and Thr are determined to be the amino acids constituting the yeast DNA topoisomerase II pocket amino acid group.
(4) a step of determining by screening whether or not the yeast DNA topoisomerase II pocket amino acid group is usable for designing the molecular structure of the inhibitor on human DNA topoisomerase II. In the step (4), the amino acids, Leu, Lys, His and Thr, which are selected as the amino acids constituting the yeast DNA topoisomerase II pocket amino acid group are screened by the following two requirements:
   (requirement 1) the amino acids are those present at the surface of yeast DNA topoisomerase II in the tertiary structure thereof; and
   (requirement 2) the amino acids are those that have been conserved among species, when a comparison is made among the primary sequence of the amino acids of yeast DNA topoisomerase II, the primary sequence of the amino acids of human DNA topoisomerase II and the primary sequence of the amino acids of topoisomerase II derived from an organism of a different species from both yeast and human, in terms of biological classification; and
   determining the amino acids, Thr-596, His-735, Leu-741 and Lys-983, satisfying the above two requirements to be usable for designing the molecular structure of the inhibitor on the target enzyme.

In the example of carrying out the second method of the present invention, the molecular structure of the inhibitor on human topoisomerase II is designed on the basis of the kinds of amino acid residues of yeast DNA topoisomerase II, to which nervonic acid is to bind, and the geometric configuration of the amino acid residues.

Another example of carrying out the second method of the present invention mentioned above is as follows. However, the second method of the present invention is not limited to this, either.

A method of designing a molecular structure of an inhibitor on human DNA topoisomerase II whose tertiary structure is unknown; by analyzing, in the tertiary structure of rat DNA polymerase β, a geometric configuration formed by amino acids to which lithocholic acid is to bind; searching, in yeast DNA topoisomerase II, the similar geometric configuration to the geometric configuration that was analyzed in rat DNA polymerase β; thereby designing the molecular structure of the inhibitor on human topoisomerase II on the basis of the kinds of amino acid residues of yeast DNA topoisomerase II, to which lithocholic acid is to bind, and the geometric configuration of the amino acid residues.

In more detail, the example of carrying out the second method of the present invention is a method of designing a molecular structure of an inhibitor on human topoisomerase II comprising the following steps (1) to (4):
(1) a step of determining, in the tertiary structure of rat DNA polymerase β, a rat DNA polymerase β pocket amino acid group consisting of plural amino acids to which lithocholic acid is to bind. In the step (1), the plural amino acids are determined by comparing the ¹H-¹⁵N HMQC spectroscopy of rat DNA polymerase β in the absence of lithocholic acid with the ¹H-¹⁵N HMQC spectroscopy of rat DNA polymerase β in the presence of lithocholic acid, and selected from those having the absolute value of ¹H chemical shift of 0.06 ppm or more, and/or having the absolute value of ¹⁵N chemical shift of 0.4 ppm or more. In this example, the plural amino acids constituting the rat DNA polymerase β pocket amino acid group are determined to be Lys-60, Leu-77 and Thr-79.
(2) a step of determining the geometric configuration of the rat DNA polymerase β pocket amino acid group. In the step (2), the distances, in the tertiary structure of rat DNA polymerase β, between the Cα atoms of each of Lys-60, Leu-77 and Thr-79 constituting the rat DNA polymerase β pocket amino acid group are measured, thereby the geometric configuration of a triangular shape formed by Lys-60, Leu-77 and Thr-79 is determined.
(3) a step of selecting, in the tertiary structure of yeast DNA topoisomerase II, a yeast DNA topoisomerase II pocket amino acid group consisting of Lys, Leu and Thr, and having a geometric configuration similar to the geometric configuration of the rat DNA polymerase β pocket amino acid group determined in the step (2). In the step (3), when the differences between (a) and (b) are within ±2 × 10⁻¹ nm, wherein (a) is respective distances, in the tertiary structure of the selected amino acids, between the Cα atoms of Lys, Leu and Thr constituting the yeast DNA topoisomerase II pocket amino acid group, and (b) is the respective distances between the Cα atoms obtained in the step (2), such Lys, Leu and Thr of the amino acids of rat polymerase β pocket amino acid group are determined to be the amino acids constituting the yeast DNA topoisomerase II pocket amino acid group.
(4) a step of determining by screening whether or not the yeast DNA topoisomerase II pocket amino acid group is usable for designing the molecular structure of the inhibitor on human DNA topoisomerase II. In the step (4), the amino acids, Lys, Leu and Thr, which are selected as the amino acids constituting the yeast DNA topoisomerase II pocket amino acid group are screened by the following two requirements:
   (requirement 1) the amino acids are those present at the surface of yeast DNA topoisomerase II in the tertiary structure thereof; and
   (requirement 2) the amino acids are those that have been conserved among species, when a comparison is made among the primary sequence of the amino acids of yeast DNA topoisomerase II, the primary sequence of the amino acids of human DNA topoisomerase II and the primary sequence of the amino acids of topoisomerase II derived from an organism of a different species from both yeast and human, in terms of biological classification; and
   determining the amino acids, Lys-720, Leu-760 and Thr-791, satisfying the above two requirements to be those constituting the target pocket amino acid group usable for designing the molecular structure of the inhibitor on the target enzyme.

In the example of carrying out the second method of the present invention, the molecular structure of the inhibitor on human topoisomerase II is designed on the basis of the kinds of amino acid residues of yeast DNA topoisomerase II, to which lithocholic acid is to bind, and the geometric configuration of the amino acid residues.

Now, the first method of the present invention, in which a molecular structure of an inhibitor on an enzyme whose secondary and tertiary structures are known will be described in more detail.

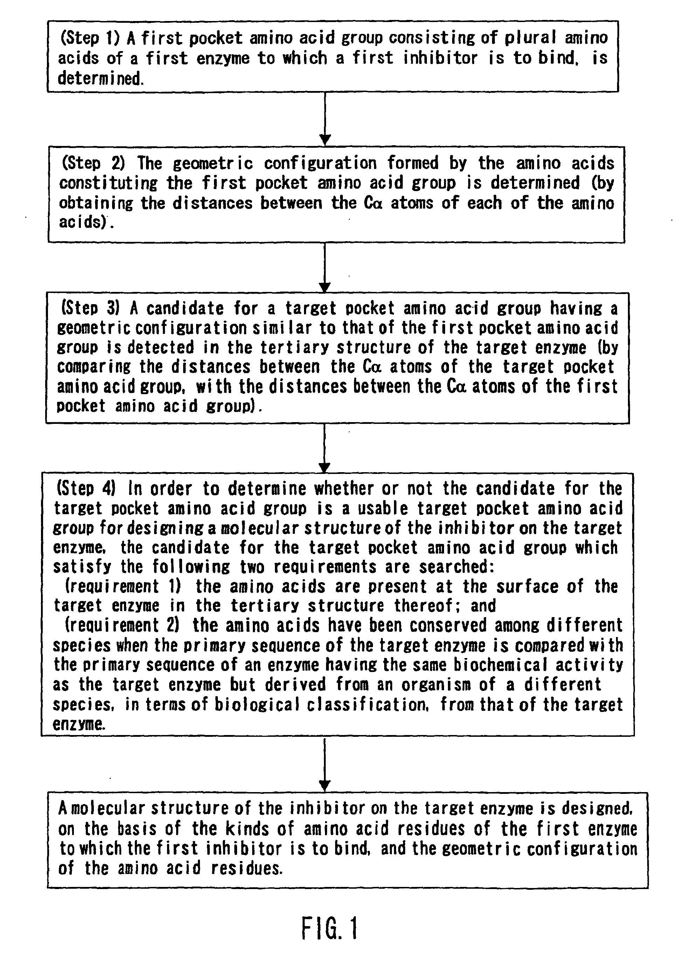

FIG. 1 is a block diagram showing the scheme of each process of the first method of the present invention.

The first method of the present invention is a method of designing a molecular structure of an inhibitor on a target enzyme whose secondary and tertiary structures are known, comprising; analyzing, in the tertiary structure of a first enzyme whose primary, secondary and tertiary structures are known, a geometric configuration formed by amino acids to which a first inhibitor having biological, inhibitory activity on the first enzyme is to be bound; and searching, in the target enzyme, amino acids constituting the similar geometric configuration to the geometric configuration that was analyzed in the first enzyme, thereby designing the molecular structure of the inhibitor on the target enzyme.

In the first method of the present invention, the primary, secondary and tertiary structures of the first enzyme used in the step (1) are known. Herein, the expression "the primary, secondary and tertiary structures are known" does not mean that every amino acid is in a state where assignment of its cross peak by NMR has been completed, but rather means that the primary, secondary and tertiary structures of the enzyme have been identified as a whole of the enzyme. In the present invention, it is convenient that using an enzyme whose primary, secondary and tertiary structures have already known. However, it is also possible to apply the first method of the present invention after analyzing the primary, secondary and tertiary structures of an enzyme. The primary, secondary and tertiary structures of an enzyme can be analyzed by employing the techniques known in the art, such as biochemical technique, NMR and X-ray crystal structure analysis, in combination.

One example of the enzyme whose primary, secondary and tertiary structures have been identified is rat DNA polymerase β (hereinafter also referred to as "rat pol. β"). Rat pol. β is the only enzyme whose primary, secondary and tertiary structures are known, among the DNA polymerase group which represent the enzymes that are essential to living organisms and catalyze the reaction of synthesizing DNA as the main body of genes.

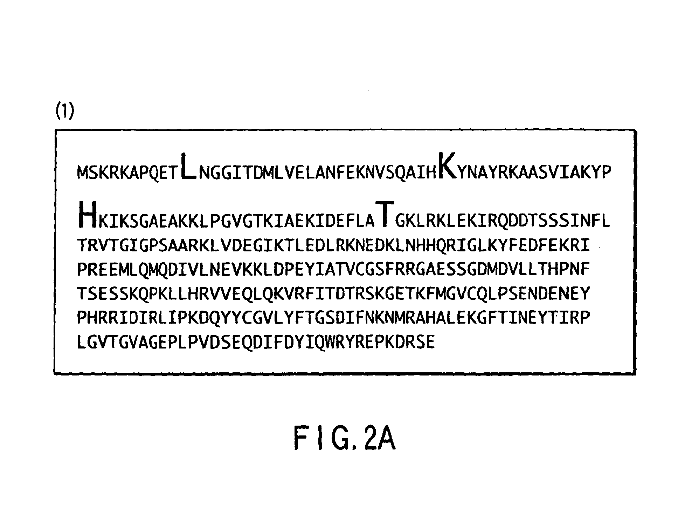

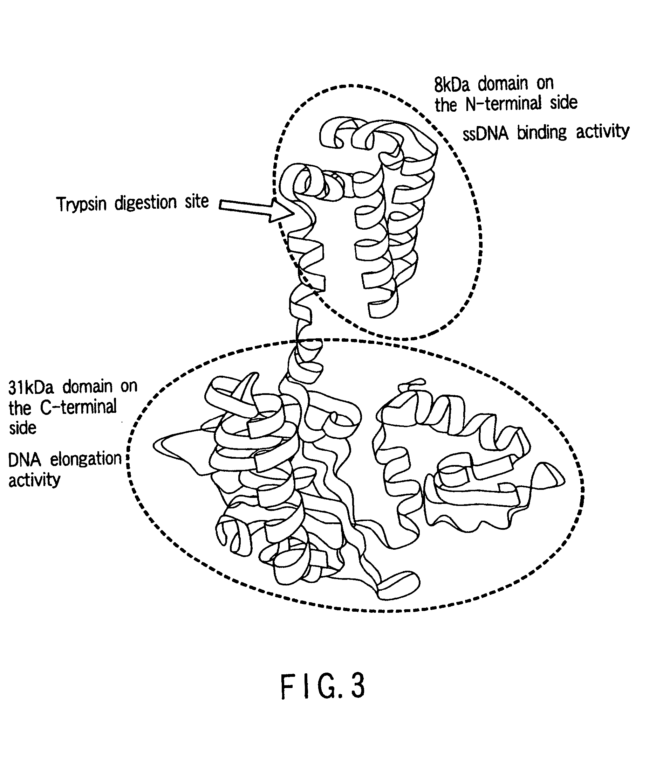

The amino acid sequence of rat pol. β is shown in reference 5, and the tertiary structure thereof (including the secondary structure) is shown in references 6 and 7. FIG. 2A shows the primary sequence of amino acid residues of rat pol. β (refer to the SQE ID No. 1 of the Sequence Listing described below). The amino acid sequence is available from SWISS-Plot Date Base. FIG. 3 is a schematic view showing the tertiary structure of rat pol. β, in which the principal chain of the amino acids is represented as a ribbon.

As shown in FIG. 3, rat pol. β is a single peptide constituted of the 8 kDa domain (1-87 amino acid residues) on the N-terminal side and the 31 kDa domain (88-335 amino acid residues) on the C-terminal side, in which the 31 kDa domain is connected with the 8 kDa domain. The 8 kDa domain can be cut off from the 31 kDa domain by mild hydrolysis with trypsin. Attribution of cross peaks by NMR has been completed in 75 residues of the total 87 amino acid residues of the 8 kDa domain (refer to reference 9).

The first inhibitor on the first enzyme used in the first method of the present invention is preferably an inhibitor whose inhibitory activity (e.g., IC₅₀) is in the order of µM or lower, although the first inhibitor is not particularly restricted thereto. The manner of inhibition may be either competition or non-competition. When the enzyme used in the first method of the present invention is rat pol. β, the first inhibitor is preferable to inhibit, in a competitive manner, the binding of rat pol. β to the DNA strand as the substrate.

Examples of the inhibitor having biochemical inhibitory activity on rat pol. β include nervonic acid (cis-15-tetracosenoic acid) and lithocholic acid (3-α-hydroxy-5β-cholanoic acid).

The chemical formulae of nervonic acid and lithocholic acid are shown below:

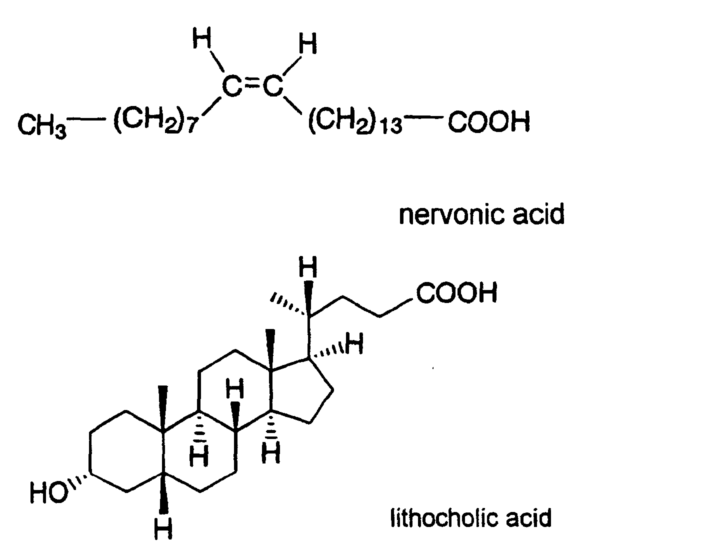

Nervonic acid is one of the fatty acids which most strongly inhibit rat pol. β. Lithocholic acid has been reported by Ogawa et al. (Jpn. J. Cancer Res. 89, 1154-1159, 1998), for example, as a representative example of steroids which are known as inhibitors on DNA polymerase. Both nervonic acid and lithocholic acid are commercially available and can easily be obtained by large amounts.

In the step (1) of the first method of the present invention, a first pocket amino acid group consisting of plural amino acids to which the first inhibitor is to bind, is determined in the tertiary structure of the first enzyme.

The plural amino acids constituting the first pocket amino acid group are decided by measuring the ¹H-¹⁵N HMQC (heteronuclear multiple-quantum correlated spectroscopy) of the first enzyme in the absence of the first inhibitor and the ¹H-¹⁵N HMQC spectroscopy of the first enzyme in the presence of the first inhibitor; overlapping the cross peak of each spectroscopy; and selecting amino acids that satisfy the condition that at least one of the absolute values of ¹H and ¹⁵N chemical shift of the amino acids is equal to or more than a specific value. Herein, the shift value is the one which can be set in accordance with the type or the like of the enzyme and the inhibitor used in the first method of the present invention. Although the values of ¹H and ¹⁵N chemical shift are generally set such that the number of the amino acid residues to be selected is three to four, the number of the amino acid residues is not restricted thereto. In the present invention, the number of amino acids is set in a range of three to four, because the minimum number of amino acids required to specify a geometric configuration formed by the amino acid residues is three and also the number of amino acids to be selected should not be uselessly enlarged in order to avoid making the identification task of the geometric configuration complicated.

The ¹H-¹⁵N HMQC spectroscopy can be measured by using Varian Unity-Plus 750 spectrometer, manufactured by Varian Co., Ltd. The measurement condition of the ¹H-¹⁵N HMQC spectroscopy may be set according to reference 4 listed below.

The amount of the inhibitor to be added when the ¹H-¹⁵N HMQC spectroscopy is measured in the presence of the inhibitor may be set by biochemically analyzing, in advance, the amount of the inhibitor which is required for inhibiting the enzymatic activity.

In the first method of the present invention, when rat pol. β and nervonic acid are used as the combination of the first enzyme and the fist inhibitor, respectively, the amount of nervonic acid to be added may be equimolar to the amount of rat pol. β. The absolute values of ¹H and ¹⁵N chemical shift as the indices may be set at 0.06 ppm or larger and 0.4 ppm or larger, respectively. In the amino acid sequence of rat pol. β, the amino acid residues that satisfy such ¹H or ¹⁵N chemical shift, are the following four amino acid residues: Leu-11, Lys-35, His-51 and Thr-79.

Further, in the first method of the present invention, when rat pol. β and lithocholic acid are used as the combination of the first enzyme and the fist inhibitor, respectively, the amount of lithocholic acid to be added may be equimolar to the amount of rat pol. β. The absolute values of ¹H and ¹⁵N chemical shift as the indices may be set at 0.06 ppm or larger and 0.6 ppm or larger, respectively. In the amino acid sequence of rat pol. β, the amino acid residues that satisfy such ¹H or ¹⁵N chemical shift are the following three amino acid residues: Lys-60, Leu-77 and Thr-79.

In the step (2) of the first method of the present invention, the geometric configuration of the first pocket amino acid group selected in the step (1) is determined. Specifically, the determination of the geometric configuration is carried out by obtaining, in the tertiary structure of the first enzyme, the distances between the Cα atoms of each of the amino acids constituting the first pocket amino acid group.

The distances between the Cα atoms can be calculated by using Protein Data Bank (Protein data Bank: 1BNO in the case of rat pol. β, and Protein data Bank: 1BJT in the case of yeast topo II), whereby the geometric configuration formed by the Cα atoms of the amino acids constituting the first pocket amino acid group can be analyzed. The technique of this analysis is described in detail in reference 4 listed below.

In the step (3) of the first method of the present invention, one or more candidates for a target pocket amino acid group having a geometric configuration similar to the geometric configuration of the rat DNA polymerase β pocket amino acid group determined in the step (2) is detected in the target enzyme. The detection is carried out by selecting the plural amino acids of the same kinds as the respective amino acids constituting the first pocket amino acid group determined in the step (2). Also, the distances, in the tertiary structure of the target enzyme, between the Cα atoms of each of the selected plural amino acids meet the following condition. That is, when comparison is made between (a) and (b), wherein (a) is the respective distances between the Cα atoms of each of the amino acids constituting the target pocket amino acid group, and (b) is the respective distances between the Cα atoms in the first pocket amino acid group obtained in the step (2), the differences in the corresponding distances between (a) and (b) are within a specific value.

Herein, "the corresponding distances between the Cα atoms of each of the amino acids" means that when the amino acids constituting the first pocket amino acid group are expressed as A, B and C and the amino acids constituting the target pocket amino acid group are expressed as a, b and c, the distance between the Cα atoms of A and B corresponds to the distance between the Cα atoms of a and b; the distance between the Cα atoms of B and C corresponds to the distance between the Cα atoms of b and c; and the distance between the Cα atoms of A and C corresponds to the distance between the Cα atoms of a and c. The amino acids A and a are the same kind to each other. Similarly, the amino acids B and b are the same kind, and the amino acids C and c are the same kind.

The differences in the corresponding distances between the Cα atoms of each of the amino acids may generally be set within a range of ±2 x 10⁻¹ nm, although the range may be changed in accordance with the type or the like of the first enzyme, the target enzyme and the first inhibitor used. It should be noted, however, that a too large difference in the corresponding distances between the Cα atoms of each of the amino acids represents a large difference between the geometric configuration formed by the amino acid residues constituting the first pocket amino acid group and that formed by the amino acid residues constituting the target pocket amino acid group, which is not preferable because the precision of the geometric configuration of the inhibitor on the target enzyme will then deteriorate.

In the first method of the present invention, when rat pol. β, nervonic acid and HIV reverse transcriptase are used as the first enzyme, the first inhibitor and the target enzyme, respectively, Leu, His, Thr and Lys having a geometric configuration similar to that of Leu-11, Lys-35, His-51 and Thr-79 of rat pol. β are Lys-65, Leu-100, His-235 and Thr-386 of HIV reverse transcriptase, which was decided as the candidate for a target pocket amino acid.

In the step (4) of the first method of the present invention, it is determined by searching whether or not the candidate for a target pocket amino acid group detected in the step (3) is usable for designing a molecular structure of an inhibitor on the target enzyme.

One feature of the first method of the present invention resides in this searching method. Specifically, the search is conducted on the basis of whether or not the amino acids constituting the candidate for a target pocket amino acid group determined in the step (3) satisfy the following two requirements:
(requirement 1) the amino acids are those present at the surface of the target enzyme in the tertiary structure thereof; and
(requirement 2) the amino acids are those that have been conserved among species, when a comparison is made between the primary sequence of the amino acids of the target enzyme and the primary sequence of the amino acids of another enzyme having the same biochemical activity as the target enzyme but derived from an organism of a different species from that of the target enzyme, in terms of biological classification.

When the amino acids of the candidate for a target pocket amino acid group determined in the step (3) satisfy the above two requirements, it is determined that the candidate(s) for a target pocket amino acid group is (are) usable for designing a molecular structure of an inhibitor on the target enzyme.

In requirement 1 of the step (4), it is required that the amino acids are those present at the surface of the target enzyme in the tertiary structure thereof, because it is presumed that such a pocket amino acid group must be present at the surface of the target enzyme in order for a specific substance (an inhibitor) to bind to the pocket amino acid group and inhibit the enzymatic activity of the target enzyme.

Further, in requirement 2 of the step (4), it is required that the amino acids are those that have been conserved among species, when a comparison is made between the primary sequence of the amino acids of the target enzyme and the primary sequence of the amino acids of another enzyme having the same biochemical activity as the target enzyme but derived from an organism of a different species from that of the target enzyme, in terms of biological classification. The reason thereof is that in a living organism, when amino acids which are essential for maintaining the activity of an enzyme (i.e., amino acids which are directly involved in the activity of the enzyme) are changed, the enzyme decreases its activity and, as a result, the living organism dies. That is, the requirement 2 is on the basis of an idea that the amino acids which play important roles in enzymatic activities will probably be maintained throughout the process of evolution of the organism.

Herein, the term "biological classification" represents genetic classification on the basis of the evolution theory of living organisms including virus. Accordingly, examples of "enzyme derived from an organism of a different species, in terms of biological classification" include enzymes derived from organisms belonging to different kingdoms, e.g., human (animal kingdom) vs. pea (plant kingdom), human (animal kingdom) vs. yeast (fungus kingdom), or enzymes derived from organisms of different species in the same animal kingdom (such as rat and mouse). In the case of virus, examples of "enzyme derived from an organism of a different species, in terms of biological classification" include enzymes derived from viruses whose hosts are of different species, in terms of biological classification (e.g., HIV vs. monkey immunodeficiency virus) and enzymes derived from viruses whose hosts are of the same species but whose genetic types are different (e.g., HIV-1 vs. HIV-2).

In the first method of the present invention, it is preferable that the search is carried out with respect to species which are as distant, in terms of both genetics and the evolution theory, as possible.

The technique which determines by searching whether or not any homology in amino acid sequence exists between a variety of different organisms has been practiced in the fields of molecular biology, information biology and the like. The present invention, however, has applied, for the first time, this technique in the field of molecular design.

It is preferable that plural types of enzyme are used in requirement 2 of the step (4), which enzymes are derived from an organism of a different species, in terms of biological classification, from the organism from which the target enzyme is derived. It is more preferable that at least ten enzymes from different species are searched. The larger the number of the enzymes, the higher precision is resulted in search, which is preferable.

With regards to the judgment on whether or not the amino acid is conserved in different species, the sample which exhibits homology of no less than 80%, in general, and preferably homology of 100%, is regarded as having "conserved amino acid". It is preferable that as many amino acids as possible, of those constituting the pocket amino acid group, are the amino acids exhibiting 100% homology between the species. However, it is not necessary that all of the amino acids constituting the pocket amino acid group are the amino acids exhibiting 100% homology between the species. It suffices that at least one of the amino acids constituting the pocket amino acid group is the amino acid exhibiting 100% homology between the species.

Whether or not the amino acid satisfies the two requirements defined in the step (4) can be determined by using a computer software which is commercially available (INSIGHT II/BINDING SITE ANALYSIS, manufactured by MSI (Molecular Simulations Inc., U.S.A.)).

In the first method of the present invention, a molecular structure of a novel inhibitor on the target enzyme is designed, on the basis of the kinds of amino acid residues of the first enzyme, to which the first inhibitor is to bind, and the three-dimensional configuration of the amino acid residues.

Specifically, designing of a molecular structure of a novel inhibitor on the target enzyme is carried out by using the method including: specifying the substituent groups of the first inhibitor, which substituent groups are to bind to the amino acids constituting the first pocket amino acid group; adjusting the distances between each of the substituent groups and the three-dimensional configuration thereof such that these substituent groups are configured so that they fit in the geometric configuration formed by the first pocket amino acid group. The types of the substituent groups which are to bind to the amino acid residues constituting the first pocket amino acid group can be specified by using a biochemical method or the like which is known to those skilled in the art.

As described above, the first method of the present invention enables a molecular structure design of an inhibitor on an enzyme whose tertiary structure is determined and whose enzymatic activity is different from that of the first enzyme. Further, as a modification of the first method of the present invention, it is possible to design a molecular structure of another novel inhibitor on the first enzyme.

Specifically, the novel inhibitor on the first enzyme may be designed so that it has substituent groups that can be bound more firmly to the amino acids in the geometric configuration identified with the first enzyme in the steps (1) and (2) of the first method of the present invention and that accords the geometric configuration of the first enzyme.

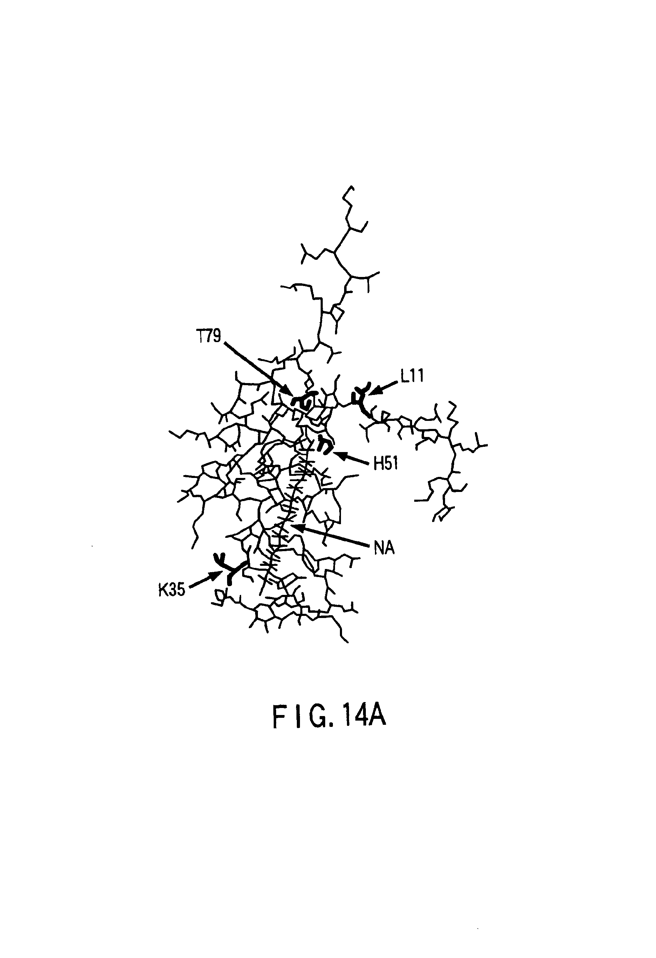

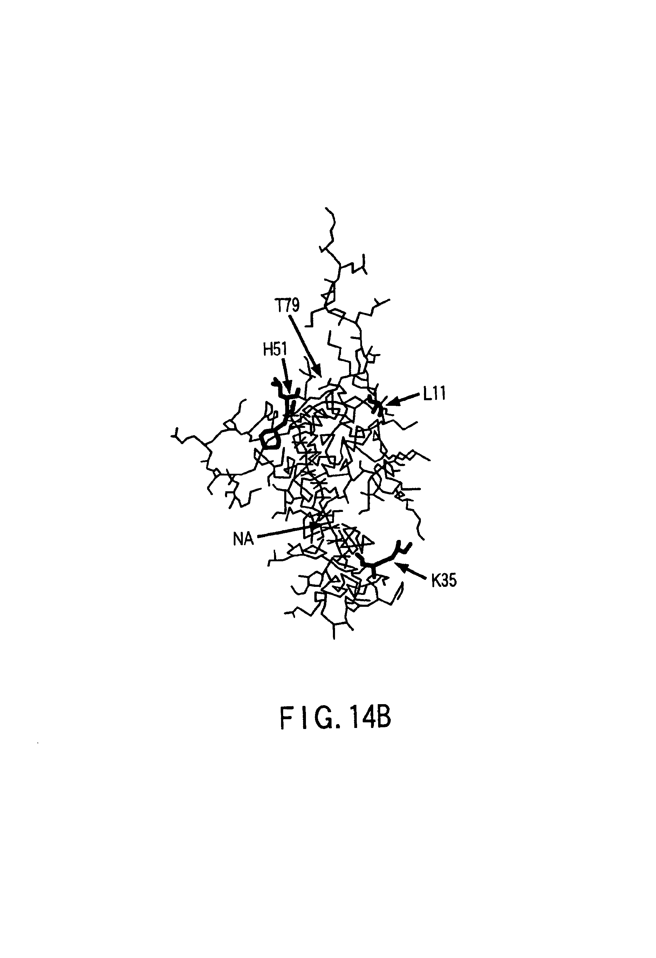

More specifically, in the binding between the amino acids, Leu-11, Lys-35, His-5 and Thr-79, of rat pol. β and nervonic acid, the carboxyl group at one terminal end of the nervonic acid chain is to bind to the hydrophilic site of Lys-35, and the methyl group at the other terminal end of the nervonic acid chain is to bind to Leu-11, His-51 and Thr-79, as shown in FIG. 14. The bent structure of the nervonic acid chain due to the double bond therein allows the nervonic acid chain to be fitted in the three-dimensional hydrophobic pocket formed by Leu-11, Lys-35, His-51 and Thr-79 of rat pol. β.

Accordingly, a molecular structure of a novel inhibitor other than nervonic acid, on rat pol. β, can be designed by adjusting the length of a fatty acid chain and the position of double bond so that the thus adjusted fatty acid is fitted in the three-dimensional pocket formed by Leu-11, Lys-35, His-51 and Thr-79 of rat pol. β in a more fitting manner.

Next, the second method of the present invention will be described in more detail.

The second method of the present invention is a method of designing a molecular structure of an inhibitor on a target enzyme whose tertiary structure is unknown, comprising: analyzing, in the tertiary structure of a first enzyme whose primary, secondary and tertiary structures are known, a geometric configuration formed by amino acids to which a first inhibitor having biological, inhibitory activity on the first enzyme is to be bound; and searching, in a second enzyme whose primary, secondary and tertiary structures are known, amino acids constructing the similar geometric configuration to the geometric configuration that was analyzed in the first enzyme; thereby designing the molecular structure of the inhibitor on the target enzyme.

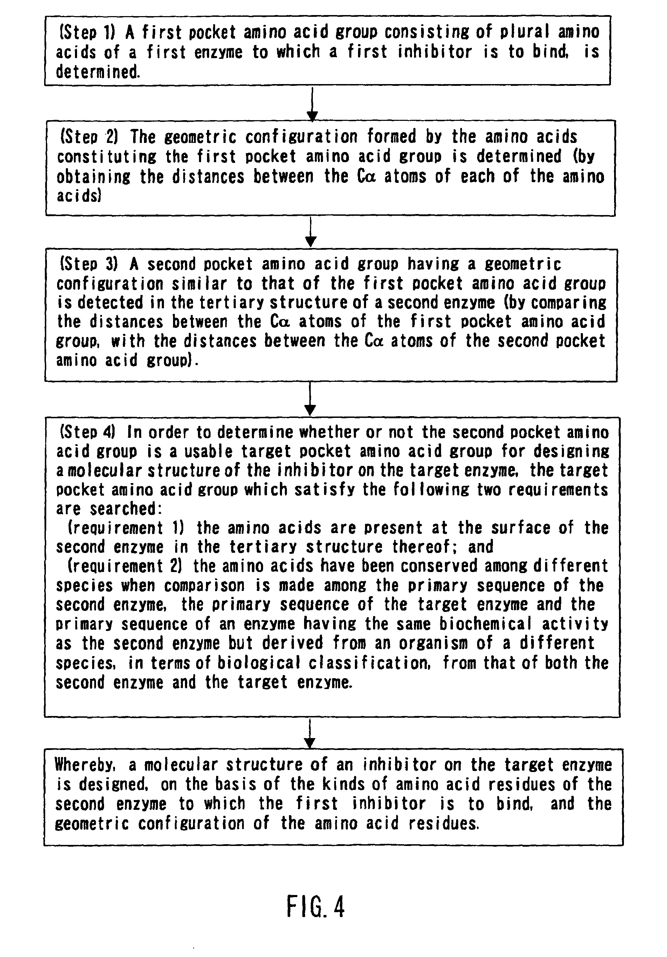

FIG. 4 is a block diagram showing the scheme of each process of the second method of the present invention.

The second method of the present invention is similar to the first method of the present invention, in that the second method employs basically the same technique utilized in the steps (1) to (4) of the latter.

That is, the first method of the present invention is to design a molecular structure of an inhibitor on a second enzyme, i.e., the target enzyme whose primary, secondary and tertiary structure have been identified, by utilizing the geometric configuration of a first enzyme. The second method of the present invention employs similar techniques on two types of enzymes, as in the first method, however, the second method is different from the first method in that the former then is to design a molecular structure of an inhibitor on a third enzyme, i.e., the target enzyme whose tertiary structure is unknown.

Herein, "a target enzyme" represents, as described above, an enzyme to which a molecular structure of an inhibitor is to be designed by implementing the methods of the present invention.

An enzyme generally has a relatively large molecular weight and a relatively complicated three-dimensional structure. Therefore, it is often extremely difficult in terms of technology to determine the tertiary structure, although the primary and secondary structures thereof can somehow be identified. Accordingly, in a group of enzymes that have the same biochemical activity but that exist in different organisms, there may be a case in which the tertiary structures of amino acids constituting the enzymes existing in some kinds of organism are known, while those of the enzymes existing in the rest of the organisms remain unknown. For example, DNA topoisomerase II, topo II, is an enzyme that enables breaking and reconnecting the double strand DNA, thereby eliminating twisting of DNA. Topo II is thus possessed by all the eucaryotes having a capacity of changing topology of DNA. Only the primary sequence of the amino acid of human topo II has been identified, although the amino acid sequence of topo II of yeast (S. cerevisiae) has been identified up to the tertiary structure level (reference 14 and reference 15 listed below). The second method of the present invention can be used for efficiently designing a molecular structure of an inhibitor on such an enzyme whose tertiary structure is unknown.

As described above, topo II is an enzyme capable of breaking and reconnecting the double strand DNA, thereby eliminating twisting of DNA and through which changing the topology of DNA. Since such a change in topology is essential in the development of cell division, the inhibitor on human topo II is likely to be applicable as an anticancer drug.

Next, the three types of enzymes, i.e., a first enzyme, a second enzyme and a target enzyme, employed in the second method of the present invention will be described in detailed below.

The structure of first and second enzymes have been identified up to the tertiary structural level. On the contrary, the target enzyme, at least the primary sequence of the amino acid thereof has been identified, but the tertiary structure thereof remains unknown. The first enzyme has biochemical activity of different type from that of the second and target enzymes. The second and target enzymes have biochemical activity of the same type, but they are derived from organisms of biologically different types to each other. The second method of the present invention can be used for designing a molecular structure of an inhibitor on an enzyme, i.e., the target enzyme, which, due to the large molecular weight thereof, does not allow structural analysis by NMR.

A single type of inhibitor is employed against the enzyme used in the second method of the present invention. The inhibitor is capable of inhibiting the activity of the first enzyme. This inhibitor will be referred to as "the first inhibitor" hereinafter.

Specific examples of the combination of the first, second and target enzymes, and the first inhibitor, include the combination of rat pol. β, yeast topo II and human topo II, and nervonic acid; and the combination of rat pol. β, yeast topo II, and human topo II, and lithocholic acid.

In the second method of the present invention, the term "pocket amino acid group" is used for each of the first, second and target enzymes. As described below, plural amino acids, in the enzyme, satisfying a specific condition, is allocated to each of the pocket amino acid groups.

In the first enzyme used in the step (1) of the second method of the present invention, the primary, the secondary and the tertiary structures are known. Herein, the expression "the primary, secondary and tertiary structures are known" does not mean that every amino acid is in a state where assignment of its cross peak by NMR has been completed, but rather means that the primary, secondary and tertiary structures of the enzyme have been identified as a whole of the enzyme.

In the present invention, it is convenient that using an enzyme whose primary, secondary and tertiary structures have already known. However, it is also possible to apply the second method of the present invention after analyzing the primary, secondary and tertiary structures of an enzyme. The primary, secondary and tertiary structures of an enzyme can be analyzed by employing the techniques known in the art, such as biochemical technique, NMR and X-ray crystal structure analysis, in combination.

One example of the enzyme whose primary, secondary and tertiary structures have been identified is rat pol. β. It is the only one enzyme whose primary, secondary and tertiary structures are known, among the DNA polymerase group which represent the enzymes that are essential to living organisms and catalyze the reaction of synthesizing DNA as the main body of genes.

The first inhibitor on the first enzyme used in the second method of the present invention is preferably an inhibitor whose inhibitory activity (e.g., IC₅₀) is in the order of µM or lower, although the first inhibitor is not particularly restricted thereto. The manner of inhibition may be either competition or non-competition. When the enzyme used in the second method of the present invention is rat pol. β, the first inhibitor is preferable to inhibit, in a competitive manner, the binding of rat pol. β to the DNA strand as the substrate.

Examples of the inhibitor having biochemical inhibitory activity on rat pol. β include nervonic acid and lithocholic acid.

In the step (1) of the second method of the present invention, a first pocket amino acid group consisting of plural amino acids to which the first inhibitor is to bind, is determined in the tertiary structure of the first enzyme.

The plural amino acids constituting the first pocket amino acid group are decided by measuring the ¹H-¹⁵N HMQC of the first enzyme in the absence of the first inhibitor and the ¹H-¹⁵N HMQC spectroscopy of the first enzyme in the presence of the first inhibitor; overlapping the cross peak of each spectroscopy; and selecting amino acids that satisfy the condition that at least one of the absolute values of ¹H and ¹⁵N chemical shift of the amino acids is equal to or more than a specific value. Herein, the shift value is the one which can be set in accordance with the type or the like of the enzyme and the inhibitor used in the second method of the present invention. Although the values of ¹H and ¹⁵N chemical shift are generally set such that the number of the amino acid residues to be selected is three to four, the number of the amino acid residues is not restricted thereto. In the present invention, the number of amino acids is set in a range of three to four, because the minimum number of amino acids required to specify a geometric configuration formed by the amino acid residues is three and also the number of amino acids to be selected should not be uselessly enlarged in order to avoid making the identification task of the geometric configuration complicated.

The ¹H-¹⁵N HMQC spectroscopy can be measured by using Varian Unity-Plus 750 spectrometer, manufactured by Varian Co., Ltd. The measurement condition of the ¹H-¹⁵N HMQC spectroscopy may be set according to reference 4 listed below.

The amount of the inhibitor to be added when the ¹H-¹⁵N HMQC spectroscopy is measured in the presence of the inhibitor may be set by biochemically analyzing, in advance, the amount of the inhibitor which is required for inhibiting the enzymatic activity.

In the second method of the present invention, when rat pol. β and nervonic acid are used as the combination of the first enzyme and the fist inhibitor, respectively, the amount of nervonic acid to be added may be equimolar to the amount of rat pol. β. The absolute values of ¹H and ¹⁵N chemical shift as the indices may be set at 0.06 ppm or larger and 0.4 ppm or larger, respectively. In the amino acid sequence of rat pol. β, the amino acid residues that satisfy such ¹H or ¹⁵N chemical shift, are the following four amino acid residues: Leu-11, Lys-35, His-51 and Thr-79.

Further, in the second method of the present invention, when rat pol. β and lithocholic acid are used as the combination of the first enzyme and the fist inhibitor, respectively, the amount of lithocholic acid to be added may be equimolar to the amount of rat pol. β. The absolute values of ¹H and ¹⁵N chemical shift as the indices may be set at 0.06 ppm or larger and 0.6 ppm or larger, respectively. In the amino acid sequence of rat pol. β, the amino acid residues that satisfy such ¹H or ¹⁵N chemical shift are the following three amino acid residues: Lys-60, Leu-77 and Thr-79.

In the step (2) of the second method of the present invention, the geometric configuration of the first pocket amino acid group selected in the step (1) is determined. Specifically, the determination of the geometric configuration is carried out by obtaining, in the tertiary structure of the first enzyme, the distances between the Cα atoms of each of the amino acids constituting the first pocket amino acid group.

The distances between the Cα atoms can be calculated by using Protein Data Bank (Protein data Bank: 1BNO in the case of rat pol. β, and Protein data Bank: 1BJT in the case of yeast topo II), whereby the geometric configuration formed by the Cα atoms of the amino acids constituting the first pocket amino acid group can be analyzed. The technique of this analysis is described in detail in reference 4 listed below.

In the step (3) of the second method of the present invention, a second pocket amino acid group having a geometric configuration similar to the geometric configuration of the first pocket amino acid group identified in the step (2) is detected in the second enzyme. The second enzyme used in the step (3) satisfies the following requirements:
(requirement 1) the second enzyme has the same biochemical activity as the target enzyme;
(requirement 2) the organism from which the second enzyme is derived, belongs to a different type, in terms of biological classification, from the organism from which the target enzyme is derived;
(requirement 3) the primary, secondary and tertiary structures of the second enzyme are known; and
(requirement 4) the second enzyme is biochemically inhibited by the first inhibitor.

The expression that "(an enzyme) has the same biochemical activity as the target enzyme" defined in requirement 1 of the step (3) means that the enzyme catalyzes, in the enzymatic reaction thereof, substantially the same substrate as the target enzyme does, thereby generating substantially the same product in the biochemical reaction.

The expression that "the organism from which the second enzyme is derived belongs to a different type, in terms of biological classification, from the organism from which the target enzyme is derived" defined in requirement 2 of the step (3) represents the same meaning as described in the first method. Further, as is in the first method, it is preferable that the organism from which the second enzyme is derived, is as distant as possible, in terms of biological classification, from the organism from which the target enzyme is derived.

With regards to " the primary, secondary and tertiary structure of an enzyme" defined in requirement 3 of the step (3), it is convenient that an enzyme whose primary, secondary and tertiary structures are already known is used, as described above. However, it is possible to apply the second method of the present invention after analyzing the primary, secondary and tertiary structures of an enzyme. The primary, secondary and tertiary structures of an enzyme can be analyzed by employing the techniques known in the art, such as biochemical technique, NMR and X-ray crystallographic analysis, in combination.

The expression that "(the second enzyme) is biochemically inhibited by the first inhibitor" defined in requirement 4 of the step (3) represents that the value of the inhibitory activity IC₅₀ of the first inhibitor effected against the second enzyme is preferably in a range 10 times to 1/10 times as much as, and more preferably substantially the same as the value of the inhibitory activity IC₅₀ of the first inhibitor against the first enzyme.

In the step (3) of the second method of the present invention, a second pocket amino acid group having a geometric configuration similar to that of the first pocket amino acid group is detected in the second enzyme. The detection is carried out by selecting the plural amino acids of the same kinds as the respective amino acids constituting the first pocket amino acid group determined in the step (2). Also, the distances, in the tertiary structure of the second enzyme, between the Cα atoms of each of the selected plural amino acids meet the following condition. That is, when comparison is made between (a) and (b), wherein (a) is the respective distances between the Cα atoms of each of the amino acids constituting the second pocket amino acid group, and (b) is the respective distances between the Cα atoms in the first pocket amino acid group obtained in the step (2), the differences between (a) and (b), i.e., the corresponding distances between the Cα atoms of each of the amino acids, are within a specific value.

Herein, "the corresponding distances between the Cα atoms of each of the amino acids" has the same meaning as described in the first method of the present invention.

The corresponding distances between the Cα atoms of each of the amino acids may generally be set within a range of ±2 × 10⁻¹ nm, although the range may be changed in accordance with the type or the like of the first enzyme, the second enzyme and the first inhibitor used. It should be noted, however, if the large distance may be set, it makes difference large between the geometric configurations of the first pocket amino acid group and the second pocket amino acid group. It is not preferable because the precision of the geometric configuration of the inhibitor on the target enzyme will then deteriorate.

When rat pol. β, yeast topo II, human topo II and nervonic acid are used as the first, second, target enzymes and the first inhibitor, respectively, the specific value may be set within a range of ±2 × 10⁻¹ nm. Similarly, the specific value may be set within the same range when lithocholic acid is used as the first inhibitor.

In the step (4) of the second method of the present invention, it is determined by searching whether or not the second pocket amino acid group determined in the step (3) is usable for designing a molecular structure of an inhibitor of the target enzyme.

One of the features of the second method of the present invention resides in the searching method. Specifically, the search is conducted on the basis of whether or not the second pocket amino acid group satisfies the following two requirements:
(requirement 1) the amino acids are those present at the surface of the target enzyme in the tertiary structure thereof; and
(requirement 2) the amino acids are those that have been conserved among species, when a comparison is made among the primary sequence of the amino acids of the second enzyme, the primary sequence of the amino acids of the target enzyme and the primary sequence of the amino acids of another enzyme having the same biochemical activity as the second enzyme but derived from an organism of a different species from that of both the second and target enzymes, in terms of biological classification.

When the amino acids of the second pocket amino acid group satisfy the two requirements, it is determined that the second pocket amino acid group are usable for designing a molecular structure of an inhibitor on the target enzyme.

The reason why the two requirements are employed in the step (4) is the same as that described in the first method.

With regards to the number of the enzyme derived from different species, in terms of biological classification used in requirement 2 of the step (4), at least two enzymes (preferably at least ten enzymes) are searched. The larger the number of the enzyme, the higher precision is resulted in search, which is preferable.

With regards to the judgment on whether or not the amino acid is conserved in different species, the sample which exhibits homology of no less than 80%, in general, and preferably homology of 100%, is regarded as "conserved amino acid". It is preferable that as many amino acids as possible, of those constituting the pocket amino acid group, are the amino acids exhibiting 100% homology between the species. However, it is not necessary that all of the amino acids constituting the pocket amino acid group are the amino acids exhibiting 100% homology between the species. It suffices that one of the amino acids constituting the pocket amino acid group is the amino acid exhibiting 100% homology between the species.

Whether or not the amino acid residues constituting the second pocket amino acid group determined in the step (3) has a three-dimensional configuration similar to the geometric configuration of the first pocket amino acid group, and whether or not the amino acid residues constituting the second pocket amino acid group satisfy the two requirements defined in the step (4), can be determined by using a computer software which is commercially available (INSIGHT II/BINDING SITE ANALYSIS, manufactured by MSI (Molecular Simulations Inc.)).

When rat pol. β, yeast topo II and human topo II, and nervonic acid are used as the first, second and target enzymes, and the first inhibitor, Thr-596, His-735, Leu-741 and Lys-983 among the amino acid residues constituting the second pocket amino acid group of yeast topo II satisfy each of the requirements in the step (3) and the step (4). On the other hand, when lithocholic acid is used as the first inhibitor, Lys-720, Leu-760 and Thr-791 satisfy these requirements.

In the second method of the present invention, when the second pocket amino acid group of the second enzyme satisfies the two requirements defined in the step (4), it is presumed that the target pocket amino acid group is present in the target enzyme. Specifically, it is presumed that respective amino acid residues constituting the target pocket amino acid group are of the same kinds as those constituting the second pocket amino acid group, and the amino acid residues of the target pocket amino acid group are arranged in a geometric configuration similar to that of the second amino acid group.

In the second method of the present invention, after the presumption as described above is carried out, a molecular structure of an inhibitor on the target enzyme is designed on the basis of the kinds of amino acid residues of the second enzyme, to which the first inhibitor is to bind, and the geometric configuration of the amino acid residues.

With regards to a specific designing method, that described in the first method can be employed.

Next, examples of the present invention will be described hereinafter. It should be noted that the present invention is not restricted to these examples.

### (Example 1)

In this example, rat pol. β, nervonic acid and HIV reverse transcriptase were used as the first enzyme, the first inhibitor and the target enzyme, respectively, for designing a molecular structure of an inhibitor on HIV reverse transcriptase.

### <1-1> The primary sequence of the amino acid residue of rat pol. β is shown in FIG. 2A. The model of the tertiary structure of rat pol. β is shown in FIG. 3.

### <1-2> Measurement of inhibitory activity effected by nervonic acid on rat pol. β

The activity of rat pol. β was measured according to the method described in reference 3 listed below, by using poly(dA)/oligo(dT)12-18 (template DNA) and [³H]-dTTP (nucleotide) as substrates and measuring radiation of [³H]-dTTP incorporated in the presence of nervonic acid of different concentrations.

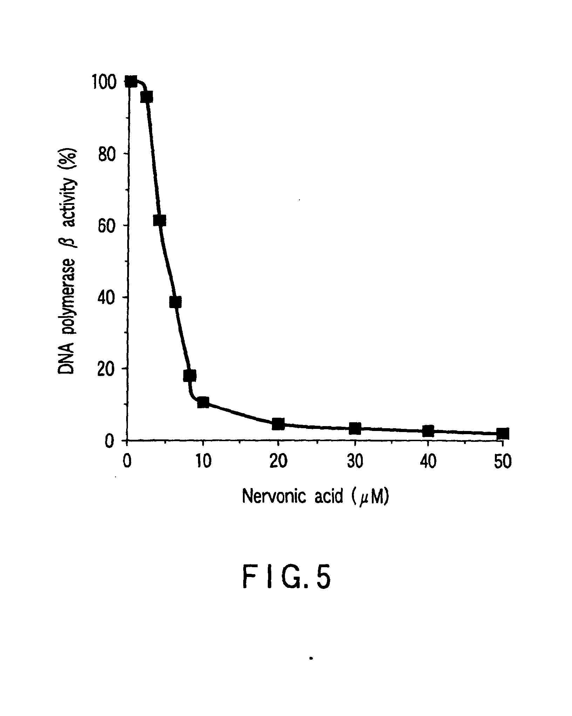

FIG. 5 is a graph showing an inhibition curve. In FIG. 5, the vertical axis represents the activity of rat pol. β and the horizontal axis represents the concentration of nervonic acid.

### <1-3> Measurements of ¹H-¹⁵N HMQC spectroscopy of rat pol. β in the presence and the absence of nervonic acid

A recombinant E. coli having 8 kDa domain of rat pol. β was constructed according to the method described in reference 4 listed below. ¹⁵NH₄Cl as the nitrogen source was added to culture medium of the recombinant E. coli for labeling. In the absence and the presence (1.25 mM, which is equimolar to 8 kDa domain of rat pol. β) of nervonic acid, ¹H-¹⁵N HMQC spectroscopy (750 Mz) was measured by using Varian Unity-Plus 750 spectrometer, manufactured by Varian Co., Ltd. The experiment was conducted only with 8 kDa domain of rat pol. β, because it has been confirmed that the amino acid residues to which nervonic acid is to bind are present only at the 8 kDa domain (references 3 and 4 listed below).

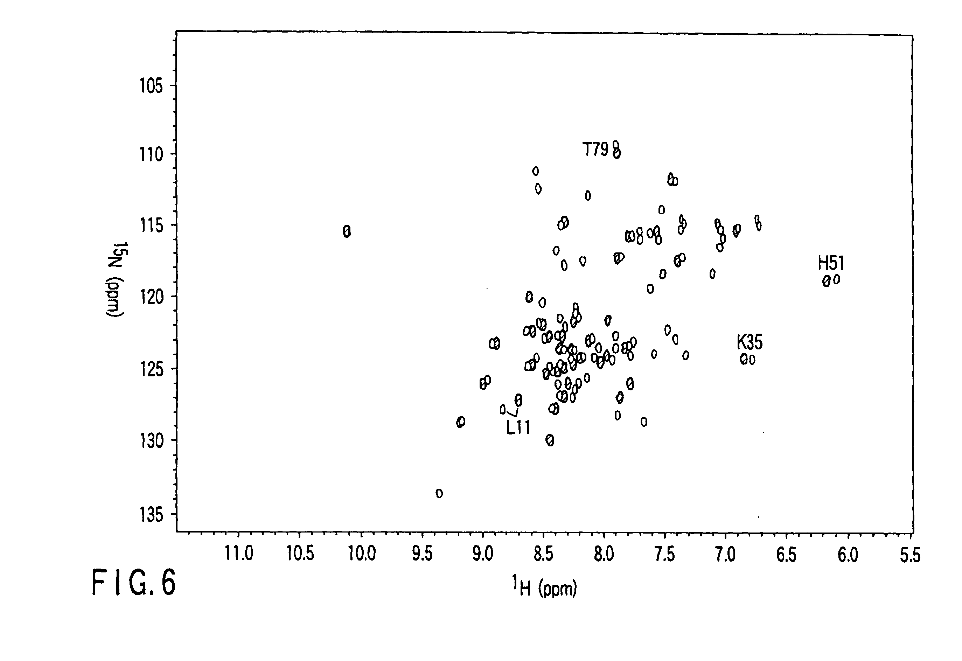

FIG. 6 shows the cross peaks of ¹H-¹⁵N HMQC spectroscopy of rat pol. β. In FIG. 6, the vertical axis represents the shift value of ¹⁵N and the horizontal axis represents the shift value of ¹H. FIG. 6 shows the cross peaks of amino acid residues whose ¹H and ¹⁵N chemical shifts were 0.015 ppm or more and 0.1 ppm or more, respectively, in a manner that the graph of the cross peaks in the presence of nervonic acid and the graph of the cross peaks in the absence of nervonic acid are overlapped. In FIG. 6, among the amino acid residues, those in which the absolute values of ¹H and ¹⁵N chemical shift were 0.06 ppm or more and 0.4 ppm or more, respectively, i.e., four amino acid residues including Leu-11, Lys-35, His-51 and Thr-79, are each indicated by one alphabetic letter representing the type of the amino acid and the sequence number thereof. The group consisting of these four amino acid residues will be referred to as "rat pol. β pocket amino acid group-1" hereinafter.

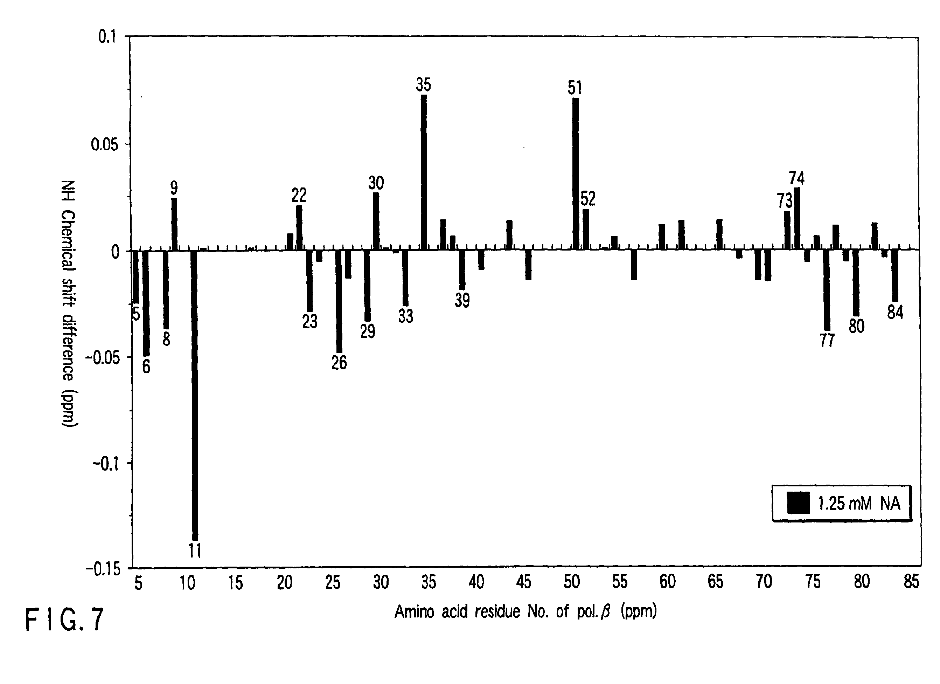

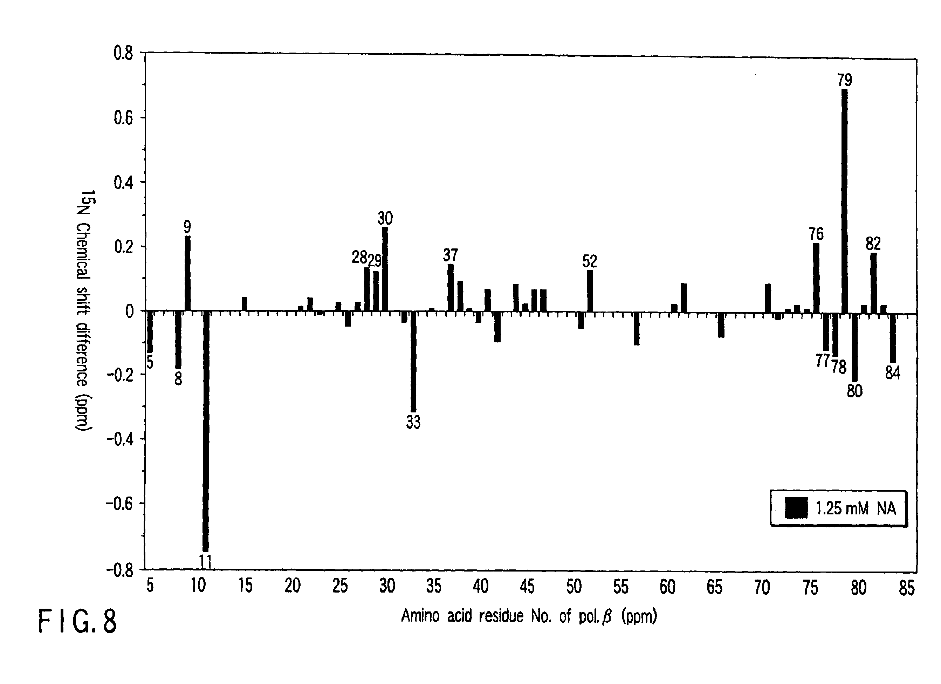

FIG. 7 is a bar graph in which the cross peaks of ¹H-¹⁵N HMQC spectroscopy shown in FIG. 6 is expressed as the relationship of ¹H chemical shift value vs. the amino acid residue number. FIG. 8 is a bar graph in which the cross peaks of ¹H-¹⁵N HMQC spectroscopy shown in FIG. 6 is expressed as the relationship of ¹⁵N chemical shift value vs. the amino acid residue number. In FIGS. 7 and 8, the vertical axis represents the chemical shift value and the horizontal axis represents the sequence number of the amino acid.

### <1-4> Measurement of the distances between the Cα atoms of the rat pol. β pocket amino acid group-1

The distances between the Cα atoms of each of Leu-11, Lys-35, His-51 and Thr-79 constituting the rat pol. β pocket amino acid group-1 obtained in the <1-3> were measured by using Protein Data Bank; lBNO and with reference to references 14, 15 and 16 listed below. The thus obtained distances between the Cα atoms are shown in Table 1.

**Table 1**

| Distance between the Cα atoms of the amino acid residues | Distance (×10⁻¹ nm) |
|---|---|
| Leu-11 and His-51 | 10.41 |
| Leu-11 and Thr-79 | 12.79 |
| Leu-11 and Lys-35 | 27.95 |
| His-51 and Thr-79 | 15.95 |
| His-51 and Lys-35 | 21.87 |
| Thy-79 and Lys-35 | 24.88 |

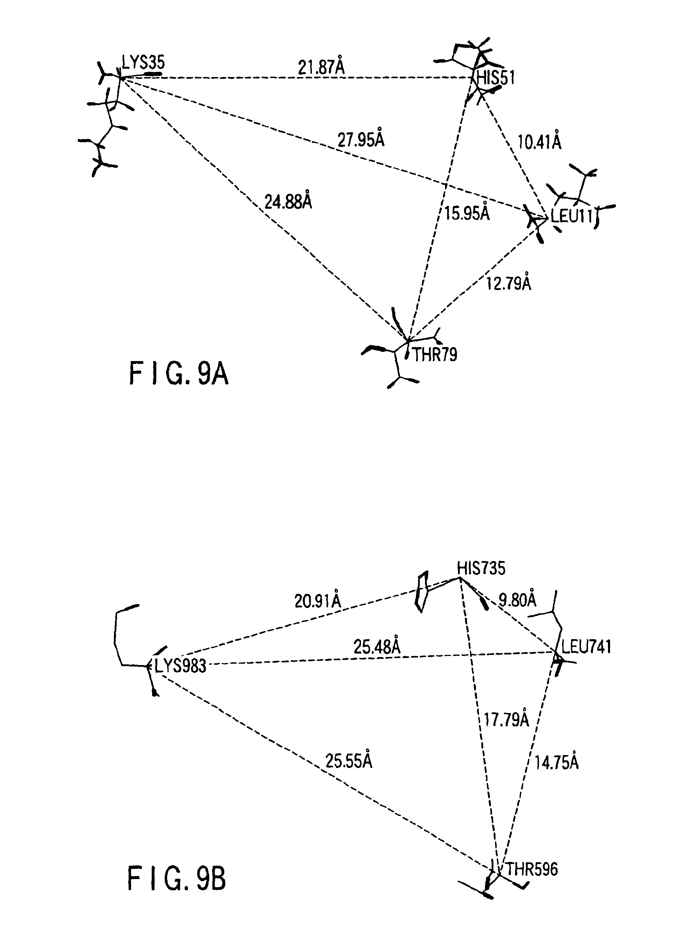

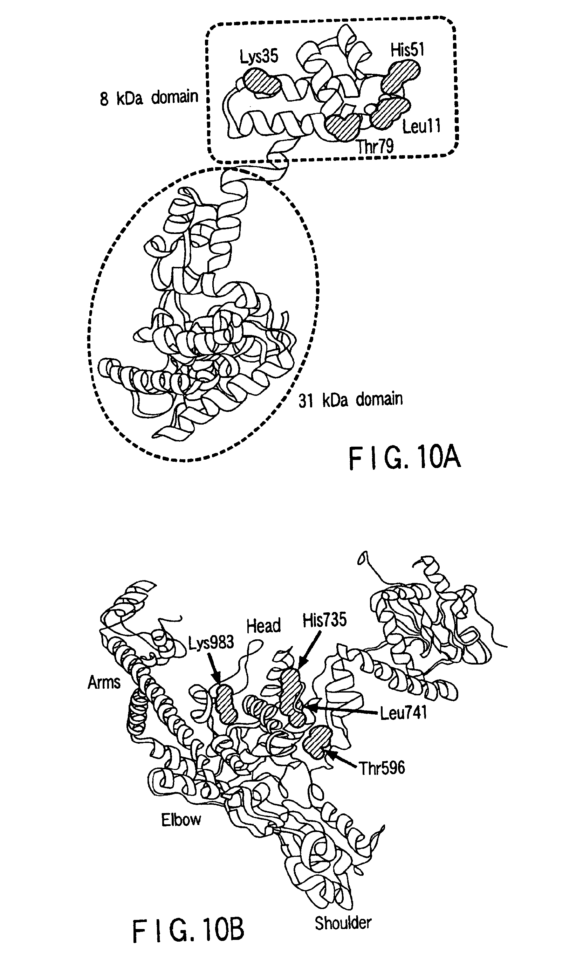

FIG. 9A is a view showing, in three dimensional manner, the geometric configuration of the Cα atoms of the amino acid residues distanced from each other as shown in Table 1 (a computer software (INSIGHT II/BINDING SITE ANALYSIS, manufactured by Molecular Simulations Inc., U.S.A) was employed). Further, FIG. 10A is a view of a model in which the four amino acid residues constituting the rat pol. β pocket amino acid group-1, to which nervonic acid has bound, are indicated in rat pol. β represented as a ribbon.

### <1-5> Determination, by searching, of a target pocket amino acid group on HIV reverse transcriptase

The target pocket amino acid group on HIV reverse transcriptase was searched by the following items 1) to 3) using a computer software (INSIGHT II/BINDING SITE ANALYSIS, manufactured by Molecular Simulations Inc., U.S.A).
Item 1) determination, by searching, of whether or not the target pocket amino acid group (HIV pocket amino acid group-1) corresponding to the rat pol. β pocket amino acid group-1 is present in the amino acid sequence of HIV reverse transcriptase;
Item 2) determination, by searching, of whether or not the amino acids constituting the HIV pocket amino acid group-1 obtained in the above item 1) are present at the surface of HIV reverse transcriptase in the tertiary structure thereof; and
Item 3) determination, by searching, of whether or not the amino acids constituting the HIV pocket amino acid group-1 are the amino acids conserved in immunodeficiency virus reverse transcriptases between other types which derived from different organisms, in terms of biological classification.

The distances between each of the Cα atoms obtained above are shown in Table 2.

**Table 2**

| Distance between the Cα atoms of the amino acid residues | Distance (×10⁻¹ nm) |
|---|---|
| Leu-100 and His-235 | 10.94 |
| Leu-100 and Thr-386 | 16.02 |
| Leu-100 and Lys-65 | 25.22 |
| His-235 and Thr-386 | 19.24 |
| His-235 and Lys-65 | 20.86 |
| Thy-386 and Lys-65 | 21.39 |

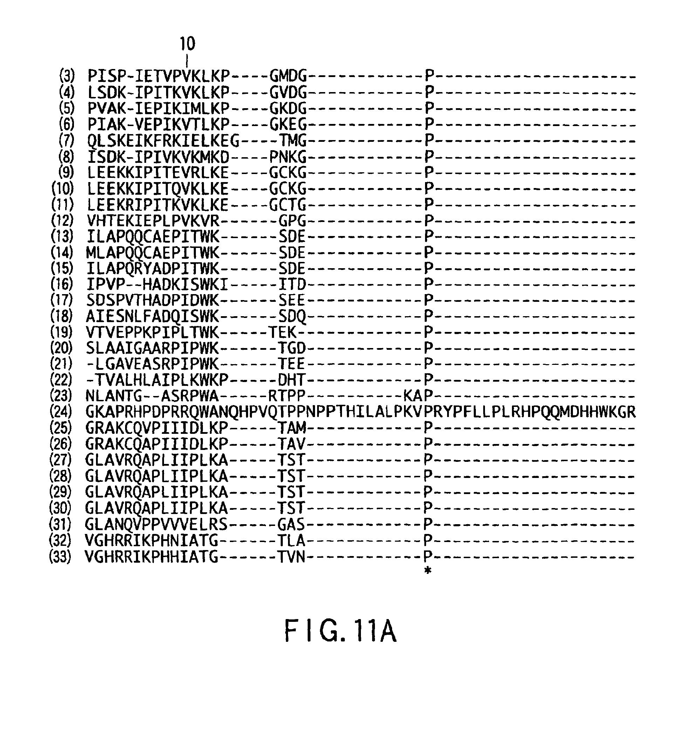

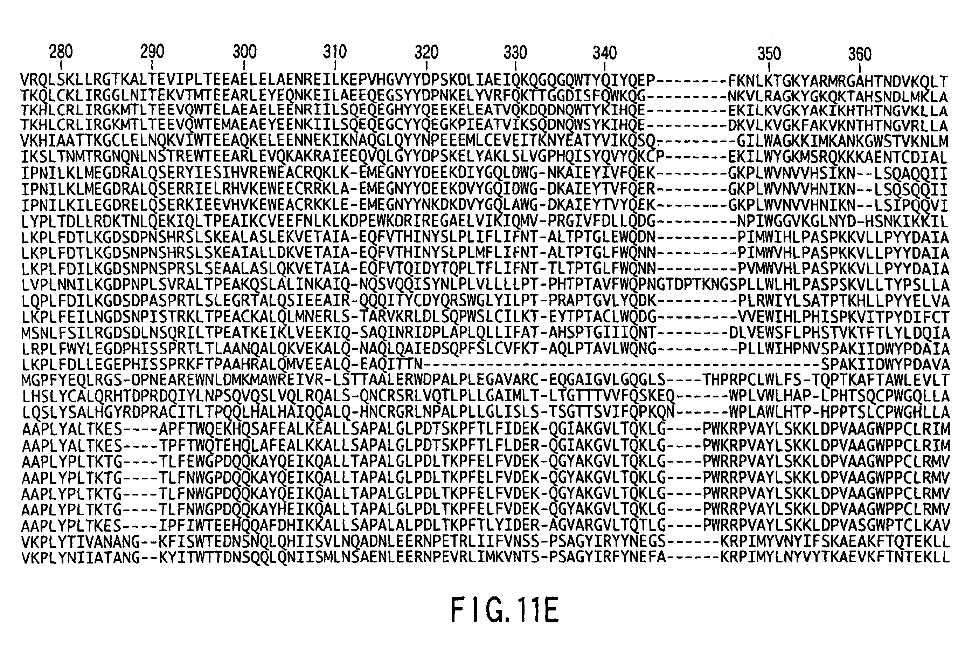

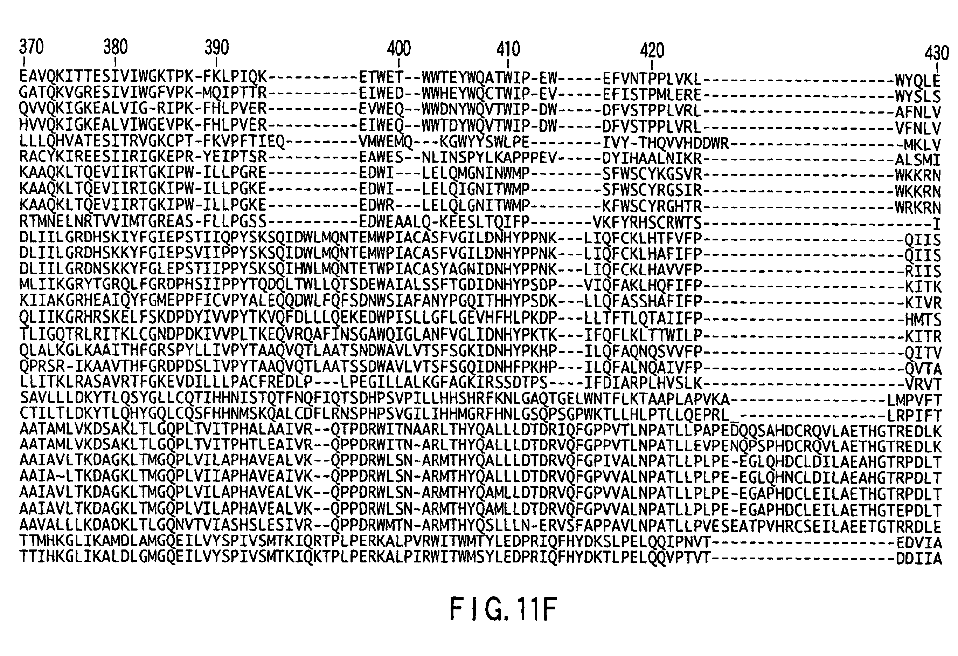

In the searching of the above item 1), the amino acids of HIV reverse transcriptase having such distances between each of the Cα atoms as within a range of ±2 × 10⁻¹nm with respect to the corresponding distances between each of the Cα atoms in pol. β shown in Table 1, were selected. Further, in the searching of the above items 2) and 3), the 31 sequences of the immunodeficiency virus reverse transcriptase each derived from organisms of different species shown in FIGS. 11A-11F were used.

FIGS. 11A-11F shows the 31 sequences of the immunodeficiency virus reverse transcriptase, which correspond to SQE ID Nos. 3 to 33, respectively, of the Sequence Listing shown below. Note that only the amino acids up to No. 100 are shown in the Sequence Listing. Each of the amino acid sequences is available from SWISS-Plot Data Base. In the sequences of FIGS. 11A-11F, a hyphen(s) is (are) inserted between specific amino acids so that conservation of the amino acid sequences among any reverse transcriptases can easily be detected (the INSIGHT II/BINDING SITE ANALYSIS was used).

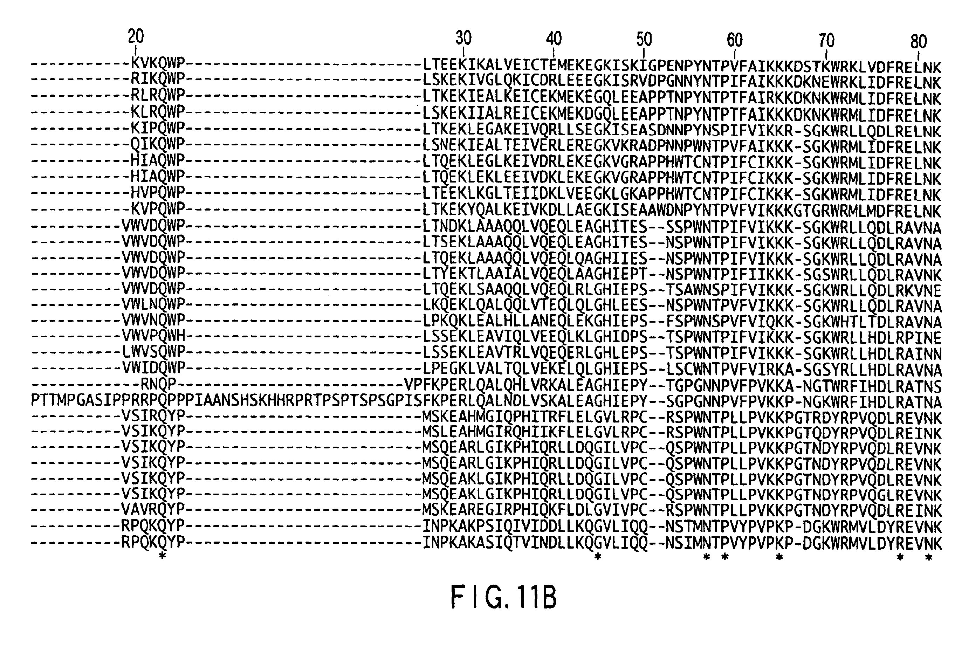

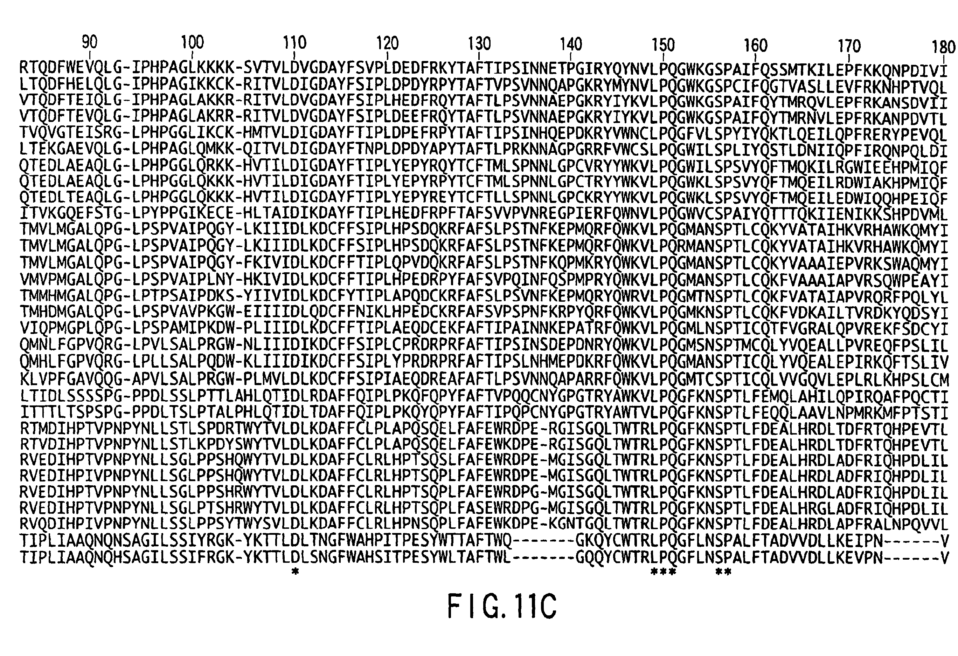

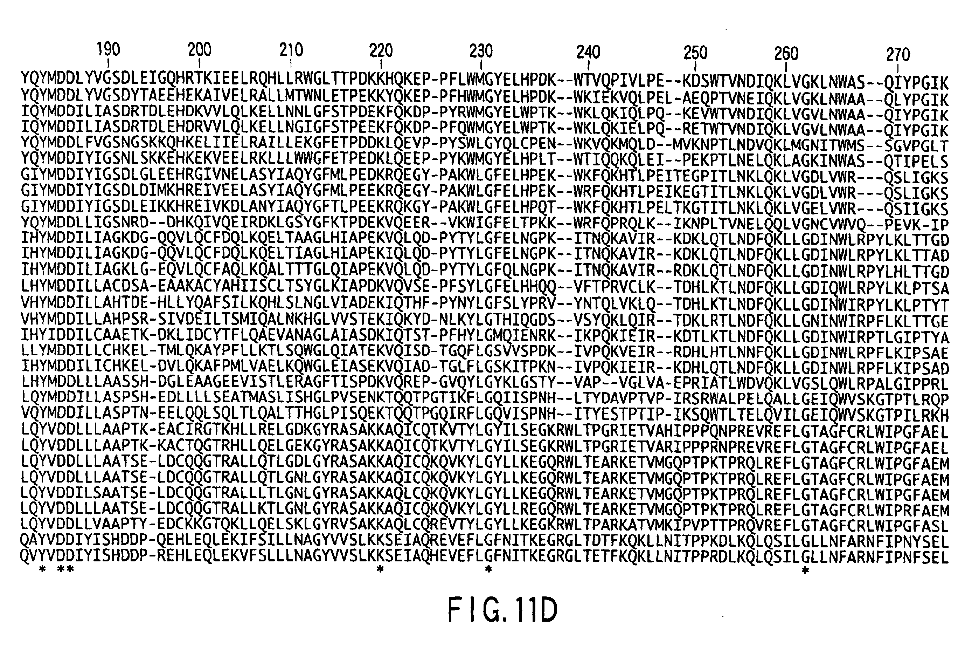

As shown in FIG. 11B, the Lys (K) as the amino acid No. 65 is conserved throughout the 31 enzymes searched. With regards to the other amino acids (e.g., the amino acid No. 100 shown in FIG. 11C) are not always conserved throughout the 31 enzymes. However, even if the amino acid is not completely conserved throughout the searched sequences, the amino acid which satisfies the items 1) and 2) mentioned above can be regarded as the amino acid constituting the target amino acid group. Herein, it is an essential requirement that the types of the amino acids constituting the rat pol. β pocket amino acid group-1 are identical with the types of the amino acids constituting HIV pocket amino acid group-1. Although the other requirements (i.e., the distances between the Cα atoms of each of the amino acids, the amino acids being present at the surface of the enzyme, and the amino acids being conserved among different organisms in terms of biological classification) are not necessarily essential, there is a decreasing priority in the order of mentioning in determining the target amino acid group, i.e., the requirement of the distances between the Cα atoms is given a highest priority.

As a result of the determination by searching, it has been confirmed that, in HIV reverse transcriptase, Lys-65, Leu-100, His-235 and Thr-386 constituting HIV pocket amino acid group-1 satisfy the above items 1), 2) and 3).

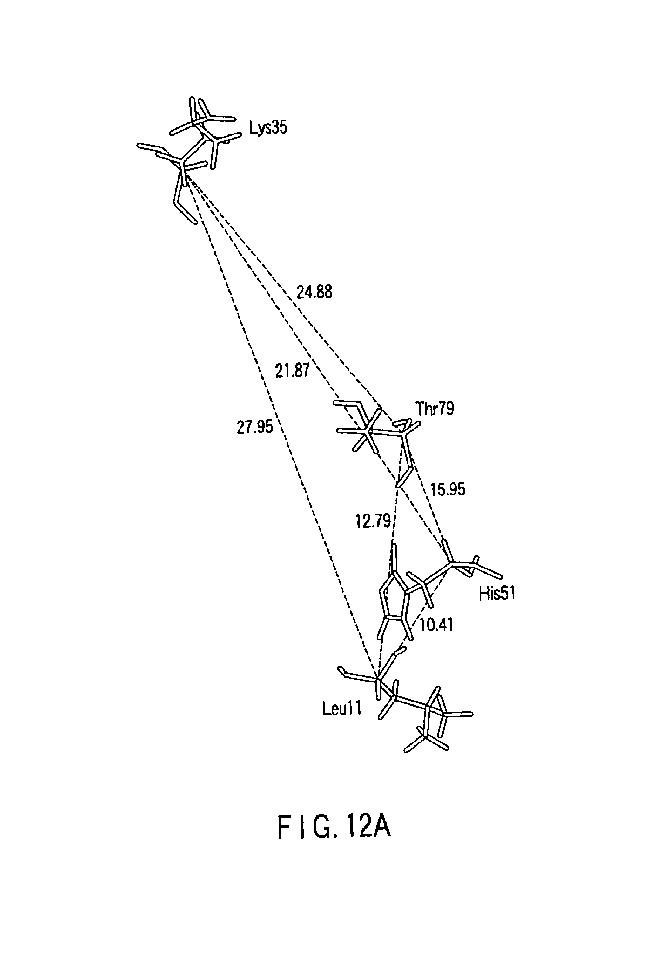

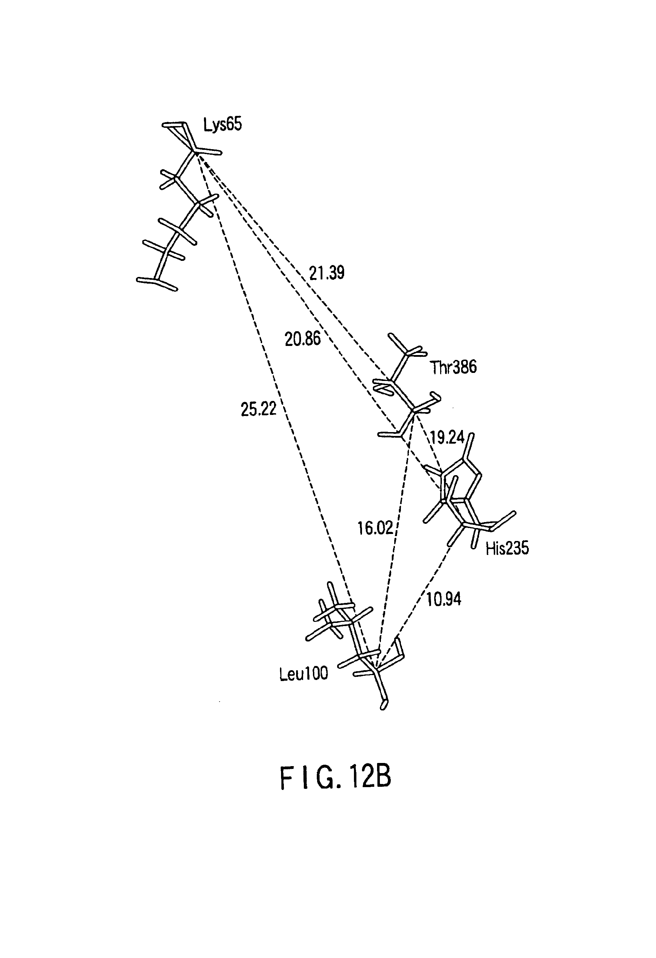

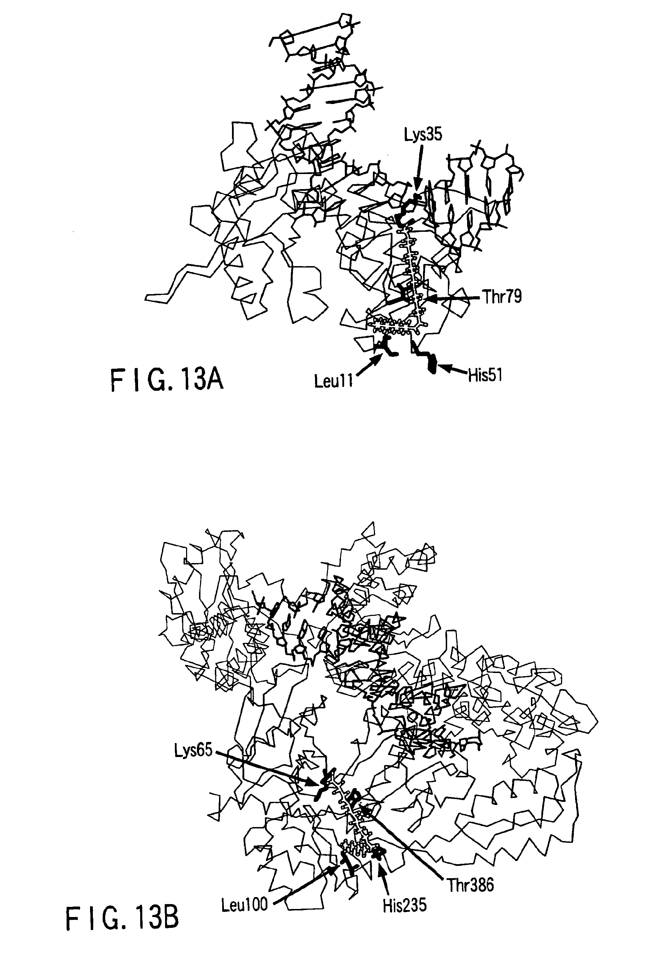

FIG. 12B shows, in the three-dimensional manner, the geometric configuration of the Cα atoms of Lys-65, Leu-100, His-235 and Thr-386. FIG. 13B shows a three-dimensional model of HIV reverse transcriptase to which nervonic acid has bound. For comparison, FIG. 12A and FIG. 13A show the geometric configuration and the three-dimensional model, respectively, of rat pol. β to which nervonic acid has bound.

### <1-6> Study of the binding manner of nervonic acid on rat pol. β

FIGS. 14A and 14B show a three-dimensional molecular model of nervonic acid (NA) and rat pol. β, in which nervonic acid has bound on the 8 kDa domain of rat pol. β (prepared by with "Mol Graph", manufactured by DAIKIN INDUSTRIES, Ltd.). FIG. 14B is a view in which the model of FIG. 14A has been turned by 90° around the vertical axis.

### <1-7>

The confirmation of the inhibitory activity of nervonic acid on HIV reverse transcriptase was carried out.

Specifically, the inhibitory activity of nervonic acid on HIV reverse transcriptase was measured according to the method described in reference 8 listed below.

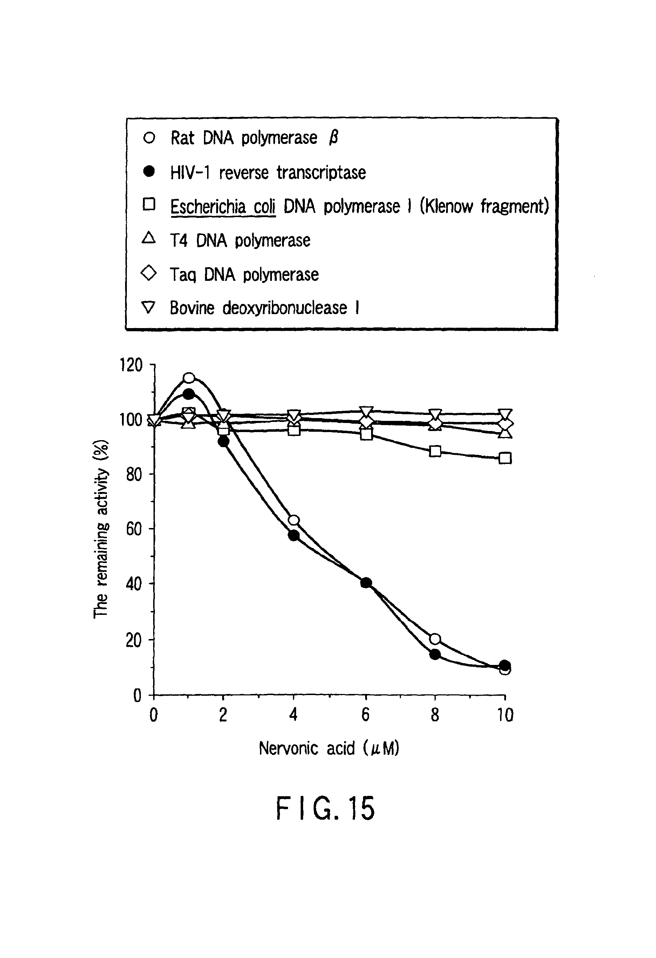

The result is shown in FIG. 15.

As is obvious from the inhibition curve shown in FIG. 15, the inhibitory activity of nervonic acid on HIV reverse transcriptase is substantially equal to the inhibitory activity of nervonic acid on rat pol. β.

### <1-8> Designing an inhibitor on HIV reverse transcriptase

As shown in the molecular models of FIGS. 13A and 13B of the <1-5>, it can be assumed that the binding manner of nervonic acid with rat pol. β is substantially the same as the binding manner of an inhibitor with HIV reverse transcriptase. Thus, it is possible to propose, as one example of the molecular structure of a preferable inhibitor on HIV reverse transcriptase, a cis-fatty acid in which the number of carbon atoms of the carbon chain is in the range of 16 to 24 and one double bond is located on the side of terminal methyl group with respect to the center of the carbon chain.

Further, designing a molecular structure as described below is also possible.

A fatty acid like nervonic acid is easily metabolized in vivo and therefore studying the role of rat pol. β is quite difficult in vivo. Thus, by modifying the molecular structure of the inhibitor such that the inhibitor will be non-metabolizable in vivo, while maintaining the molecular structure which presumably effects inhibitory activity on HIV reverse transcriptase, it is possible to provide a compound which maintains the inhibitory activity thereof in a body of a living organism in vivo.

### (Example 2)

In this example, rat pol. β, yeast topo II, nervonic acid and human topo II were used as the first enzyme, the second enzyme, the first inhibitor and the third (target) enzyme, respectively, for designing a molecular structure of an inhibitor on human topo II.

### <2-1> The geometric configuration of the binding sites of rat pol. β to which nervonic acid is to bind, was determined in a manner similar to that described in <1-4> of example 1.

### <2-2> The inhibitory activity of nervonic acid on yeast (S. cerevisiae) topoisomerase II

The inhibitory activity of nervonic acid on yeast topo II was measured according to the method described in references 10 and 11.

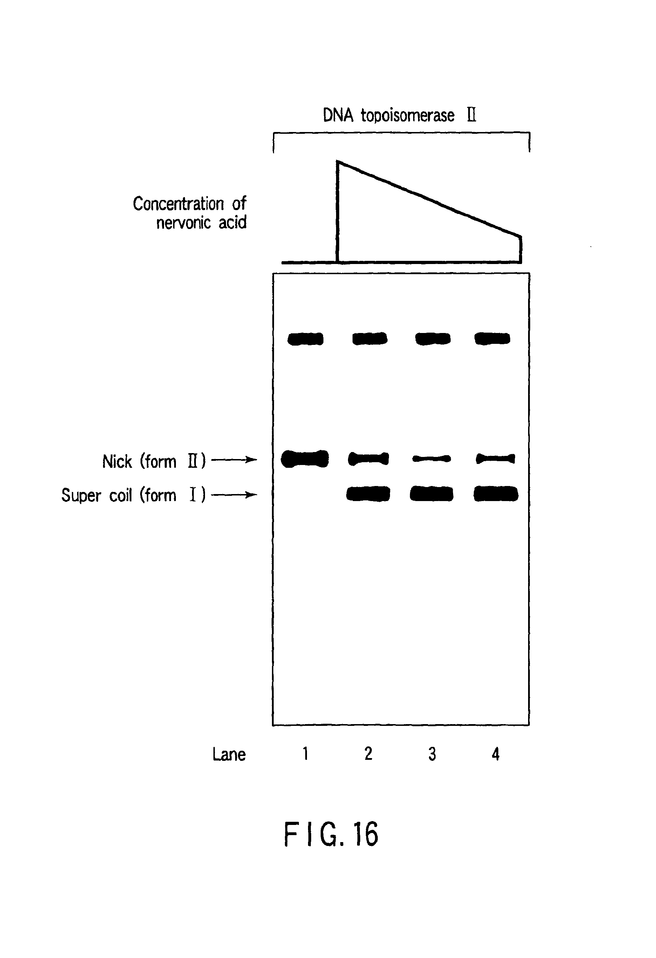

FIG. 16 shows the results of the measurement. FIG. 16 is a view showing the result of the electrophoresis in which lane 1 represents the absence of nervonic acid and other lanes each represent different concentrations of nervonic acid, which are 100 µM, 50 µM and 25 µM.

In FIG. 16, the nick (Form II) represents the band of the reaction product and the super coil (Form I) represents the band of the unreacted substance.

### <2-3> Determination, by searching, of the target amino acid group on yeast topo II

Whether such amino acids as those satisfying the following items 1) to 3) exist or not was searched using a computer software (INSIGHT II/BINDING SITE ANALYSIS, manufactured by MSI).
Item 1) determination, by searching, of whether or not a pocket amino acid group (yeast topo II pocket amino acid group-1) corresponding to the rat pol. β pocket amino acid group-1 is present in the amino acid sequence of yeast topo II;
Item 2) determination, by searching, of whether or not the amino acids constituting the yeast topo II pocket amino acid group-1 are present at the surface of yeast topo II in the tertiary structure thereof; and
Item 3) determination, by searching, of whether or not the amino acids constituting the yeast topo II pocket amino acid group-1 are conserved among topo II enzymes from different species, in terms of biological classification, from yeast.

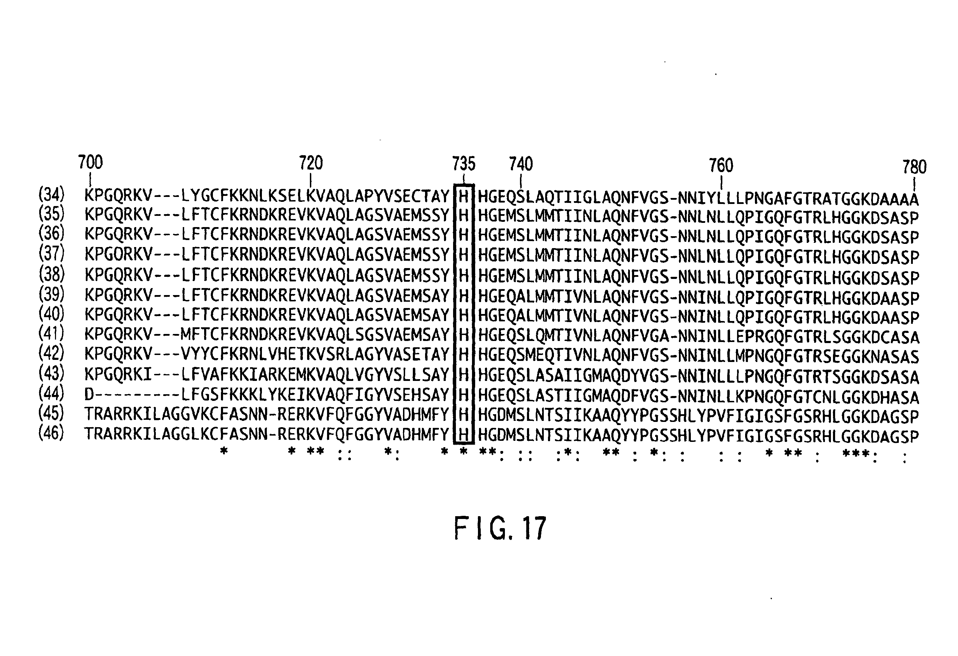

In the searching of the above item 1), the amino acids of yeast topo II having such distances between each of the Cα atoms as those within a range of ±2 × 10⁻¹ nm with respect to the corresponding distances between each of the Cα atoms in pol. β shown in Table 1, were selected. Further, in the search of the above item 3), 13 topo II enzymes derived from different species shown in FIG. 17 were used.

FIG. 17 shows a portion (amino acid Nos. 700 to 789) of the amino acid residues of 13 topo II enzymes, which correspond to SQE ID Nos. 34 to 46, respectively, of the Sequence Listing shown below.

From FIG. 17, it is revealed that His (H) of the amino acid No. 735 and Leu (L) of the amino acid No. 741 have been conserved, in terms of the evolution theory, in 13 topo II enzymes derived from different species.

As a result of the search, it was confirmed that Thr-596, His-735, Leu-741 and Lys-983 (the topo II pocket amino acid group-1) satisfy the above items 1) to 3).

FIG. 9B shows, in a three-dimensional manner, the geometric configuration of the Cα atoms of Thr-596, His-735, Leu-741 and Lys-983. Further, FIG. 10B indicates these four amino acid residues constituting the topo II pocket amino acid group-1, in a model in which the amino acid residues of topo II are expressed as ribbons.

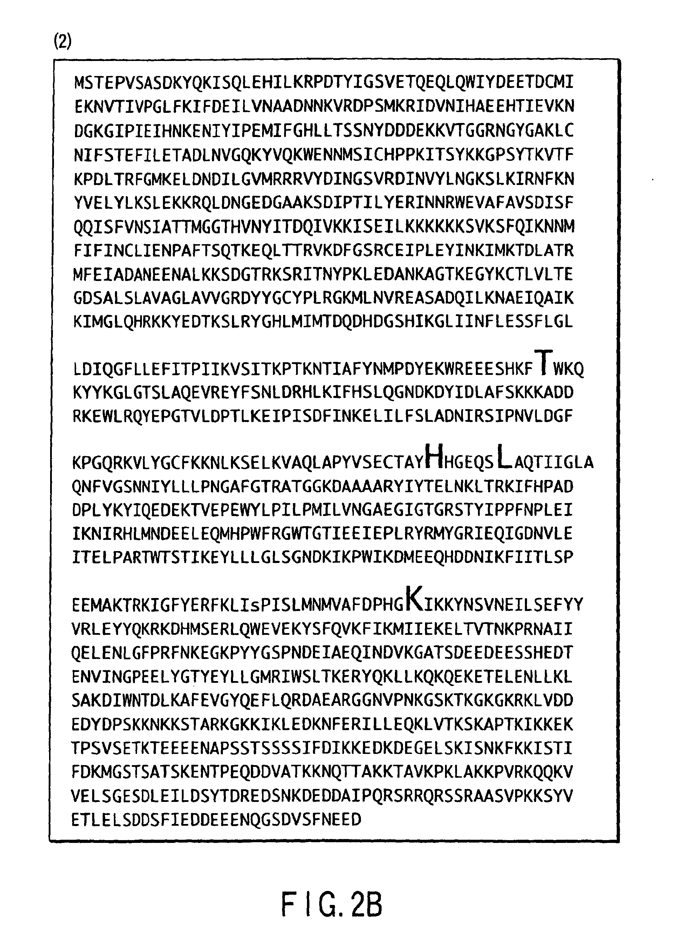

FIG. 2B shows the amino acid sequence of yeast topo II (SEQ ID No. 2 of the Sequence Listing shown below). The amino acid sequence is available from SWISS-Plot Data Base: 1BJT.

When the sequence of yeast topo II shown in FIG. 2B is compared with the sequence of rat pol. β shown in FIG. 2A, the order in which Leu-11, Lys-35, His-51 and Thr-79 constituting the rat pol. *β* pocket amino acid group-1 appear in the primary sequence of the amino acid residues is different from the order in which Thr-596, His-735, Leu-741 and Lys-983 constituting the yeast topo II pocket amino acid group-1, except that the rat pol. β pocket amino acid group-1 and the topo II pocket amino acid group-1 have the same order of Leu and Lys therein. Further, the number of the amino acid residues present between each of the four amino acid residues is different, in the rat pol. β pocket amino acid group-1 and the topo II pocket amino acid group-1. Thus, it is very difficult to deduce the binding sites where nervonic acid is to bind to the two enzymes, from the primary sequences of the amino acid residues thereof.

### (Example 3)

An experiment was conducted in a manner similar to that of example 2, except that lithocholic acid was used instead of nervonic acid as the first inhibitor.

### <3-1> Measurements of ¹H-¹⁵N HMQC spectroscopy of rat pol. β in the presence and the absence of lithocholic acid

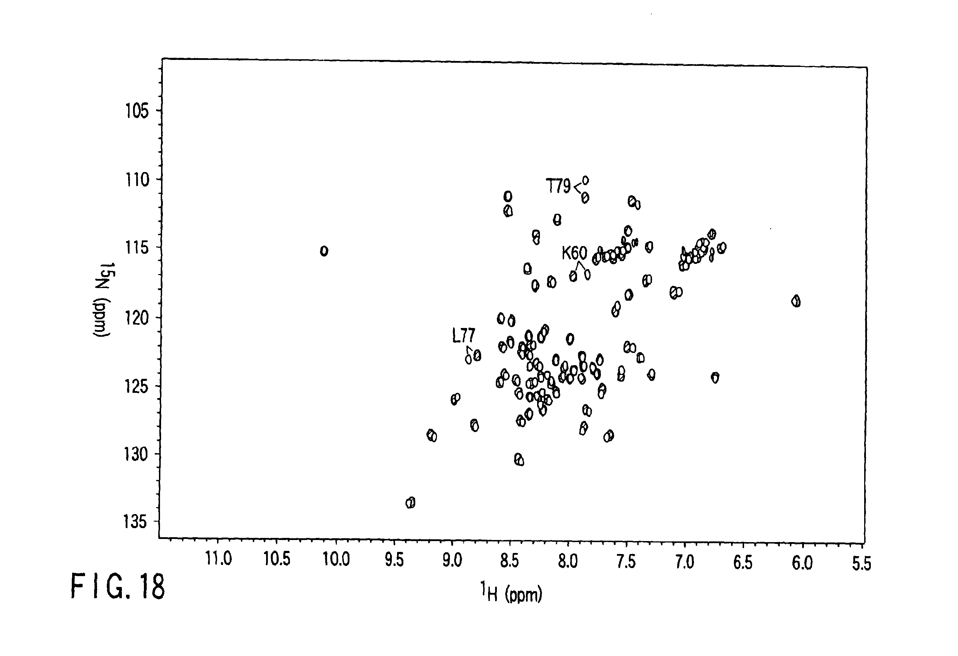

FIG. 18 shows the result of the ¹H-¹⁵N HMQC spectroscopy.

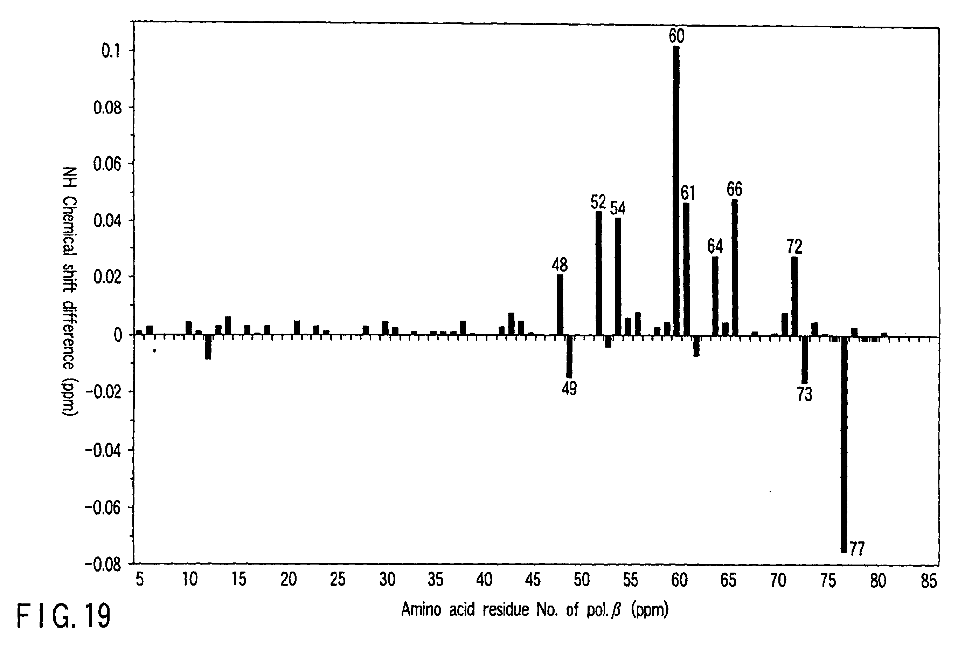

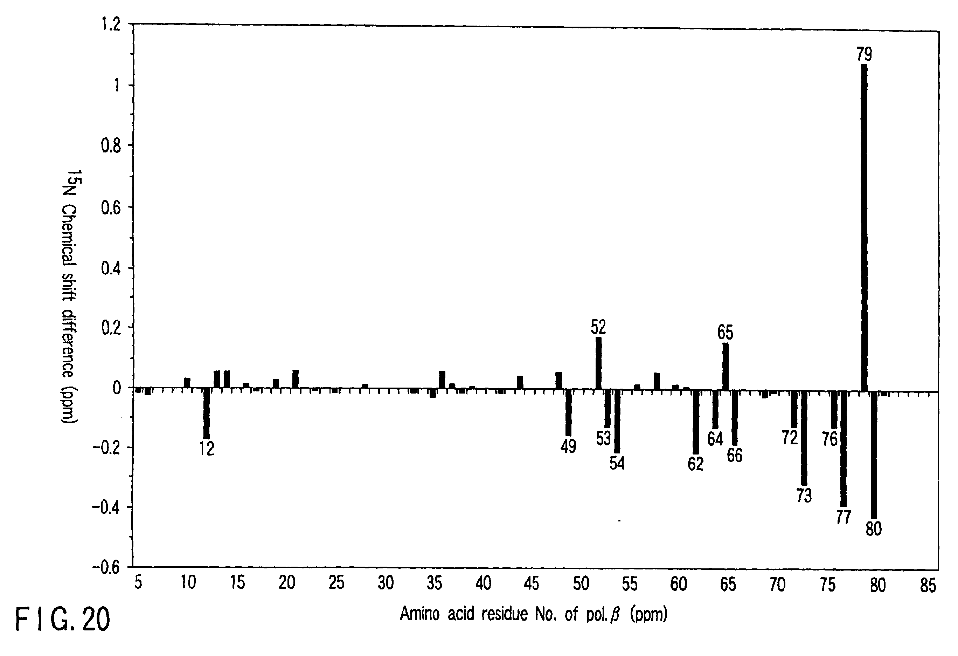

FIG. 19 is a bar graph in which the cross peaks of ¹H-¹⁵N HMQC spectroscopy shown in FIG. 18 is expressed as the relationship of ¹H chemical shift value vs. the amino acid residue number. FIG. 20 is a bar graph in which the cross peaks of ¹H-¹⁵N HMQC spectroscopy shown in FIG. 18 is expressed as the relationship of ¹⁵N chemical shift value vs. the amino acid residue number. In FIGS. 19 and 20, the vertical axis represents the chemical shift value and the horizontal axis represents the sequence number of the amino acid.

In this example, among the searched amino acid residues, those in which the absolute values of ¹H chemical shift and ¹⁵N chemical shift were 0.06 ppm or more and 0.6 ppm or more, respectively, were three amino acid residues: Lys-60, Leu-77 and Thr-79. The group consisting of these three amino acid residues will be referred to as "rat pol. β pocket amino acid group-2" hereinafter.

### <3-2> Measurements of the distances between the Cα atoms of each of the rat pol. β pocket amino acid group-2 2

Table 3 shows the distances between the Cα atoms of the rat pol. β pocket amino acid group-2.

**Table 3**

| Distance between the Cα atoms of the amino acid residues | Distance (×10⁻¹ nm) |
|---|---|
| Lys-60 and Leu-77 | 14.97 |
| Lys-60 and Thr-79 | 19.67 |
| Leu-77 and Thr-79 | 5.70 |

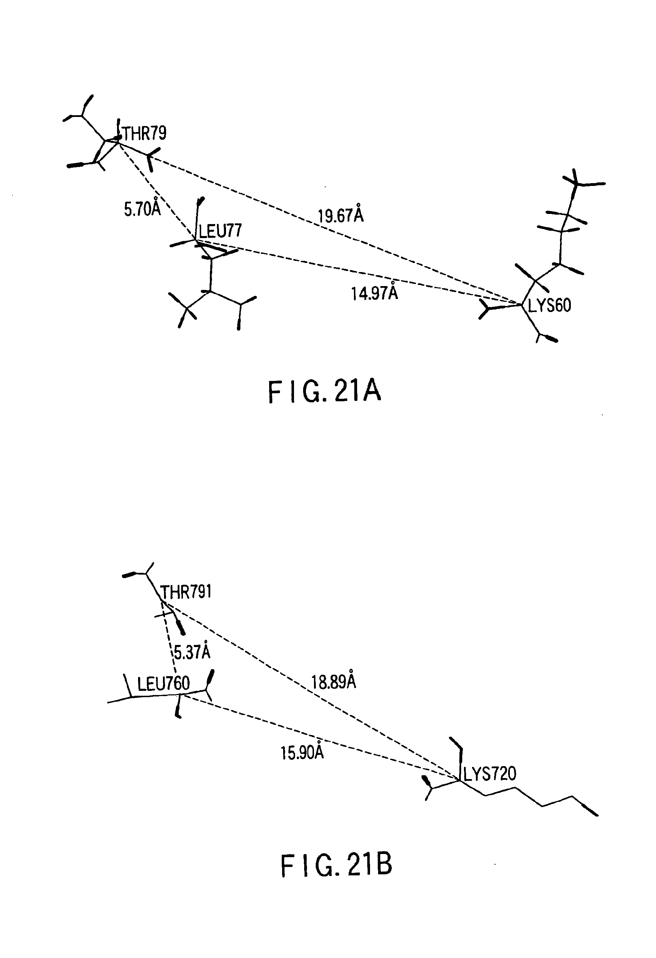

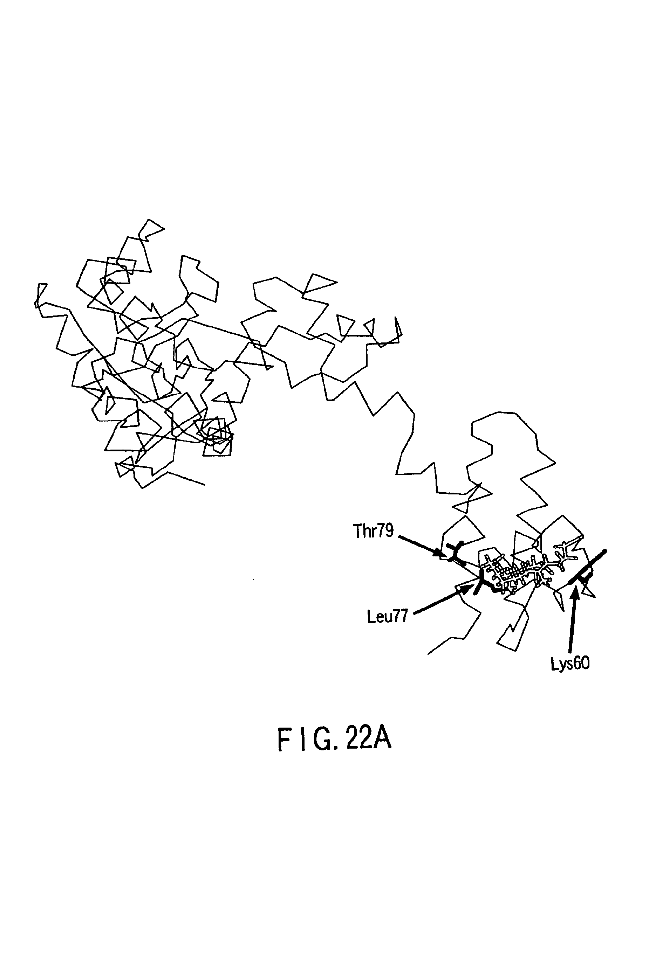

FIG. 21A is a view showing, in the three dimensional manner, the geometric configuration of the Cα atoms of the amino acid residues distanced from each other as shown in Table 3. FIG. 22A is a view showing, in the three dimensional manner, the amino acid residues of the rat pol. β, in a binding state with lithocholic acid, and the distances between the Cα atoms of these amino acid residues. Further, FIG. 22A is a view indicating the three amino acid residues constituting the rat pol. β pocket amino acid group-2, to which lithocholic acid has bound, in a model in which the amino acid residues of rat pol. β are expressed as a line.

### <3-3> Determination, by searching, of the target amino acid group in yeast topo II

Item 1) determination, by searching, of whether or not a pocket amino acid group (yeast topo II pocket amino acid group-2) corresponding to the rat pol. β pocket amino acid group-2 are present in the amino acid sequence of yeast topo II;
Item 2) determination of whether or not the amino acids constituting the yeast topo II pocket amino acid group-2 are present at the surface of yeast topo II in the tertiary structure thereof; and
Item 3) determination, by searching, of whether or not the amino acids constituting the yeast topo II pocket amino acid group-2 are the amino acids conserved in other topo II enzymes derived from different species, in terms of biological classification, from yeast.

In the searching of the above item 1), the amino acids of yeast topo II having such distances between each of the Cα atoms as those within a range of ±2 × 10⁻¹ nm with respect to the corresponding distance between each of the Cα atoms in pol. β shown in Table 3, were selected.

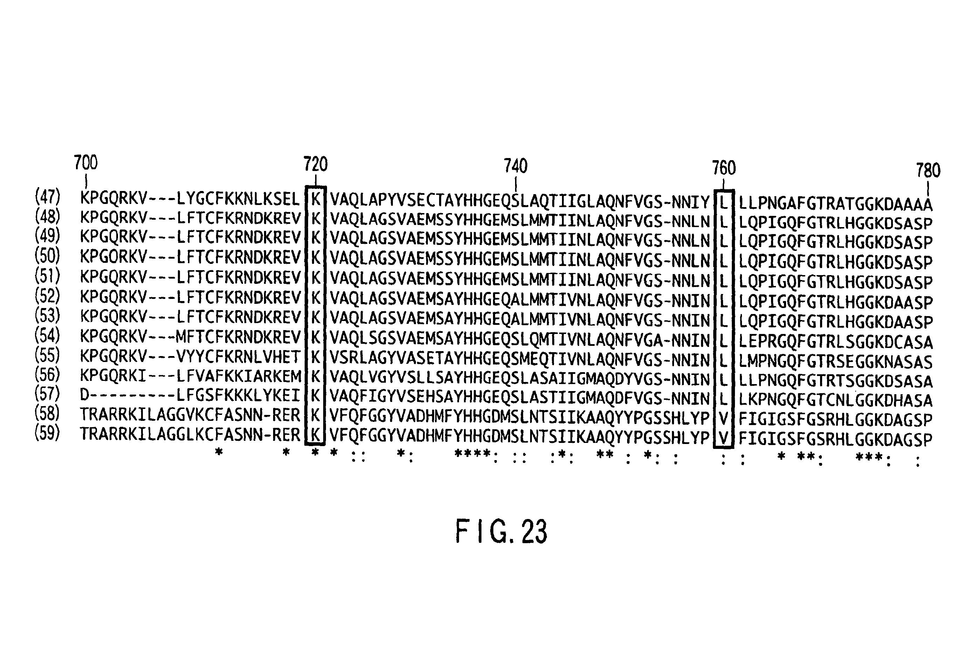

FIG. 23 shows a portion (amino acid Nos. 700 to 780) of the amino acid residues of topo II of 13 types which correspond to SQE ID Nos. 34 to 46, respectively, of the Sequence Listing shown below.

From FIG. 23, it is understood that Lys (K) of the amino acid No. 720 and Leu (L) of the amino acid No. 760 have been conserved, in terms of the evolution theory, in 13 topo II enzymes derived from different species.

As a result of the search, it was confirmed that Lys-720, Leu-760 and Thr-791 (topo II pocket amino acid group-2) satisfy the above items 1) to 3).

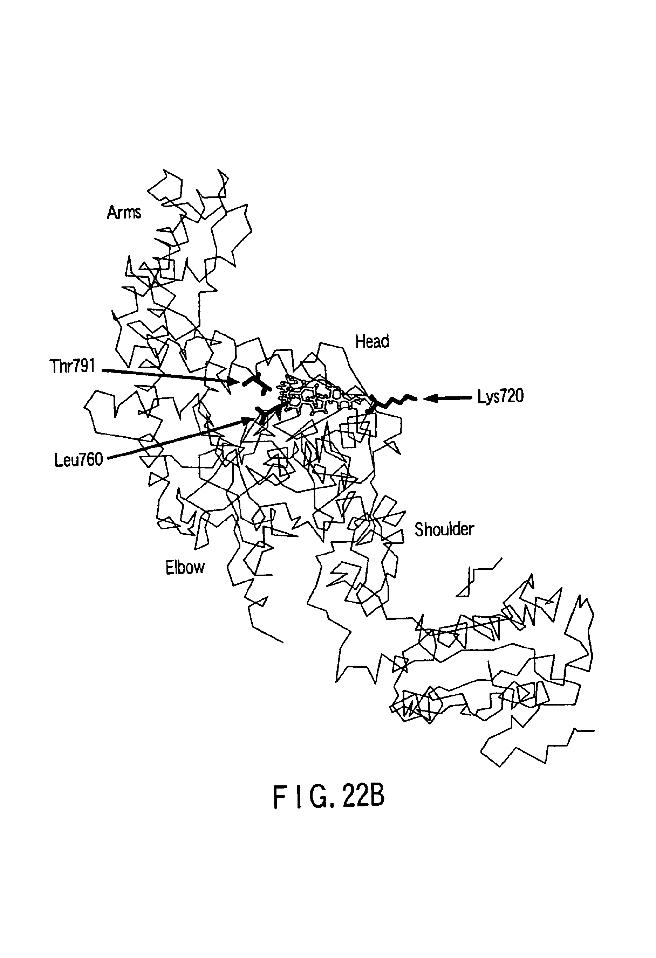

FIG. 21B shows, in a three-dimensional manner, the geometric configuration of the Cα atoms of Lys-720, Leu-760 and Thr-791. Further, FIG. 22B indicates these three amino acid residues constituting the topo II pocket amino acid group-2, to which lithocholic acid has bound, in a model in which the amino acid residues of topo II are expressed as a line.

### <3-4> Designing a novel inhibitor of human topo II

In consideration of the binding manner of lithocholic acid to rat pol. β described in the <2-6>, it is expected that a steroid skeleton having at least a carboxyl group in the vicinity of the terminal end of the molecular structure thereof will advantageously be used as the structure of an inhibitor of human topo II. Further, it will also be advantageous to add a side chain such as an alkyl group to the molecular structure, such that the inhibitor is to bind more fittingly to the triangle formed by Lys-720, Leu-760 and Thr-791.

### (References)

The entire contents disclosed in the following references are incorporated herein by reference.
(1) Mizushina et al., J. Antibiot. (Tokyo) 49, 491-492 (1996)
(2) Mizushina et al., Biochem. Biophys. Acta 1308, 256-262 (1996)
(3) Mizushina et al., Biochem. Biophys. Acta 1336, 509-521 (1997)
(4) Mizushina et al., Biol. Chem. 274, 25599-25607 (1999)
(5) B. Z. Zmudzka et al., Proc. Natl. Acad. Sci. U.S.A., 83, 5106-5110 (1986)
(6) H. Pelletier et al., Science, 264, 1891-1903 (1994)
(7) M. R. Sawaya et al., Science, 264, 1930-1935 (1994)
(8) Mizushina et al., Biochem. Biophys. Acta, 1336, 509-521 (1997)
(9) D. Liu et al., Biochemistry, 33, 9537-9545 (1994)
(10) F. Liu et al., Proc. Natl. Acad. Scie. USA 78, 3487-3491 (1981)
(11) A. Ferrp et al., J. Biol. Chem. 259, 547-554 (1984)
(12) D. Liu et al., Biochemistry, 35, 6188-6200 (1996)
(13) R. Prasad et al., J. Biol. Chem., 273, 111121-11126 (1998)
(14) Kobayashi et al., Nature Struct. Biol. 4, 677 (1997)
(15) Wallace et al., Protein Sci. 6, 2308-2323 (1997)
(16) Russell et al., J. Mol. Biol. 279, 1211-1227 (1998)

## Claims

1. A method of designing a molecular structure of an inhibitor on a target enzyme whose secondary and tertiary structures are known, **characterized in that** the designing is carried out by
analyzing, in the tertiary structure of a first enzyme whose primary, secondary and tertiary structures are known, a geometric configuration formed by amino acids to which a first inhibitor, having biological, inhibitory activity on the first enzyme, is to bind; and
searching, in the target enzyme, the similar geometric configuration to the geometric configuration that was analyzed in the first enzyme;
thereby designing the molecular structure of the inhibitor on the target enzyme, and the method **characterized in** which comprises:
(1) a step of determining, in the tertiary structure of the first enzyme, a first pocket amino acid group consisting of plural amino acids to which the first inhibitor is to bind, wherein the plural amino acids are determined by comparing ¹H-¹⁵N HMQC spectroscopy of the first enzyme in the absence of the first inhibitor with ¹H-¹⁵N HMQC spectroscopy of the first enzyme in the presence of the first inhibitor, and selecting amino acids that satisfy the condition that the absolute value of ¹H chemical shift is equal to or more than a specific value and/or the absolute value of ¹⁵N chemical shift is equal to or more than another specific value;
(2) a step of determining the geometric configuration of the first pocket amino acid group, by measuring, in the tertiary structure of the first enzyme, distances between the Cα atoms of each of the plural amino acids constituting the first pocket amino acid group;
(3) a step of detecting, in the tertiary structure of the target enzyme, one or more candidates for a target pocket amino acid group having a geometric configuration similar to the geometric configuration formed by the first pocket amino acid group determined in the step (2), wherein it is presumed that the candidate for the target pocket amino acid group exists on the target enzyme, if the following conditions are satisfied:
- respective amino acids constituting the candidate for the target pocket amino acid group are of the same kinds as the plural amino acids constituting the first pocket amino acid group determined in the step (2), and
- the absolute values of differences between (a) and (b) are within a specific value, wherein (a) is respective distances, in the tertiary structure of the target enzyme, between the Cα atoms of each of the amino acids constituting the target pocket amino acid group, and (b) is respective distances between the Cα atoms in the first pocket amino acid group obtained in the step (2); and
(4) a step of screening the amino acids constituting the candidate for the target pocket amino acid group by the following two requirements:
(requirement 1) the amino acids are those that exist on the surface of the target enzyme in the tertiary structure thereof; and
(requirement 2) the amino acids are those that have been conserved among species, when a comparison is made between the primary sequence of the amino acids of the target enzyme and the primary sequence of the amino acids of another enzyme having the same biochemical activity as the target enzyme but derived from an organism of a different species from that of the target enzyme, in terms of biological classification; and
determining the amino acids satisfying the above two requirements to be those constituting the usable target pocket amino acid group for designing the molecular structure of the inhibitor on the target enzyme; thereby
the molecular structure of the inhibitor on the target enzyme is designed on the basis of the kinds of amino acid residues of the first enzyme, to which the first inhibitor is to bind, and the geometric configuration of the amino acid residues.

2. A method of designing a molecular structure of an inhibitor on human immunodeficiency virus reverse transcriptase, **characterized in that** the designing is carried out by
analyzing, in the tertiary structure of rat DNA polymerase β, a geometric configuration formed by amino acids to which nervonic acid is to bind; and
searching, in human immunodeficiency virus reverse transcriptase, the similar geometric configuration to the geometric configuration that was analyzed in rat DNA polymerase β;
thereby designing the molecular structure of the inhibitor on human immunodeficiency virus reverse transcriptase, and the method **characterized in** which comprises:
(1) a step of determining, in the tertiary structure of rat DNA polymerase β, a rat DNA polymerase β pocket amino acid group consisting of plural amino acids to which nervonic acid is to bind, wherein the plural amino acids are determined by comparing ¹H-¹⁵N HMQC spectroscopy of rat DNA polymerase β in the absence of nervonic acid with ¹H-¹⁵N HMQC spectroscopy of rat DNA polymerase β in the presence of nervonic acid, and selecting amino acids that satisfy the condition that the absolute value of ¹H chemical shift is 0.06 ppm or more or the absolute value of 15_{N} chemical shift is 0.4 ppm or more, thereby determining the rat DNA polymerase β pocket amino acid group consisting of Leu-11, Lys-35, His-51 and Thr-79;
(2) a step of determining the geometric configuration of a triangular pyramid formed by Leu-11, Lys-35, His-51 and Thr-79 of the rat DNA polymerase β pocket amino acid group, by measuring, in the tertiary structure of rat DNA polymerase β, distances between the Cα atoms of each of Leu-11, Lys-35, His-51 and Thr-79 constituting the rat DNA polymerase β pocket amino acid group;
(3) a step of detecting, in the tertiary structure of the human immunodeficiency virus reverse transcriptase, one or more candidates for a target pocket amino acid group having a geometric configuration similar to the geometric configuration formed by the rat DNA polymerase β pocket amino acid group determined in the step (2), wherein it is presumed that the candidate for the target pocket amino acid group exists on human immunodeficiency virus reverse transcriptase if the following conditions are satisfied:
- respective amino acids constituting the candidate for the target pocket amino acid group are of the same kinds as Leu, Lys, His and Thr constituting the rat DNA polymerase β pocket amino acid group determined in the step (2), and
- the differences between (a) and (b) are within ± 2 × 10⁻¹ nm, wherein (a) is respective distances, in the tertiary structure of human immunodeficiency virus reverse transcriptase, between the Cα atoms of each of the amino acids constituting the target pocket amino acid group, and (b) is respective distances between the Cα atoms of the rat DNA polymerase β pocket amino acid group obtained in the step (2); and
(4) a step of screening the amino acids, Lys, Leu, His and Thr, constituting the candidate for the target pocket amino acid group by the following two requirements:
(requirement 1) the amino acids are those that exist on the surface of human immunodeficiency virus reverse transcriptase in the tertiary structure thereof; and
(requirement 2) the amino acids are those that have been conserved among species, when a comparison is made between the primary sequence of human immunodeficiency virus reverse transcriptase and the primary sequence of the amino acids of immunodeficiency virus reverse transcriptase derived from an organism except for human; and
determining the amino acids, Lys-65, Leu-100, His-235 and Thr-386, satisfying the above two requirements to be those constituting the usable target pocket amino acid group for designing the molecular structure of the inhibitor on human immunodeficiency virus reverse transcriptase; thereby
the molecular structure of the inhibitor on human immunodeficiency virus reverse transcriptase is designed on the basis of the kinds of amino acid residues of rat DNA polymerase β, to which nervonic acid is to bind, and the geometric configuration of the amino acid residues.

3. A method of designing a molecular structure of an inhibitor on a target enzyme whose tertiary structure is unknown, **characterized in that** the designing is carried out by
analyzing, in the tertiary structure of a first enzyme whose primary, secondary and tertiary structures are known, a geometric configuration formed by amino acids to which a first inhibitor having biological, inhibitory activity on the first enzyme is to bind; and
searching, in a second enzyme whose primary, secondary and tertiary structures are known, the similar geometric configuration to the geometric configuration that was analyzed in the first enzyme;
thereby designing the molecular structure of the inhibitor on the target enzyme, and the method **characterized in** which comprises:
(1) a step of determining, in the tertiary structure of the first enzyme, a first pocket amino acid group consisting of plural amino acids to which the first inhibitor is to bind, wherein the plural amino acids are determined by comparing ¹H-¹⁵N HMQC spectroscopy of the first enzyme in the absence of the first inhibitor with ¹H-¹⁵N HMQC spectroscopy of the first enzyme in the presence of the first inhibitor, and selecting amino acids that satisfy the condition that the absolute value of ¹H chemical shift is equal to or more than a specific value and/or the absolute value of ¹⁵N chemical shift is equal to or more than another specific value;
(2) a step of determining the geometric configuration of the first pocket amino acid group, by measuring, in the tertiary structure of the first enzyme, distances between the Cα atoms of each of the plural amino acids constituting the first pocket amino acid group;
(3) a step of detecting, in the tertiary structure of the second enzyme, a second pocket amino acid group having a geometric configuration similar to the geometric configuration formed by the first pocket amino acid group determined in the step (2), wherein it is presumed that the second pocket amino acid group has a geometric configuration similar to the geometric configuration formed by the first pocket amino acid group if the following requirements are satisfied:
(requirement 1) the second enzyme has the same biochemical activity as that of the target enzyme;
(requirement 2) the organism from which the second enzyme is derived, belongs to a different species, in terms of biological classification, from the organism from which the target enzyme is derived;
(requirement 3) the primary, secondary and tertiary structures of the second enzyme are known; and
(requirement 4) the second enzyme is biochemically inhibited by the first inhibitor, and
respective amino acids constituting the second pocket amino acid group are of the same kinds as the plural amino acids constituting the first pocket amino acid group determined in the step (2), and
the absolute values of differences between (a) and (b) are within a specific value, wherein (a) is respective distances, in the tertiary structure of the second enzyme, between the Cα atoms of each of the amino acids constituting the second pocket amino acid group, and (b) is respective distances between the Cα atoms in the first pocket amino acid group obtained in the step (2); and
(4) a step of screening the amino acids constituting the second pocket amino acid group by the following two requirements:
(requirement 1) the amino acids are those that exist on the surface of the second enzyme in the tertiary structure thereof; and
(requirement 2) the amino acids are those that have been conserved among species, when a comparison is made among the primary sequence of the amino acids of the second enzyme, the primary sequence of the amino acid of the target enzyme and the primary sequence of the amino acids of another enzyme having the same biochemical activity as that of the second enzyme but derived from an organism of a different species from that of both the second enzyme and the target enzyme, in terms of biological classification; and
determining the amino acids satisfying the above two requirements to be those constituting the usable target pocket amino acid group for designing the molecular structure of the inhibitor on the target enzyme; thereby
the molecular structure of the inhibitor on the target enzyme is designed on the basis of the kinds of amino acid residues of the second enzyme, to which the first inhibitor is to bind, and the geometric configuration of the amino acid residues.

4. A method of designing a molecular structure of an inhibitor on human DNA topoisomerase II, **characterized in that** the designing is carried out by
analyzing, in the tertiary structure of rat DNA polymerase β, a geometric configuration formed by amino acids to which nervonic acid is to bind; and
searching, in yeast DNA topoisomerase II, the similar geometric configuration to the geometric configuration that was analyzed in rat DNA polymerase β;
thereby designing the molecular structure of the inhibitor on human DNA topoisomerase II, and the method **characterized in** which comprises:
(1) a step of determining, in the tertiary structure of rat DNA polymerase β, a rat DNA polymerase β pocket amino acid group consisting of plural amino acids to which nervonic acid is to bind, wherein the plural amino acids are determined by comparing ¹H-¹⁵N HMQC spectroscopy of rat DNA polymerase β in the absence of nervonic acid with ¹H-¹⁵N HMQC spectroscopy of rat DNA polymerase β in the presence of nervonic acid, and selecting amino acids that satisfy the condition that the absolute value of ¹H chemical shift is 0.06 ppm or more or the absolute value of ¹⁵N chemical shift is 0.4 ppm or more, thereby determining the rat DNA polymerase β pocket amino acid group consisting of Leu-11, Lys-35, His-51 and Thr-79;
(2) a step of determining the geometric configuration of a triangular pyramid formed by rat DNA polymerase β pocket amino acid group, by measuring, in the tertiary structure of rat DNA polymerase β, distances between the Cα atoms of each of Leu-11, Lys-35, His-51 and Thr-79 constituting rat DNA polymerase β pocket amino acid group;
(3) a step of selecting, in the tertiary structure of yeast DNA topoisomerase II, yeast DNA topoisomerase II pocket amino acid group consisting of Leu, Lys, His and Thr, and having a geometric configuration similar to the geometric configuration formed by rat DNA polymerase β pocket amino acid group determined in the step (2), wherein
the differences between (a) and (b) are within ± 2 × 10⁻¹ nm, wherein (a) is respective distances, in the tertiary structure of yeast DNA topoisomerase II, between the Cα atoms of each of Leu, Lys His and Thr constituting yeast DNA topoisomerase II pocket amino acid group, and (b) is respective distances between the Cα atoms in rat DNA polymerase β pocket amino acid group obtained in the step (2); and
(4) screening the amino acids constituting yeast DNA topoisomerase II pocket amino acid group by the following two requirements:
(requirement 1) the amino acids are those that exist on the surface of yeast DNA topoisomerase II in the tertiary structure thereof; and
(requirement 2) the amino acids are those that have been conserved among species, when a comparison is made among the primary sequence of the amino acids of yeast DNA topoisomerase II, the primary sequence of the amino acids of human DNA topoisomerase II and the primary sequence of the amino acids of DNA topoisomerase II derived from an organism except for both yeast and human ; and determining the amino acids, Thr-596, His-735, Leu-741 and Lys-983, satisfying the above two requirements to be those constituting the usable target pocket amino acid group for designing the molecular structure of the inhibitor on human DNA topoisomerase II; thereby
the molecular structure of the inhibitor on human DNA topoisomerase II is designed on the basis of the kinds of amino acid residues on yeast DNA topoisomerase II, to which nervonic acid is to bind, and the geometric configuration of the amino acid residues.

5. A method of designing a molecular structure of an inhibitor on human DNA topoisomerase II, **characterized in that** the designing is carried out by
analyzing, in the tertiary structure of rat DNA polymerase β, a geometric configuration formed by amino acids to which lithocholic acid is to bind; and
searching, in yeast DNA topoisomerase II, the similar geometric configuration to the geometric configuration that was analyzed in rat DNA polymerase β;
thereby designing the molecular structure of the inhibitor on human DNA topoisomerase II, and the method **characterized in** which comprises:
(1) a step of determining, in the tertiary structure of rat DNA polymerase β, a rat DNA polymerase β pocket amino acid group consisting of plural amino acids to which lithocholic acid is to bind, wherein the plural amino acids are determined by comparing ¹H-¹⁵N HMQC spectroscopy of rat DNA polymerase β in the absence of lithocholic acid with ¹H-¹⁵N HMQC spectroscopy of rat DNA polymerase β in the presence of lithocholic acid, and selecting amino acids that satisfy the condition that the absolute value of ¹H chemical shift is 0.06 ppm or more or the absolute value of ¹⁵N chemical shift is 0.4 ppm or more, thereby determining the rat DNA polymerase β pocket amino acid group consisting of Lys-60, Leu-77 and Thr-79;
(2) a step of determining the geometric configuration of a triangular shape formed by rat DNA polymerase β pocket amino acid group, by measuring, in the tertiary structure of rat DNA polymerase β, distances between the Cα atoms of each of Lys-60, Leu-77 and Thr-79 constituting rat DNA polymerase β pocket amino acid group;
(3) a step of selecting, in the tertiary structure of yeast DNA topoisomerase II, yeast DNA topoisomerase II pocket amino acid group consisting of Lys, Leu and Thr, and having a geometric configuration similar to the geometric configuration formed by rat DNA polymerase β pocket amino acid group determined in the step (2), wherein the differences between (a) and (b) are within ± 2 × 10⁻¹ nm, wherein (a) is respective distances, in the tertiary structure of yeast DNA topoisomerase II, between the Cα atoms of Lys, Leu and Thr constituting yeast DNA topoisomerase II pocket amino acid group, and (b) is respective distances between the Cα atoms of rat DNA polymerase β pocket amino acid group obtained in the step (2); and
(4) a step of screening the amino acids constituting yeast DNA topoisomerase II pocket amino acid group by the following two requirements:
(requirement 1) the amino acids are those that exist on the surface of yeast DNA topoisomerase II in the tertiary structure thereof; and
(requirement 2) the amino acids are those that have been conserved among species, when a comparison is made among the primary sequence of the amino acids of yeast DNA topoisomerase II, the primary sequence of the amino acids of human DNA topoisomerase II and the primary sequence of the amino acids of DNA topoisomerase II derived from an organism except for both yeast and human; and determining the amino acids, Lys-720, Leu-760 and Thr-791, satisfying the above two requirements to be those constituting the usable target pocket amino acid group for designing the molecular structure of the inhibitor on human DNA topoisomerase II; thereby
the molecular structure of the inhibitor on human DNA topoisomerase II is designed on the basis of the kinds of amino acid residues on yeast DNA topoisomerase II, to which lithocholic acid is to bind, and the geometric configuration of the amino acid residues.
